# EUROPEAN PATENT SPECIFICATION

(11) **EP 1 496 877 B1**
(45) Date of publication and mention of the grant of the patent: **01.10.2008**
(21) Application number: 03729217.4
(22) Date of filing: 13.01.2003
(51) Int. Cl.: A61K 31/00

(54) **METHOD AND COMPOSITION FOR TREATMENT OF DIABETES, HYPERTENSION, CHRONIC HEART FAILURE AND FLUID RETENTIVE STATES**
VERFAHREN UND ZUSAMMENSETZUNG ZUR BEHANDLUNG VON DIABETES, HYPERTONIE, CHRONISCHER HERZINSUFFIZIENZ UND MIT FLÜSSIGKEITSRETENTION EINHERGEHENDEN ZUSTÄNDEN
METHODE ET COMPOSITION PERMETTANT DE TRAITER LE DIABETE, L'HYPERTENSION, L'INSUFFISANCE CARDIAQUE CHRONIQUE ET LES TROUBLES DE RETENTION DE FLUIDE

(30) Priority: 11.01.2002 DK 200200047; 14.01.2002 US 348332 P
(43) Date of publication of application: 19.01.2005
(73) Proprietor: NOVO NORDISK A/S, 2880 Bagsvaerd (DK)
(72) Inventor: CARR, Richard, David, DK-3500 Vaerlose (DK)
(86) International application number: PCT/DK2003/000017
(87) International publication number: WO 2003/057200

(56) References cited:
- EP-A- 0 274 234
- WO-A-02/059343
- US-A1- 2002 137 120
- US-A1- 2002 193 562
- US-B2- 6 380 398
- MOTWANI J G ET AL: "Natriuretic response to neutral endopeptidase inhibition is blunted by enalapril in healthy men." HYPERTENTION, vol. 25, 1995, pages 637-642, XP002902889 American heart association Inc.
- KIRK J E ET AL: "Renal effects of concurrent E-24.11 and ACE inhibition in the aorto-venocaval fistula rat." BRITISH JOURNAL OF PHARMACOLOGY, vol. 119, 1996, pages 943-948, XP002902890 Stockton press 1996 ISBN: 0007-1188
- HOLST J J ET AL: "Inhibition of the activity of dipeptidyl-peptidase IV as a treatment for type 2 diabetes." DIABETES. UNITED STATES NOV 1998, vol. 47, no. 11, November 1998 (1998-11), pages 1663-1670, XP002260039 ISSN: 0012-1797
- POSPISILIK J ANDREW ET AL: "Dipeptidyl peptidase IV inhibitor treatment stimulates beta-cell survival and islet neogenesis in streptozotocin-induced diabetic rats." DIABETES. UNITED STATES MAR 2003, vol. 52, no. 3, March 2003 (2003-03), pages 741-750, XP002260040 ISSN: 0012-1797
- HUPE-SODMANN ET AL: 'Characterisation of the the processing by human neutral endopeptidase 24.11 of GLP-1(7-36)amide and comparison of the substrate specificity of the enzyme for other glucagon-like peptides' REGULATORY PEPTIDES vol. 58, no. 3, 1995, pages 149 - 156

## Description

### FIELD OF THE INVENTION

This invention relates to a pharmaceutical composition comprising a Dipeptidyl Peptidase-IV inhibitor in combination with an inhibitor of Neutral Endopeptidase.

### BACKGROUND OF THE INVENTION

Dipeptidyl peptidase-IV (DPP-IV), a serine protease belonging to the group of post-proline/alanine cleaving amino-dipeptidases, specifically removes the two N-terminal amino acids from proteins having proline or alanine in position 2.

Although the physiological role of DPP-IV has not been completely established, it is believed to play an important role in neuropeptide metabolism, T-cell activation, gastric ulceration, functional dyspepsia, obesity, appetite regulation, impaired fasting glucose (IFG) and diabetes.

DPP-IV has been implicated in the control of glucose metabolism because its substrates include the insulinotropic hormones Glucagon like peptide-1 (GLP-1) and Gastric inhibitory peptide (GIP). GLP-1 and GIP are active only in their intact forms; removal of their two N-terminal amino acids inactivates them. It is also speculated that other, as yet unknown substrates may participate in the beneficial effects of DPP-IV inhibitors in treatment of diabetes In vivo administration of synthetic inhibitors of DPP-IV prevents N-terminal degradation of

GLP-1 and GIP, resulting in higher plasma concentrations of these hormones, increased insulin secretion and, therefore, improved glucose tolerance. Therefore, such inhibitors have been proposed for the treatment of patients with Type 2 diabetes, a disease characterised by decreased glucose tolerance. (Holst, J. J.; Deacon, C. F. Diabetes 47 (1998) 1663-70) Diabetic dyslipidaemia is characterized by multiple lipoprotein defects, including moderately high serum levels of cholesterol and triglycerides, small LDL particles, and low levels of HDL cholesterol. The results of recent clinical trials reveal beneficial effects of cholesterol-lowering therapy in diabetic and non-diabetic patients, thus supporting increased emphasis on treatment of diabetic dyslipidaemia. The National Cholesterol Education Program's Adult Treatment Panel II advocated this need for intensive treatment of diabetic dyslipidaemia.

Obesity is a well-known risk factor for the development of many very common diseases such as atherosclerosis, hypertension and diabetes. The incidence of obese people and thereby also these diseases is increasing throughout the entire industrialised world. Except for exercise, diet and food restriction no convincing pharmacological treatment for reducing body weight effectively and acceptably currently exist. However, due to its indirect but important effect as a risk factor in mortal and common diseases it will be important to find treatment for obesity or appetite regulation. Even mild obesity increases the risk for premature death, diabetes, hypertension, atherosclerosis, gallbladder disease and certain types of cancer. In the industrialised western world the prevalence of obesity has increased significantly in the past few decades. Because of the high prevalence of obesity and its health consequences, its prevention and treatment should be a high public health priority.

At present a variety of techniques are available to effect initial weight loss. Unfortunately, initial weight loss is not an optimal therapeutic goal. Rather, the problem is that most obese patients eventually regain their weight. An effective means to establish and/or sustain weight loss is the major challenge in the treatment of obesity today.

Neutral Endopeptidase (NEP) is an enzyme known to be responsible for the metabolism of polypeptide hormones (e.g. atrial natriuretic factor and brain (B-type) natriuretic factor) which are involved with the regulation of extracellular fluid (volume/water/sodium ion) homeostasis. Furthermore, NEP is known to be involved in the metabolism of other biologically active peptides.

NEP inhibitors are useful as they are diuretic agents and, as such, are known medicines in the treatment if hypertension and chronic heart failure. They are effective at reducing peripheral vascular resistance and lowering the circulating volume (thus lowering cardiac preload). They are useful when treating both cardiac and non-cardiac sources of oedema. Hypertension and chronic heart failure are life threatening diseases, which increase the risk of cerebrovascular stroke and myocardial infarction. Diuretic agents, including thiazide diuretics and loop diuretics provide important drug therapy for these two disorders. NEP inhibitors, either administered alone (Westheim, AS., Bostrom, P., Christensen, CC.et al J Am Coll Cardiol., 1999, 34: 1794-1801), or in combination with ACE inhibitors (Newby, DE., McDonagh, T., Currie, PF. et al Eur Heart J., 1998; 19: 1808-1813) have also been shown to cause favourable effects in these disease states. Furthermore compounds showing dual inhibition of both NEP and ACE (e.g. omapatrilat) show promise in treatment of hypertension and heart failure (Trippodo, NC., Fox, M,. Monticello, TM et al J Cardiovasc. Pharmacol., 1999; 34: 782-790) as a result of the useful addition of the two effects of the component drugs on polypeptide levels. NEP is also reported to be involved in metabolism of GLP-1 (Hupe-Sodmann, K., McGregor, GP., Bridenbaugh, R et al Regulatory Peptides 1995; 58: 149-56, Hupe-Sodmann, K, Goeke, R., Goeke, B et al Peptides 1997; 18: 625-32).

### DEFINITIONS

The term "DPP-IV" as used herein is intended to mean Dipeptidyl peptidase IV (EC 3.4.14.5; DPP-IV), also known as CD26. DPP-IV cleaves a dipeptide from the N terminus of a polypeptide chain containing a proline or alanine residue in the penultimate position.

The term "NEP" as used herein is intended to mean Neutral Endopeptidase (E.C. 3.4.24.11; NEP).

The term "ACE" as used herein is intended to mean Angiotensin Converting Enzyme (E.C. 3.4.15.1; ACE).

The term "inhibitor" is intended to indicate a molecule that exhibits inhibition of the enzymatic activity of the indicated enzyme, such as from 1-100% inhibition. The enzymatic activity of DPP-IV may be measured in the assay as described in the section "Methods for measuring the activity of compounds which inhibit the enzymatic activity of CD26/DPP-IV".

In the present context "an inhibitor" is also intended to comprise active metabolites and prodrugs thereof, such as active metabolites and prodrugs of the inhibitors.

The term "hypoglycaemia" is a condition of low blood sugar levels, for example a blood sugar level below 4 mmol/l, such as below 3 mmol/l, for example below 2.5 mmol/l, such as below 2 mmol/l.

By the term "treatment" is understood the management and care of a patient for the purpose of combating the disease, condition, or disorder.

The term "beta cell degeneration" is intended to mean loss of beta cell function, beta cell dysfunction, and death of beta cells, such as necrosis or apoptosis of beta cells.

The term "Impaired Glucose Tolerance" (IGT) is intended to mean a condition indicated by a 2-h postload glucose (2-h PG) between 7.8 mmol/l and 11.1 mmol/l in an Oral Glucose Tolerance Test (OGTT) using a glucose load containing the equivalent of 75 g anhydrous glucose dissolved in water.

The term "Impaired Fasting Glucose" (IFG) is intended to mean a condition indicated by a Fasting Plasma Glucose (FPG) between 6.1 mmol/l and 7.0 mmol/l, where fasting is defined as no caloric intake for at least 8 hours.

The term "non-insulin demanding type 2 diabetes" is intended to mean a condition where the individual has insulin resistance, insulin deficiency and either a FPG of more than 7.0 mmol/l or a 2-h PG of more than 11.1 mmol/l when untreated, and where normoglycemia can be achieved without insulin injections.

The term "insulin-demanding type 2 diabetes" is intended to mean a condition where the individual has insulin resistance, insulin deficiency and either a FPG of more than 7.0 mmol/l or a 2-h PG of more than 11.1 mmol/l when untreated, and where normoglycemia can only be achieved with insulin injections.

The term "C₁-C₁₀ alkyl" as used herein, alone or in combination, refers to a straight or branched, saturated hydrocarbon chain having from 1-10 carbon atoms such as but not limited to e.g. methyl, ethyl, n-propyl, isopropyl, n-butyl, sec. Butyl, isobutyl, tert. Butyl, n-pentyl, 2-methylbutyl, 3-methylbutyl, n-hexyl, 4-methylpentyl, neopentyl, 2,2-dimethylpropyl and the like.

The term "C₂-C₁₀-alkenyl" used herein, alone or in combination, refers to a straight or branched, unsaturated hydrocarbon chain having from 2-10 carbon atoms and at least one double bond such as but not limited to vinyl, 1-propenyl, allyl, isopropenyl, n-butenyl, n-pentenyl and n-hexenyl and the like.

The term "C₂-C₁₀ alkynyl" as used herein, alone or in combination, refers to an unsaturated hydrocarbon chain having from 2-10 carbon atoms and at least one triple bond such as but not limited to -C≡CH, -C≡CCH₃, -CH₂C≡CH, -CH₂-CH₂-C≡CH, -CH(CH₃) C≡CH and the like. The term "C₁₋₁₀-alkoxy" as used herein, alone or in combination is intended to include those C_{1-*10}-alkyl groups of the designated length in either a linear or branched or cyclic configuration linked through an ether oxygen having its free valence bond from the ether oxygen. Examples of linear alkoxy groups are methoxy, ethoxy, propoxy, butoxy, pentoxy and hexoxy. Examples of branched alkoxy are isopropoxy, sec-butoxy, tert-butoxy, isopentoxy and isohexoxy. Example of cyclic alkoxy are cyclopropyloxy, cyclobutyloxy, cyclopentyloxy and cyclohexyloxy.

The term "C₃-C₁₀ cycloalkyl" as used herein refers to a radical of one or more saturated cyclic hydrocarbon having from 3-10 carbon atoms such as but not limited to cyclopropyl, cyclobutyl, cyclopentyl, cyclohexyl, adamantyl and the like.

The term "C₅-C₁₀ cycloalkenyl" as used herein refers to a radical of one or more cyclic hydrocarbon having at least one double bond having from 5-10 carbon atoms such as but not limited to cyclopentenyl, cyclohexenyl and the like

The term "C₂-C₆ cycloheteroalkyl" as used herein refers to a radical of totally saturated heterocycle like a cyclic hydrocarbon containing one or more heteroatoms selected from nitrogen, oxygen and sulphur independently in the cycle such as pyrrolidine (1- pyrrolidine; 2- pyrrolidine; 3- pyrrolidine; 4- pyrrolidine; 5- pyrrolidine); pyrazolidine (1- pyrazolidine; 2- pyrazolidine; 3- pyrazolidine; 4-pyrazolidine; 5-pyrazolidine); imidazolidine (1- imidazolidine; 2-imidazolidine; 3- imidazolidine; 4- imidazolidine; 5- imidazolidine); thiazolidine (2- thiazolidine; 3- thiazolidine; 4- thiazolidine; 5- thiazolidine); piperidine (1- piperidine; 2-piperidine; 3- piperidine; 4- piperidine; 5- piperidine; 6- piperidine); piperazine (1- piperazine; 2- piperazine; 3- piperazine; 4- piperazine; 5- piperazine; 6- piperazine); morpholine (2- morpholine; 3- morpholine; 4- morpholine; 5- morpholine; 6- morpholine); thiomorpholine (2-thiomorpholine; 3- thiomorpholine; 4- thiomorpholine; 5- thiomorpholine; 6- thiomorpholine); 1,2-oxathiolane (3-(1,2-oxathiolane); 4-(1,2-oxathiolane); 5-(1,2-oxathiolane); 1,3-dioxolane (2-(1,3-dioxolane); 4-(1,3-dioxolane); 5-(1,3-dioxolane); tetrahydropyrane; (2-tetrahydropyrane; 3-tetrahydropyrane; 4-tetrahydropyrane; 5-tetrahydropyrane; 6-tetrahydropyrane); hexahydropyridazine (1-(hexahydropyridazine); 2-(hexahydropyridazine); 3-(hexahydropyridazine); 4-(hexahydropyridazine); 5-(hexahydropyridazine); 6-(hexahydropyridazine)).

The term "aryl" as used herein includes carbocyclic aromatic ring systems. Aryl is also intended to include the partially hydrogenated derivatives of the carbocyclic systems.

The term "heteroaryl" as used herein includes heterocyclic unsaturated ring systems containing one or more heteroatoms selected from nitrogen, oxygen and sulphur such as furyl, thienyl, pyrrolyl, heteroaryl is also intended to include the partially hydrogenated derivatives of the heterocyclic systems enumerated below.

The terms "aryl" and "heteroaryl" as used herein refers to an aryl which can be optionally substituted or a heteroaryl which can be optionally substituted and includes phenyl, biphenyl, indenyl, naphthyl (1-naphthyl, 2-naphthyl), N-hydroxytetrazolyl, N-hydroxytriazolyl, N-hydroxyimidazolyl, anthracenyl (1-anthracenyl, 2-anthracenyl, 3-anthracenyl), thiophenyl (2-thienyl, 3-thienyl), furyl (2-furyl, 3-furyl), indolyl, oxadiazolyl, isoxazolyl, quinazolinyl, fluorenyl, xanthenyl, isoindanyl, benzhydryl, acridinyl, thiazolyl, pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4-triazol-3-yl), oxazolyl (2-oxazolyl, 4-oxazolyl, 5-oxazolyl), thiazolyl (2-thiazolyl, 4-thiazolyl, 5-thiazolyl), pyridyl (2-pyridyl, 3-pyridyl, 4-pyridyl), pyrimidinyl (2-pyrimidinyl, 4-pyrimidinyl, 5-pyrimidinyl, 6-pyrimidinyl), pyrazinyl, pyridazinyl (3- pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4-isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-dihydro-benzo[b]furanyl), 3-(2,3-dihydro-benzo[b]furanyl), 4-(2,3-dihydro-benzo[b]furanyl), 5-(2,3-dihydro-benzo[b]furanyl), 6-(2,3-dihydro-benzo[b]furanyl), 7-(2,3-dihydro-benzo[b]furanyl), benzo[b]thiophenyl (2-benzo[b]thiophenyl, 3-benzo[b]thiophenyl, 4-benzo[b]thiophenyl, 5-benzo[b]thiophenyl, 6-benzo[b]thiophenyl, 7-benzo[b]thiophenyl), 2,3-dihydro-benzo[b]thiophenyl (2-(2,3-dihydro-benzo[b]thiophenyl), 3-(2,3-dihydro-benzo[b]thiophenyl), 4-(2,3-dihydro-benzo[b]thiophenyl), 5-(2,3-dihydro-benzo[b]thiophenyl), 6-(2,3-dihydro-benzo[b]thiophenyl), 7-(2,3-dihydro-benzo[b]thiophenyl), indolyl (1-indolyl, 2-indolyl, 3-indolyl, 4-indolyl, 5-indolyl, 6-indolyl, 7-indolyl), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benzoxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), carbazolyl (1-carbazolyl, 2-carbazolyl, 3-carbazolyl, 4-carbazolyl), 5H-dibenz[b,f]azepine (5H-dibenz[b,f]azepin-1-yl, 5H-dibenz[b,f]azepine-2-yl, 5H-dibenz[b,f]azepine-3-yl, 5H-dibenz[b,f]azepine-4-yl, 5H-dibenz[b,f]azepine-5-yl), 10,11-dihydro-5H-dibenz[b,f]azepine (10,11-dihydro-5H-dibenz[b,f]azepine-1-yl, 10,11-dihydro-5H-dibenz[b,f]azepine-2-yl, 10,11-dihydro-5H-dibenz[b,f]azepine-3-yl, 10,11-dihydro-5H-dibenz[b,f]azepine-4-yl, 10,11-dihydro-5H-dibenz[b,f]azepine-5-yl).

The term halogen as used herein refers to fluorine, chlorine, bromine or iodine.

The term "antidiabetic" is meant to encompass any substance or pharmaceutical composition, which can be used for prophylactic, ameliorative or curative treatment of diabetes mellitus, wherein diabetes mellitus may be any type of diabetes.

Suitable antidiabetics comprise insulin, GLP-1 derivatives such as those disclosed in WO 98/08871 (Novo Nordisk A/S), as well as orally active hypoglycemic agents.

In one preferred embodiment the antidiabetic is insulin or an analogue thereof or a derivative thereof. More preferably the antidiabetic is human insulin or an analogue thereof or a derivative thereof. However, porcine insulin is also an insulin species, which may be employed with the present invention. Preferably, porcine insulin is highly purified naturally produced porcine insulin.

Human insulin could be naturally produced insulin, preferably however human insulin is recombinantly produced. Recombinant human insulin may be produced in any suitable host cell for example the host cells may be bacterial, fungal (including yeast), insect, animal or plant cells. Preferably, the host cells are yeast cells or bacterial cells such as for example *E*. *coli.*

Preferably, the analogue of human insulin is a rapid-acting analogue. For example the analogue may be selected from the group consisting of AspB28 human insulin and

LysB28ProB29 human insulin.

In one preferred embodiment the derivative is human insulin or an analogue thereof containing a C₆ to C₄₀ lipophilic substituent in position B29. Preferably, the derivative may be selected from the group consisting of B29-N^{ε}-myristoyl-des(B30) human insulin, B29-N^{ε}-paimitoyi-des(B30) human insulin, B29-N^{ε}-myristoyl human insulin, B29-N^{ε}-palmitoyl human insulin, B28-N^{ε}-myristoyl Lys^{B28} Pro^{B29} human insulin, B28-N^{ε}-paimitoyl Lys^{B28} Pro^{B29} human insulin, B30-N^{ε}-myristoyl- Thr^{B29}Lys^{B30} human insulin, B30-N^{ε}-palmitoyl-Thr^{B29}Lys^{B30} human insulin, B29-N^{ε}-(N-palmitoyl-y-glutamyl)-des(B30) human insulin, B29-N^{ε}-(N-lithocholyl-γ-glutamyl)-des(B30) human insulin, B29-N^{ε}-(ω-carboxyheptadecanoyl)-des(B30) human insulin and B29-N^{ε}-(ω-carboxyheptadecanoyl) human insulin.

In addition, a variety of different insulin compositions are antidiabetic which should also be considered to fall within the scope of the present invention. For example this includes regular insulin, Semilente® insulin, isophane insulin, insulin zinc suspensions, protamine zinc insulin, and Ultralente® insulin.

Isophane insulin is an isophane mixture of protamine and insulin, wherein a ratio of protamine to insulin is mixed, which is equal to the ratio in a solution made by mixing equal parts of a solution of the two in which all the protamine precipitates and a solution of the two in which all the insulin precipitates.

In one embodiment insulin compositions according to the present invention are characterised by a fast onset of action, while in other embodiments the insulin compositions have a relatively slow onset but show a more or less prolonged action. Fast acting insulin compositions are usually solutions of insulin, while retarded acting insulin compositions can be suspensions containing insulin in crystalline and/or amorphous form precipitated by addition of zinc salts alone or by addition of protamine or by a combination of both. In addition, some compositions have both a fast onset of action and a more prolonged action. Such a composition may be an insulin solution wherein protamine insulin crystals are suspended. Furthermore, compositions obtained by mixing an insulin solution with a suspension composition in the desired ratio are useful with the present invention.

The present invention preferably, may be used in connection with compositions comprising analogues and/or derivatives of human insulin. Thus, the insulin composition according to the invention may comprise one or more fast-acting analogues of human insulin, in particular analogues wherein the amino acid residue at position B28 is Asp, Lys, Leu, Val or Ala and the amino acid residue at position B29 is Lys or Pro; or des(B28-B30), des(B27) or des(B30) human insulin. The insulin analogue is preferably selected from analogues of human insulin wherein the amino acid residue at position B28 is Asp or Lys, and the amino acid residue at position B29 is Lys or Pro. The most preferred analogues are Asp_{B28} human insulin and

Lys_{B28} Pro_{B29} human insulin.

In another embodiment the insulin composition according to the invention comprises an insulin derivative having a protracted profile of action, such an insulin having one or more lipophilic substituents. Lipophilic insulins may be acylated insulins, including those described in WO 95/07931, e.g. human insulin derivatives wherein the ε-amino group of Lys_{B29} contains an acyl substituent which comprises at least 6 carbon atoms.

In another embodiment of the present invention the antidiabetic belongs to the group of antidiabetica which can be administrated orally.

For example, the antidiabetic according to the present invention may be an orally active hypoglycemic agent. Orally active hypoglycemic agents preferably comprise sulfonylureas, biguanides, meglitinides, oxadiazolidinediones, thiazolidinediones, α-glucosidase inhibitors, glucagon antagonists such as those disclosed in WO 99/01423 and WO 00/39088 (Novo Nordisk A/S and Agouron Pharmaceuticals, Inc.), GLP-1 agonists such as those disclosed in WO 00/42026 (Novo Nordisk A/S and Agouron Pharmaceuticals, Inc.), potassium channel openers such as those disclosed in WO 97/26265, WO 99/03861 and WO 00/37474 (Novo Nordisk A/S), insulin sensitizers, PTPase inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents, compounds lowering food intake, PPAR and RXR agonists and agents acting on the ATP-dependent potassium channel of the β-cells.

The group of biguanides decreases the blood sugar levels by inhibition of glucose uptake in the intestine, increase of peripheral glucose uptake and inhibition of glucose synthesis in the liver. The group for example comprises metformin.

The group of sulfonylureas stimulates the β-cells of the pancreas to produce more insulin. The group of sulfonylureas for example comprises glibenclamide, glicazide, acetohexamide, chlorpropamide, glimepiride, glipizide, glyburide, tolazamide and tolbutamide.

Alpha-glucosidase inhibitors may for example be selected from the group consisting of acarbose or miglitol.

Meglitinides may for example be selected from the group consisting of repaglinide, nateglinide or senaglinide.

Thiazolidinedione may for example be selected from the group consisting of pioglitazone, rosiglitazone, troglitazone, ciglitazone and the compounds disclosed in WO 97/41097, WO 97/41119, WO 97/41120, WO 00/41121 and WO 98/45202 (Dr. Reddy's Research Foundation).

Insulin sensitizers may for example be those disclosed in WO 99/19313, WO 00/50414, WO 00/63191, WO 00/63192, WO 00/63193 (Dr. Reddy's Research Foundation) and WO 00/23425, WO 00/23415, WO 00/23451, WO 00/23445, WO 00/23417, WO 00/23416, WO 00/63153, WO 00/63196, WO 00/63209, WO 00/63190 and WO 00/63189 (Novo Nordisk A/S).

Agents acting on the ATP-dependent potassium channel of the β-celles may for example be selected from the group consisting of tolbutamide, glibenclamide, glipizide, glicazide and repaglinide.

Preferably the oral antidiabetic is selected from the group consisting of tolbutamid, pioglitazone, rosiglitazone, glibenclamid, gliclazide, glipizide, acarbose, metformin and repaglinide.

### DESCRIPTION OF THE INVENTION

The present invention demonstrates the possibility of administering inhibitors of the enzymes NEP and DPP-IV to individuals suffering from one or more of the following conditions: Diabetes, hypertension, chronic heart failure and fluid retentive states. Inhibition of the activity of these two enzymes will potentiate the insulin releasing activity of endogenous GLP-1 and other DPP-IV substrates like GIP.

The present invention demonstrates that there is an improved effect of administering both a DPP-IV inhibitor and a NEP inhibitor. Preferably, there is a synergistic effect of administering both a DPP-IV inhibitor and a NEP inhibitor.

Accordingly, the pharmaceutical preparation according to the invention can provide treatment and prevention of one or more condition that may be regulated or normalised via inhibition of DPP-IV and NEP.

The inhibitors of DPP-IV and NEP are administered as a kit-of-parts. The kit-of-parts according to the present invention may be administrated in a manner, so that one or more components of the kit-of-parts are administrated by one route and another one or more components of the kit-of-parts are administrated by another route. By way of example, one component may be administrated orally, whereas another component may be administrated by subcutaneous injection.

Furthermore, the individual compounds of the kit-of-parts according to the present invention may be administered simultaneously, either as separate formulations or combined in a unit dosage form, or they may be administered sequentially.

The compounds according to the invention may also be administered with at least one additional compound.

The dosage requirements will vary with the particular drug composition employed, the route of administration and the particular individual being treated. Ideally, an individual to be treated by the present method will receive a pharmaceutically effective amount of the compound in the maximum tolerated dose, generally no higher than that required before drug resistance develops.

The daily oral dosage regimen will preferably be from about 0.01 to about 80 mg/kg of total body weight. The daily parenteral dosage regimen will preferably be from about 0.001 to about 80 mg/kg of total body weight. The daily topical dosage regimen will preferably be from 0.1 mg to 150 mg, administered one to four, preferably two or three times daily. The daily inhalation dosage regimen will preferably be from about 0.01 mg/kg to about 1 mg/kg per day. It will also be recognized by one of skill in the art that the optimal quantity and spacing of individual dosages of a compound or a pharmaceutically acceptable salt thereof will be determined by the nature and extent of the condition being treated, the form, route and site of administration, and the particular patient being treated, and that such optimums can be determined by conventional techniques. It will also be appreciated by one of skill in the art that the optimal course of treatment, i.e., the number of doses of a compound or a pharmaceutically acceptable salt thereof given per day for a defined number of days, can be ascertained by those skilled in the art using conventional course of treatment determination tests.

The term "unit dosage form" as used herein refers to physically discrete units suitable as unitary dosages for human and animal individuals, each unit containing a predetermined quantity of a compound, alone or in combination with other agents, calculated in an amount sufficient to produce the desired effect in association with a pharmaceutically acceptable diluent, carrier, or vehicle. The specifications for the unit dosage forms of the present invention depend on the particular compound or compounds employed and the effect to be achieved, as well as the pharmacodynamics associated with each compound in the host. The dose administered should be an "effective amount" or an amount necessary to achieve an "effective level" in the individual patient.

Since the "effective level" is used as the preferred endpoint for dosing, the actual dose and schedule can vary, depending on interindividual differences in pharmacokinetics, drug distribution, and metabolism. The "effective level" can be defined, for example, as the blood or tissue level desired in the individual that corresponds to a concentration of one or more compounds according to the invention.

The DPP-IV inhibitor and NEP inhibitor may thus be administered in a regimen consisting of:
- Co-administration of DPP-IV and NEP inhibitors in separate formulations
- Sequential administration of DPP-IV and NEP inhibitors in separate formulations
- Administration of dual inhibitors, i.e. one compound that inhibits both DPP-IV and NEP
- Administration of formulations containing mixtures of DPP-IV inhibitors and NEP inhibitors
- Combinations of any one of the above with other diuretic agents, antidiabetics, other treatments for hypertension, chronic heart failure and fluid retentive states, such as digitalis inotropic agents, sympathomimetic agents, vasodilators, ACE inhibitors, angiotensin II receptor antagonists
- Combinations of any one of the above with other forms of therapy, e.g. diet or exercise
The conditions indicated above may thus be treated and/or prevented by using one or more of these regimens.

It is an objective of the present invention to provide pharmaceutical preparations containing a DPP-IV inhibitor and a NEP inhibitor.

In one embodiment of the invention, the Dipeptidyl Peptidase-IV inhibitor to be applied in the present invention is selected from known inhibitors, e.g. from those disclosed in DD 296075 (Martin-Luther-Universität), WO 91/16339 and WO 93/08259 (New England Medical Centre Hospitals, Inc. and Tufts University School of Medicine), WO 95/15309, WO 01/40180, WO 01/81337 and WO 01/81304 (Ferring B.V.), WO 98/19998, US 6110949, WO 00/34241 and WO 01/96295 (Novartis AG), WO 99/46272 (Fondatech Benelux N.V.), WO 99/61431, WO 99/67278 ,WO 99/67279 and WO 01/14318 (Probiodrug Gesellschaft für Artzneimittelforschung Mbh.), WO 01/55105 (Novo Nordisk A/S) or WO 01/68603 (Bristol-Myers Squibb Co.).

In a preferred embodiment, the Dipeptidyl Peptidase-IV inhibitor to be applied in the present invention is (S)-1-[3-hydroxy-1-adamantyl)amino]acetyl-2-cyano-pyrrolidine or (S)-1-{2-[(5-cyanopyridin-2-yl)amino]ethyl-aminoacetyl}-2-cyano-pyrrolidine.

In another embodiment of the invention, the Dipeptidyl Peptidase-IV inhibitor to be applied in the present invention is a compound of formula I 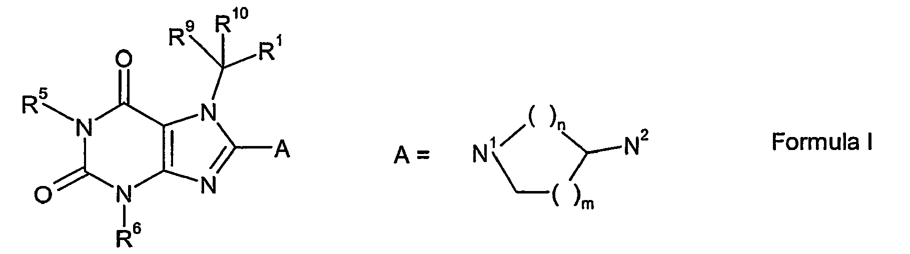 wherein A may be attached at either N¹ or at N² to the purine system and
each n and m is one or two independently
R¹ is aryl optionally substituted with one or more R² independently or heteroaryl optionally substituted with one or more R² independently;
R² is H; C₁-C₇ alkyl; C₂-C₇ alkenyl; C₂-C₇ alkynyl; C₃-C₇ cycloalkyl; C₃-C₇ cycloheteroalkyl; -NHCOR³; -NHSO₂R³; -SR³; -SOR³; -SO₂R³; -OCOR³; -CO₂R⁴; -CON(R⁴)₂; -CSN(R⁴)₂; -NHCON(R⁴)₂; -NHCSN(R⁴)₂; -NHCONNH₂; -SO₂N(R⁴)₂; -OR⁴; cyano; nitro; halogen, wherein each alkyl, alkenyl, alkynyl, cycloalkyl and cycloheteroalkyl is optionally substituted with one or more R³ independently;
R³ is C₁-C₁₀ alkyl; C₂-C₁₀ alkenyl; C₂-C₁₀ alkynyl; C₃-C₇ cycloalkyl; aryl; heteroaryl; OR"; N(R¹¹)₂; SR¹¹, wherein each alkyl, alkenyl, alkynyl, cycloalkyl, aryl and heteroaryl is substituted with one or more R¹¹ independently;
R⁴ is H; C₁-C₁₀ alkyl; C₂-C₁₀ alkenyl; C₂-C₁₀ alkynyl; C₃-C₇ cycloalkyl; C₃-C₇ cycloheteroalkyl; aryl; aryl-C₁-C₅ alkyl; heteroaryl; heteroaryl-C₁-C₅ alkyl, wherein each alkyl, alkenyl, alkynyl, cycloalkyl, cycloheteroalkyl, aryl, aryl-C₁-C₅ alkyl, heteroaryl, and heteroaryl-C₁-C₅ alkyl is substituted with one or more R¹¹ independently;
R⁵ is H; C₁-C₁₀ alkyl; C₂-C₁₀ alkenyl; C₂-C₁₀ alkynyl; C₃-C₇ cycloalkyl; C₃-C₇ cycloalkyl-C₁-C₅ alkyl; C₃-C₇ cycloheteroalkyl; C₃-C₇ cycloheteroalkyl-C₁-C₅ alkyl; aryl; heteroaryl; aryl-C₁-C₅ alkyl; heteroaryl-C₁-C₅alkyl; -OR⁷; -[(CH₂)ₒ-O]ₚ-alkyl, wherein o and p are 1-3 independently, and wherein each alkyl, alkenyl, alkynyl, cycloalkyl, cycloalkyl-C₁-C₅ alkyl, cycloheteroalkyl, C₃-C₇ cycloheteroalkyl-C₁-C₅ alkyl, aryl, aryl-C₁-C₅ alkyl, heteroaryl, aryl-C₁-C₅alkyl, and heteroaryl-C₁-C₅ alkyl is optionally substituted with one or more R⁷ independently;
R⁶ is C₁-C₁₀ alkyl; C₂-C₁₀ alkenyl; C₂-C₁₀ alkynyl; C₃-C₇ cycloalkyl; C₃-C₇ cycloheteroalkyl; aryl; heteroaryl; aryl-C₁-C₅ alkyl; heteroaryl-C₁-C₅ alkyl; C₃-C₇ cycloheteroalkyl-C₁-C₅ alkyl, wherein each alkyl, alkenyl, alkynyl, cycloalkyl, cycloheteroalkyl, C₃-C₇ cycloheteroalkyl-C₁-C₅ alkyl, aryl, aryl-C₁-C₅ alkyl, heteroaryl, and heteroaryl-C₁-C₅ alkyl is optionally substituted with one or more R¹¹ independently;
R⁷ is H; =O; C₁-C₁₀ alkyl; C₂-C₁₀ alkenyl; C₂-C₁₀ alkynyl; C₃-C₇ cycloalkyl; C₃-C₇ cycloheteroalkyl; aryl; heteroaryl, OR¹¹; N(R¹¹)₂; SR¹¹, wherein each alkyl, alkenyl, alkynyl, cycloalkyl, cycloheteroalkyl, aryl, and heteroaryl is optionally substituted with one or more R¹¹ independently;
R⁸ is C₁-C₁₀ alkyl; C₂-C₁₀ alkenyl; C₂-C₁₀ alkynyl; C₃-C₇ cycloalkyl; C₃-C₇ cycloheteroalkyl; aryl; heteroaryl, OR¹¹; N(R¹¹)₂; SR¹¹, wherein each alkyl, alkenyl, alkynyl, cycloalkyl, cycloheteroalkyl, aryl, and heteroaryl is optionally substituted with one or more R¹¹ independently;
R⁹ and R¹⁰ is independently H, C₁-C₁₀ alkyl optionally substituted with one or more R⁸ independently, halogen;
R¹¹ is H; -CF₃; -CCl₃; -OCF₃; -OMe; cyano; halogen; -OH, COMe; -CONH₂; CONHMe; CONMe₂; -NO₂;
If R⁹ and R¹⁰ is C₁-C₁₀ alkyl they may be connected to form a cyclopropyl ring;
if two R⁴ or two R¹¹ are attached to the same nitrogen they may be connected to form a 3- to 7-membered ring;
or a salt thereof with a pharmaceutically acceptable acid or base.

In a further embodiment of the compounds of formula I A is

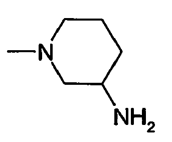

In a further embodiment of the compounds of formula I R¹ is aryl optionally substituted with one or more R² independently.

In a further embodiment of the compounds of formula I R¹ is aryl.

In a further embodiment of the compounds of formula I R¹ is phenyl.

In a further embodiment of the compounds of formula I R₂ is C₁-C₇ alkyl; C₂-C₇ alkynyl; cyano; or halogen, wherein each alkyl and alkynyl is optionally substituted with one or more R³ independently.

In a further embodiment of the compounds of formula I R₂ is C₁-C₇ alkyl; C₂-C₇ alkynyl; cyano; or halogen.

In a further embodiment of the compounds of formula I R₂ is cyano or halogen.

In a further embodiment of the compounds of formula I R³ is C₁-C₁₀ alkyl or aryl, wherein each alkyl or aryl is substituted with one or more R¹¹ independently.

In a further embodiment of the compounds of formula I R³ is C₁-C₁₀ alkyl or aryl.

In a further embodiment of the compounds of formula I R³ is methyl or phenyl.

In a further embodiment of the compounds of formula I R⁴ is H; C₁-C₁₀ alkyl or aryl, wherein each alkyl or aryl is substituted with one or more R¹¹ independently.

In a further embodiment of the compounds of formula I R⁴ is H; C₁-C₁₀ alkyl or aryl.

In a further embodiment of the compounds of formula I R⁴ is H, methyl or phenyl.

In a further embodiment of the compounds of formula I R⁵ is H; C₁-C₁₀ alkyl; aryl-C₁-C₅ alkyl; or heteroaryl-C₁-C₅ alkyl, wherein each alkyl, aryl-C₁-C₅ alkyl and heteroaryl-C₁-C₅ alkyl is optionally substituted with one or more R⁷ independently.

In a further embodiment of the compounds of formula I R⁵ is H or C₁-C₁₀ alkyl optionally substituted with one or more R⁷ independently.

In a further embodiment of the compounds of formula I R⁵ is H or C₁-C₁₀ alkyl.

In a further embodiment of the compounds of formula I R⁵ is H.

In a further embodiment of the compounds of formula I R⁵ is methyl.

In a further embodiment of the compounds of formula I R⁶ is C₁-C₁₀ alkyl; aryl-C₁-C₅alkyl; or heteroaryl-C₁-C₅ alkyl, wherein each alkyl, aryl-C₁-C₅ alkyl and heteroaryl-C₁-C₅ alkyl is optionally substituted with one or more R¹¹ independently.

In a further embodiment of the compounds of formula I R⁶ is C₁-C₁₀ alkyl; aryl-C₁-C₅alkyl; or heteroaryl-C₁-C₅ alkyl.

In a further embodiment of the compounds of formula I R⁶ is C₁-C₁₀ alkyl optionally substituted with one or more R¹¹ independently.

In a further embodiment of the compounds of formula I R⁶ is C₁-C₁₀ alkyl.

In a further embodiment of the compounds of formula I R⁶ is methyl.

In a further embodiment of the compounds of formula I R⁷ is H; =O; aryl; heteroaryl, OR¹¹; N(R¹¹)₂; SR¹¹, wherein each aryl and heteroaryl is optionally substituted with one or more R¹¹ independently.

In a further embodiment of the compounds of formula I R⁷ is H; =O; aryl; or heteroaryl.

In a further embodiment of the compounds of formula I R⁷ is H; =O; OR¹¹; N(R¹¹)₂; or SR¹¹.

In a further embodiment of the compounds of formula I R⁷ is H or =O.

In a further embodiment of the compounds of formula I R⁸ is aryl or heteroaryl, wherein each aryl and heteroaryl is optionally substituted with one or more R¹¹ independently.

In a further embodiment of the compounds of formula I R⁸ is aryl or heteroaryl.

In a further embodiment of the compounds of formula I R⁸ is phenyl.

In a further embodiment of the compounds of formula I R⁹ is H; C₁-C₁₀ alkyl; or halogen.

In a further embodiment of the compounds of formula I R⁹ is H.

In a further embodiment of the compounds of formula I R¹⁰ is H; C₁-C₁₀ alkyl; or halogen.

In a further embodiment of the compounds of formula I R¹⁰ is H.

In a further embodiment of the compounds of formula I R¹¹ is cyano; halogen; -CONHMe; or - CONMe₂.

In a further embodiment of the compounds of formula I R¹¹ is cyano or halogen.

In a further embodiment of the compounds of formula I n is one.

In a further embodiment of the compounds of formula I m is one.

In another embodiment of the invention, the Dipeptidyl Peptidase-IV inhibitor to be applied in the present invention is a compound of formula II 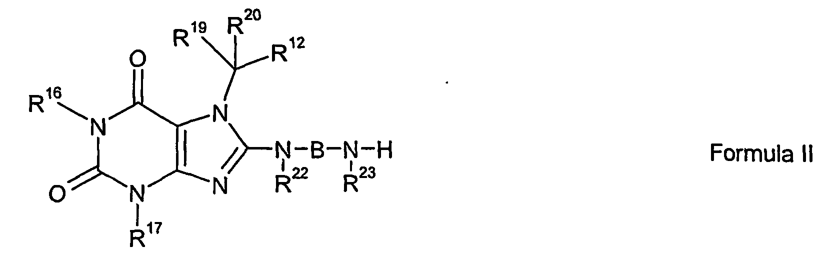 wherein
B is C₂-C₆ alkylene; C₂-C₁₀ alkenylene; C₃-C₇ cycloalkylene; C₃-C₇ cycloheteroalkylene; arylene; heteroarylene; C₁-C₂ alkylene-arylene; arylene-C₁-C₂ alkylene; C₁-C₂ alkylene-arylene-C₁-C₂ alkylene, wherein each alkylene, alkenylene, cycloalkylene, cycloheteroalkylene, arylene, or heteroarylene is optionally substituted with one or more R¹⁴ independently;
R¹² is aryl optionally substituted with one or more R¹³ independently or heteroaryl optionally substituted with one or more R¹³ independently;
R¹³ is H; C₁-C₇ alkyl; C₂-C₇ alkenyl; C₂-C₇ alkynyl; C₃-C₇ cycloalkyl; C₃-C₇ cycloheteroalkyl; -NHCOR¹⁴; -NHSO₂R¹⁴; -SR¹⁴; -SOR¹⁴; -SO₂R¹⁴; -OCOR¹⁴; -CO₂R¹⁵; -CON(R¹⁵)₂; -CSN(R¹⁵)₂; -NHCON(R¹⁵)₂; -NHCSN(R¹⁵)₂; -NHCONNH₂; -SO₂N(R¹⁵)₂; -OR¹⁵; cyano; nitro; halogen, wherein each alkyl, alkenyl, alkynyl, cycloalkyl and cycloheteroalkyl is optionally substituted with one or more R¹⁴ independently;
R¹⁴ is C₁-C₁₀ alkyl; C₂-C₁₀alkenyl; C₂-C₁₀alkynyl; C₃-C₇ cycloalkyl; aryl; heteroaryl; OR²¹; N(R²¹)₂; SR²¹, wherein each alkyl, alkenyl, alkynyl, cycloalkyl, aryl and heteroaryl is optionally substituted with one or more R²¹ independently;
R¹⁵ is H; C₁-C₁₀ alkyl; C₂-C₁₀alkenyl; C₂-C₁₀ alkynyl; C₃-C₇ cycloalkyl; C₃-C₇ cycloheteroalkyl; aryl; aryl-C₁-C₅ alkylene; heteroaryl; heteroaryl-C₁-C₅ alkylene, wherein each alkyl, alkenyl, alkynyl, cycloalkyl, cycloheteroalkyl, aryl, aryl-C₁-C₅ alkylene, heteroaryl, and heteroaryl-C₁-C₅ alkylene is optionally substituted with one or more R²¹ independently;
R¹⁶ is H; C₁-C₁₀ alkyl; C₂-C₁₀alkenyl; C₂-C₁₀ alkynyl; C₃-C₇ cycloalkyl; C₃-C₇ cycloheteroalkyl; aryl; heteroaryl; -OR¹⁸; -[(CH₂)ₒ-O]ₚ-C₁-C₅alkyl, wherein o and p are 1-3 independently, and wherein each alkyl, alkenyl, alkynyl, cycloalkyl, cycloheteroalkyl, aryl, and heteroaryl is optionally substituted with one or more R¹⁸ independently;
R¹⁷ is H; C₁-C₁₀ alkyl; C₂-C₁₀alkenyl; C₂-C₁₀alkynyl; C₃-C₇ cycloalkyl; C₃-C₇ cycloheteroalkyl; aryl; heteroaryl; aryl-C₁-C₅ alkylene; heteroaryl-C₁-C₅ alkylene; C₃-C₇ cycloheteroalkyl-C₁-C₅ alkylene, wherein each alkyl, alkenyl, alkynyl, cycloalkyl, cycloheteroalkyl, C₃-C₇ cycloheteroalkyl-C₁-C₅ alkylene, aryl, aryl-C₁-C₅ alkylene, heteroaryl, aryl-C₁-C₅ alkylene, and heteroaryl-C₁-C₅ alkylene is optionally substituted with one or more R²¹ independently;
R¹⁸ is H; =O; C₁-C₁₀ alkyl; C₂-C₁₀ alkenyl; C₂-C₁₀ alkynyl; C₃-C₇ cycloalkyl; C₃-C₇ cycloheteroalkyl; aryl; heteroaryl, OR²¹; N(R²¹)₂; SR²¹; cyano; hydroxy; halogen; -CF₃; -CCl₃; -OCF₃; or -OCH₃ wherein each alkyl, alkenyl, alkynyl, cycloalkyl, cycloheteroalkyl, aryl, and heteroaryl is optionally substituted with one or more R²¹ independently;
R¹⁹ is H; C₁-C₁₀ alkyl; C₂-C₁₀alkenyl; C₂-C₁₀alkynyl; C₃-C₇ cycloalkyl; C₃-C₇ cycloheteroalkyl; aryl; heteroaryl, OR²¹; N(R²¹)₂; SR²¹, wherein each alkyl, alkenyl, alkynyl, cycloalkyl, cycloheteroalkyl, aryl, and heteroaryl is optionally substituted with one or more R²¹ independently;
R²⁰ is H; C₁-C₁₀ alkyl optionally substituted with one or more R¹⁹ independently; or halogen;
R²¹ is H; -CF₃; -CCl₃; -OCF₃; -OCH₃; cyano; halogen; -OH, -COCH₃; -CONH₂; -CONHCH₃; -CON(CH₃)₂; -NO₂; -SO₂NH₂; or -SO₂N(CH₃)₂;
if two R¹⁵ or two R²¹ are attached to the same nitrogen they may be connected to form a 3- to 7-membered ring;
R²² is H; C₁-C₆ alkyl optionally substituted with one or more R¹⁴ independently;
R²³ is H; C₁-C₆ alkyl optionally substituted with one or more R¹⁴ independently; or If B is C₃-C₇ cycloalkylene or C₃-C₇ cycloheteroalkylene R²³ may be a valence bond between the nitrogen to which R²³ is attached and one of the atoms in the cycloalkylene or cycloheteroalkylene;
or a salt thereof with a pharmaceutically acceptable acid or base.

In another embodiment of the compounds of formula II B is C₂-C₆ alkylene; C₂-C₁₀ alkenylene; C₃-C₇ cycloalkylene; C₃-C₇ cycloheteroalkylene; or arylene, wherein each alkylene, alkenylene, cycloalkylene, cycloheteroalkylene, or arylene is optionally substituted with one or more R¹⁴ independently;

In another embodiment of the compounds of formula II B is C₃-C₇ cycloalkylene optionally substituted with one or more R¹⁴ independently.

In another embodiment of the compounds of formula II B is cyclohexylene optionally substituted with one or more R¹⁴ independently.

In another embodiment of the compounds of formula II B is cyclohexylene.

In another embodiment of the compounds of formula II R¹² is aryl optionally substituted with one or more R¹³ independently.

In another embodiment of the compounds of formula II R¹² is phenyl optionally substituted with one or more R¹³ independently.

In another embodiment of the compounds of formula II R¹³ is C₁-C₇ alkyl; C₂-C₇ alkynyl; cyano; or halogen, wherein each alkyl and alkynyl is optionally substituted with one or more R¹⁴ independently.

In another embodiment of the compounds of formula II R¹³ is C₁-C₇ alkyl; C₂-C₇ alkynyl; cyano; or halogen.

In another embodiment of the compounds of formula II R¹³ is halogen.

In another embodiment of the compounds of formula II R¹⁴ is C₁-C₁₀ alkyl or aryl, wherein each alkyl or aryl is substituted with one or more R²¹ independently.

In another embodiment of the compounds of formula II R¹⁴ is C₁-C₁₀ alkyl or aryl.

In another embodiment of the compounds of formula II R¹⁴ is methyl or phenyl.

In another embodiment of the compounds of formula II R¹⁵ is H; C₁-C₁₀ alkyl or aryl, wherein each alkyl or aryl is substituted with one or more R²¹ independently.

In another embodiment of the compounds of formula II R¹⁵ is H; C₁-C₁₀ alkyl or aryl.

In another embodiment of the compounds of formula II R¹⁵ is H, methyl or phenyl.

In another embodiment of the compounds of formula II R¹⁶ is H; C₁-C₁₀ alkyl; aryl-C₁-C₅ alkylene; or heteroaryl-C₁-C₅ alkylene, wherein each alkyl, aryl-C₁-C₅ alkylene and heteroaryl-C₁-C₅ alkylene is optionally substituted with one or more R¹⁸ independently.

In another embodiment of the compounds of formula II R¹⁶ is H; C₁-C₁₀ alkyl optionally substituted with one or more R¹⁸ independently; or C₂-C₁₀ alkenyl optionally substituted with one or more R¹⁸ independently.

In another embodiment of the compounds of formula II R¹⁶ is H or C₁-C₁₀ alkyl optionally substituted with one or more R¹⁸ independently.

In another embodiment of the compounds of formula II R¹⁶ is H.

In another embodiment of the compounds of formula II R¹⁶ is methyl or ethyl optionally substituted with one or more R¹⁸ independently.

In another embodiment of the compounds of formula II R¹⁷ is C₁-C₁₀ alkyl; aryl-C₁-C₅ alkylene; or heteroaryl-C₁-C₅ alkylene, wherein each alkyl, aryl-C₁-C₅ alkylene and heteroaryl-C₁-C₅ alkylene is optionally substituted with one or more R²¹ independently.

In another embodiment of the compounds of formula II R¹⁷ is C₁-C₁₀ alkyl; aryl-C₁-C₅alkylene; or heteroaryl-C₁-C₅ alkylene.

In another embodiment of the compounds of formula II R¹⁷ is C₁-C₁₀ alkyl optionally substituted with one or more R²¹ independently.

In another embodiment of the compounds of formula II R¹⁷ is C₁-C₁₀ alkyl.

In another embodiment of the compounds of formula II R¹⁷ is methyl or ethyl optionally substituted by one or more R²¹ independently.

In another embodiment of the compounds of formula II R¹⁸ is H; =O; C₁-C₁₀ alkyl; C₃-C₇ cycloalkyl; C₃-C₇ cycloheteroalkyl; aryl; heteroaryl, OR²¹; N(R²¹)₂; SR²¹, wherein each alkyl, cycloalkyl, cycloheteroalkyl, aryl, and heteroaryl is optionally substituted with one or more R²¹ independently.

In another embodiment of the compounds of formula II R¹⁸ is =O; C₃-C₇ cycloalkyl; C₃-C₇ cycloheteroalkyl; aryl; or heteroaryl, wherein each cycloalkyl, cycloheteroalkyl, aryl, and heteroaryl is optionally substituted with one or more R²¹ independently.

In another embodiment of the compounds of formula II R¹⁸ is =O; C₃-C₇ cycloalkyl optionally substituted with one or more R²¹ independently or aryl optionally substituted with one or more R²¹ independently.

In another embodiment of the compounds of formula II R¹⁸ is =O or aryl optionally substituted with one or more R²¹ independently.

In another embodiment of the compounds of formula II R¹⁸ is =O or phenyl optionally substituted by one or more R²¹ independently.

In another embodiment of the compounds of formula II R¹⁹ is aryl or heteroaryl, wherein each aryl and heteroaryl is optionally substituted with one or more R²¹ independently.

In another embodiment of the compounds of formula II R¹⁹ is aryl or heteroaryl.

In another embodiment of the compounds of formula II R¹⁹ is phenyl.

In another embodiment of the compounds of formula II R²⁰ is H; C₁-C₁₀ alkyl; or halogen.

In another embodiment of the compounds of formula II R²⁰ is H.

In another embodiment of the compounds of formula II R²¹ is H; -CF₃; -OH; cyano; halogen; -OCF₃; or -OCH₃.

In another embodiment of the compounds of formula II R²¹ is H; cyano; halogen; or -OCH₃.

In another embodiment of the compounds of formula II R²² is H.

In another embodiment of the compounds of formula II R²³ is H.

In another embodiment of the invention, the Dipeptidyl Peptidase-IV inhibitor to be applied in the present invention is a compound of formula III 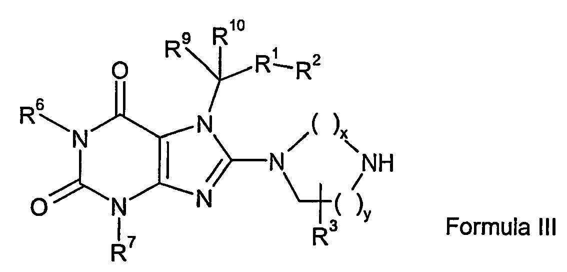 wherein
x and y are one or two independently
R¹ is C=O; C=S; C₁-C₂ alkyl optionally substituted with one or more R⁴ independently; C₂ alkenyl substituted with one or more R⁴ independently; C₂ alkynyl; C₃-C₇ cycloalkyl optionally substituted with one or more R⁴ independently; C₃-C₇ cycloheteroalkyl optionally substituted with one or more R⁴ independently; aryl optionally substituted with one or more R⁴ independently; aryl C₁-C₃ alkyl optionally substituted with one or more R⁴ independently; heteroaryl optionally substituted with one or more R⁴ independently; heteroaryl C₁-C₃ alkyl optionally substituted with one or more R⁴ independently; perhalo C₁-C₁₀ alkyl; perhalo C₁-C₁₀ alkyloxy;
R² is H; C₁-C₇ alkyl optionally substituted with one or more R⁴ independently; C₂-C₇ alkenyl optionally substituted with one or more R⁴ independently; C₂-C₇ alkynyl optionally substituted with one or more R⁴ independently; C₃-C₇ cycloalkyl optionally substituted with one or more R⁴ independently; C₃-C₇ cycloheteroalkyl optionally substituted with one or more R⁴ independently; aryl optionally substituted with one or more R⁴ independently; aryl C₁-C₃ alkyl optionally substituted with one or more R⁴ independently; heteroaryl C₁-C₃ alkyl optionally substituted with one or more R⁴ independently; heteroaryl optionally substituted with one or more R⁴ independently, -SH; -SR⁵; SOR⁵; SO₂R⁵; -CHO; -CH(OR⁵)₂; carboxy; -CO₂R⁴; NHCONNH₂; -NHCSNH₂; -NHCONH₂; -NHCOR⁴; -NHSO₂R⁵; -O-CO-(C₁-C₅) alkyl optionally substituted with one or more R⁴ independently; cyano; nitro; halogen; hydroxy; perhalo C₁-C₇ alkyl; perhalo C₁-C₇ alkyloxy; -SO₂NH₂; -SO₂NH(R⁵); -SO₂(R⁵)₂; -CONH₂; -CSNH₂; -CON₂H₃; -CONH(R⁵); -CON(R⁵)₂; C₁-C₁₀ alkyloxy optionally substituted with R⁴ independently; C₂-C₁₀ alkenyloxy optionally substituted with R⁴; C₂-C₁₀ alkynyloxy optionally substituted with R⁴ independently, aryloxy optionally substituted with R⁴ independently; heteroaryloxy optionally substituted with R⁴ independently;
R³ is H; C₁-C₁₀ alkyl optionally substituted with one or more R⁴ independently; C₂-C₁₀ alkenyl optionally substituted with one or more R⁴ independently; C₂-C₁₀ alkynyl optionally substituted with one or more R⁴ independently; C₃-C₇ cycloalkyl optionally substituted with one or more R⁴ independently; C₃-C₇ cycloheteroalkyl optionally substituted with one or more R⁴ independently; aryl optionally substituted with one or more R⁴ independently; aryl C₁-C₃ alkyl optionally substituted with one or more R⁴ independently; heteroaryl C₁-C₃ alkyl optionally substituted with one or more R⁴ independently; heteroaryl optionally substituted with one or more R⁴ independently; C₁-C₁₀ alkyl-NH(CH₂)₁₋₄NH-aryl optionally substituted with one or more R⁴ independently; C₁-C₁₀ alkyl-NH(CH₂)₁₋₄NH-heteroaryl optionally substituted with one or more R⁴ independently; C₁-C₁₀ alkyl-O(CH₂)₁₋₄NH-aryl optionally substituted with one or more R⁴ independently; C₁-C₁₀ alkyl-O(CH₂)₁₋₄NH-heteroaryl optionally substituted with one or more R⁴ independently; C₁-C₁₀ alkyl-O(CH₂)₁₋₄O-aryl optionally substituted with one or more R⁴ independently; C₁-C₁₀ alkyl-O(CH₂)₁₋₄O-heteroaryl optionally substituted with one or more R⁴ independently; C₁-C₁₀ alkyl-S(CH₂)₁₋₄NH-aryl optionally substituted with one or more R⁴ independently; C₁-C₁₀ alkyl-S(CH₂)₁₋₄NH-heteroaryl optionally substituted with one or more R⁴ independently; C₁-C₁₀ alkyl-S(CH₂)₁₋₄S-aryl optionally substituted with one or more R⁴ independently; C₁-C₁₀ alkyl-S(CH₂)₁₋₄S-heteroaryl optionally substituted with one or more R⁴ independently; C₁-C₁₀ alkyl-O-C₁-C₅alkyl optionally substituted with one or more R⁴; -NHCOR⁴; -NHSO₂R⁵; -O-CO-(C₁-C₅ ) alkyl optionally substituted with one or more R⁴ independently; -SH; -SR⁵; -SOR⁵; -SO₂R⁵; -CHO; -CH(OR⁵)₂; carboxy; cyano; nitro; halogen; hydroxy; -SO₂NH₂; -SO₂NH(R⁵); -SO₂N(R⁵)₂; -CONH₂; -CONH(R⁵); -CON(R⁵)₂; -CSNH₂; -CONHNH₂; -CO2R⁴; -NHCNHNH₂; -NHCSNH₂; -NHCONH₂;
R⁴ is C₁-C₁₀ alkyl optionally substituted with one or more R⁸ independently; C₂-C₁₀ alkenyl optionally substituted with one or more R⁸ independently; C₂-C₁₀ alkynyl optionally substituted with one or more R⁸ independently; C₃-C₇ cycloalkyl optionally substituted with one or more R⁸ independently; C₃-C₇ cycloheteroalkyl optionally substituted with one or more R⁸ independently; aryl optionally substituted with one or more R⁸ independently; heteroaryl optionally substituted with one or more R⁸ independently; amino; amino substituted with one or more C₁-C₁₀ alkyl optionally substituted with one or more R⁸; amino substituted with one or two aryl optionally substituted with one or more R⁸ independently; heteroaryl optionally substituted with one or more R⁸ independently; =O; =S; -CO-R5; -COOR5; -O-CO-(C₁-C₅) alkyl optionally substituted with one or more R⁸ independently; NH(CH₂)₁₋₄NH-aryl; NH(CH₂)₁₋₄NH-heteroaryl; -NHCOR⁵; -SOR⁵; SO₂R⁵; carboxy; cyano; N-hydroxyimino; nitro; halogen; hydroxy; perhalo C₁-C₁₀ alkyl; perhalo C₁-C₁₀ alkyloxy; -SH; -SR⁵; -SO₃H; -SO₃R⁵; -SO₂R⁵; -SO₂NH₂; -SO₂NH(R⁵); -SO₂N(R⁵)₂; -CONH₂; -CONH(R⁵); -CON(R⁵)₂; C₁-C₁₀ alkyloxy optionally substituted with one or more R⁸ independently; C₂-C₁₀ alkenyloxy optionally substituted with one or more R⁸ independently; C₂-C₁₀ alkynyloxy optionally substituted with one or more R⁸ independently; aryloxy optionally substituted with one or more R⁸ independently; heteroaryloxy optionally substituted with one or more R⁸ independently;
and two R⁴ attached to the same carbon atom may form a spiroheterocyclic system, preferably hydantoin; thiohydantoin; oxazolidine-2,5-dione;
R⁵ is C₁-C₁₀ alkyl optionally substituted with one or more R⁸ independently; C₂-C₁₀ alkenyl optionally substituted with one or more R⁸ independently; C₂-C₁₀ alkynyl optionally substituted with one or more R⁸ independently; C₃-C₇ cycloalkyl optionally substituted with one or more R⁸ independently; C₃-C₇ cycloheteroalkyl optionally substituted with one or more R⁸ independently; aryl optionally substituted with one or more R⁸ independently; aryl C₁-C₅ alkyl optionally substituted with one or more R⁸ independently; heteroaryl optionally substituted with one or more R⁸ independently; heteroaryl C₁-C₅ alkyl optionally substituted with one or more R⁸ independently;
R⁶ is H; C₁-C₁₀ alkyl optionally substituted with one or more R⁴ independently; C₂-C₁₀alkenyl optionally substituted with one or more R⁴ independently; C₂-C₁₀ alkynyl optionally substituted with one or more R⁴ independently; C₃-C₇ cycloalkyl optionally substituted with one or more R⁴ independently; C₃-C₇ cycloheteroalkyl optionally substituted with one or more R⁴ independently; aryl optionally substituted with one or more R⁴ independently; heteroaryl optionally substituted with one or more R⁴ independently;
R⁷ is H; C₁-C₁₀ alkyl optionally substituted with one or more R⁴ independently; C₂-C₁₀ alkenyl optionally substituted with one or more R⁴ independently; C₂-C₁₀ alkynyl optionally substituted with one or more R⁴ independently; C₃-C₇ cycloalkyl optionally substituted with one or more R⁴ independently; C₃-C₇ cycloheteroalkyl optionally substituted with one or more R⁴ independently; aryl optionally substituted with one or more R⁴ independently; heteroaryl optionally substituted with one or more R⁴ independently;
R⁸ is H, amidoxime; nitro, tetrazole; pentafluorophenyl; -CH₂OH; -CHO; -C(OCH₃)₂; -COCH₃; -CF₃; -CCl₃; -OCF₃; -OCH₃; -CN; -CO₂H; -CO₂CH₃; -CONH₂; -CSNH₂; -CON₂H₃; -SO₃H; -SO₂NH₂; -SO₂NHCH₃; -SO₂N(CH₃)₂; -SO₂ (1-piperazinyl);-SO₂ (4-methylpiperazin-1-yl); -SO₂ (pyrrolidin-1-yl); -SO₂ (piperidin-1-yl); -SO₂ (morpholin-4-yl); N-hydroxyimino; -NH₂; -NHCH₃; -N(CH₃)₂; -NHCNHNH₂; -NHCNHNHCH₃; -NHCSNH₂; -NHCSNHCH₃; -NHCONH₂; -NHCONHCH₃; -NHCOCH₃; -NHSO₂CH₃; piperazinyl; morhpolin-4-yl; thiomorpholin-4-yl; pyrrolidin-1-yl; piperidin-1-yl; halogen; -OH; -SH; -SCH₃; -aminoacetyl; -OPO₃H; -OPO₂OCH₃; -PO₃H₂; -PO(OCH₃)₂; PO(OH)(OCH₃);
R⁹ is H; halogen; C₁-C₁₀ alkyl optionally substituted with one or more R⁴ independently
R¹⁰ is H; halogen;
or, R⁹ and R¹⁰ may be connected to form a cyclopropyl ring;
or a salt thereof with a pharmaceutically acceptable acid or base;
with the exception of the following compounds:
1,3-dimethyl-7-(2-oxo-propyl) -8-piperazin-1-yl-3,7-dihydro-purine-2,6-dione, 1,3,1',3',7'-pentamethyl-8-piperazin-1-yl-3,7,3',7'-tetrahydro-7,8'-methanediyl-bis-purine-2,6-dione,
3,4,5-trimethoxy-benzoic acid 2-(1,3-dimethyl-2,6-dioxo-8-piperazin-1-yl-1,2,3,6-tetrahydro-purin-7-yl) -ethyl ester,
7-[2-Hydroxy-3-(4-methoxy-phenoxy) -propyl]-3-methyl-8-piperazin-1-yl-3,7-dihydro-purine-2,6-dione,
7-[2-hydroxy-2-(4-nitro-phenyl) -ethyl]-3-methyl-8-piperazin-1-yl-3,7,8,9-tetrahydro-purine-2,6-dione,
7-Benzyl-3-methyl-8-piperazin-1-yl-3,7-dihydro-purine-2,6-dione,
7-(4-Chloro-benzyl) -3-methyl-8-piperazin-1-yl-3,7-dihydro-purine-2,6-dione,
7-(2-Chloro-benzyl) -3-methyl-8-piperazin-1-yl-3,7-dihydro-purine-2,6-dione,
7-Ethyl-3-methyl-8-piperazin-1-yl-3,7-dihydro-purine-2,6-dione,
3-Methyl-8-piperazin-1-yl-1,7-dipropyl-3,7-dihydro-purine-2,6-dione,
3-Methyl-7-(3-methyl-butyl) -8-piperazin-1-yl-3,7-dihydro-purine-2,6-dione,
7-Butyl-3-methyl-8-piperazin-1-yl-3,7-dihydro-purine-2,6-dione,
3-Methyl-7-(3-phenyl-propyl) -8-piperazin-1-yl-3,7-dihydro-purine-2,6-dione,
7-But-2-enyl-3-methyl-8-piperazin-1-yl-3,7-dihydro-purine-2,6-dione,
7-(3-Chloro-but-2-enyl) -3-methyl-8-piperazin-1-yl-3,7-dihydro-purine-2,6-dione,
7-Heptyl-3-methyl-8-piperazin-1-yl-3,7-dihydro-purine-2,6-dione,
3-Methyl-7-(1-phenyl-ethyl) -8-piperazin-1-yl-3,7-dihydro-purine-2,6-dione,
3-Methyl-7-(3-methyl-benzyl) -8-piperazin-1-yl-3,7-dihydro-purine-2,6-dione,
3-Methyl-7-propyl-8-piperazin-1-yl-3,7-dihydro-purine-2,6-dione, and
3-Methyl-7-pentyl-8-piperazin-1-yl-3,7-dihydro-purine-2,6-dione.

In another embodiment of the compounds of formula III, R¹ is C₂ alkenyl optionally substituted with one or more R⁴ independently or aryl-C₁-C₃ alkyl optionally substituted with one or more R⁴ independently.

In another embodiment of the compounds of formula III, R² is H, C₁-C₇ alkyl optionally substituted with one or more R⁴ independently, cyano, nitro, or halogen.

In another embodiment of the compounds of formula III, R⁴ is C₁-C₁₀ alkyl optionally substituted with one or more R⁸ independently; C₂-C₁₀ alkenyl optionally substituted with one or more R⁸ independently; C₂-C₁₀ alkynyl optionally substituted with one or more R⁸ independently; C₃-C₇ cycloalkyl optionally substituted with one or more R⁸ independently; aryl optionally substituted with one or more R⁸ independently; heteroaryl optionally substituted with one or more R⁸ independently; amino; amino substituted with one or more C₁-C₁₀ alkyl optionally substituted with one or more R⁸; amino substituted with one or two aryl optionally substituted with one or more R⁸ independently; heteroaryl optionally substituted with one or more R⁸ independently; =O; =S; -CO-R⁵; -COOR⁵, carboxy; cyano; nitro; halogen; hydroxy; -SH; -SR⁵; -CONH₂; -CONH(R⁵); -CON(R⁵)₂; C₁-C₁₀ alkyloxy optionally substituted with one or more R⁸ independently; C₂-C₁₀ alkenyloxy optionally substituted with one or more R⁸ independently; aryloxy optionally substituted with one or more R⁸ independently; heteroaryloxy optionally substituted with one or more R⁸ independently;
and two R⁴ attached to the same carbon atom may form a spiroheterocyclic system, preferably hydantoin; thiohydantoin; oxazolidine-2,5-dione

In another embodiment of the compounds of formula III, R⁴ is C₁-C₁₀ alkyl optionally substituted with one or more R⁸ independently; C₂-C₁₀ alkenyl optionally substituted with one or more R⁸ independently; C₂-C₁₀ alkynyl optionally substituted with one or more R⁸ independently; C₃-C₇ cycloalkyl optionally substituted with one or more R⁸ independently; aryl optionally substituted with one or more R⁸ independently; heteroaryl optionally substituted with one or more R⁸ independently; amino; =O; =S; -CO-R⁵; -COOR⁵, carboxy; cyano; nitro; halogen; hydroxy; -SH; -SR⁵; -CONH₂; -CONH(R⁵); -CON(R⁵)₂; C₁-C₁₀ alkyloxy optionally substituted with one or more R⁸ independently.

In another embodiment of the compounds of formula III, R⁴ is C₁-C₁₀ alkyl optionally substituted with one or more R⁸ independently; C₂-C₁₀ alkenyl optionally substituted with one or more R⁸ independently; C₂-C₁₀ alkynyl optionally substituted with one or more R⁸ independently; C₃-C₇ cycloalkyl optionally substituted with one or more R⁸ independently; phenyl optionally substituted with one or more R⁸ independently; amino; =O; =S; -CO-R⁵; -COOR⁵, carboxy; cyano; nitro; halogen; hydroxy; -SH; -SR⁵; -CONH₂; -CONH(R⁵); -CON(R⁵)₂.

In another embodiment of the compounds of formula III, R⁵ is C₁-C₁₀ alkyl optionally substituted with one or more R⁸ independently; C₂-C₁₀ alkenyl optionally substituted with one or more R⁸ independently; C₃-C₇ cycloalkyl optionally substituted with one or more R⁸ independently; aryl optionally substituted with one or more R⁸ independently; aryl C₁-C₅ alkyl optionally substituted with one or more R⁸ independently; heteroaryl optionally substituted with one or more R⁸ independently; heteroaryl C₁-C₅ alkyl optionally substituted with one or more R⁸ independently

In another embodiment of the compounds of formula III, R⁶ is H; C₁-C₁₀ alkyl optionally substituted with one or more R⁴ independently; C₂-C₁₀ alkenyl optionally substituted with one or more R⁴ independently; C₃-C₇ cycloalkyl optionally substituted with one or more R⁴ independently; phenyl optionally substituted with one or more R⁴ independently.

In another embodiment of the compounds of formula III, R⁷ is H; C₁-C₁₀ alkyl optionally substituted with one or more R⁴ independently; C₂-C₁₀ alkenyl optionally substituted with one or more R⁴ independently; C₃-C₇ cycloalkyl optionally substituted with one or more R⁴ independently; phenyl optionally substituted with one or more R⁴ independently.

In another embodiment of the compounds of formula III, R⁸ is H, nitro, tetrazole; -CH₂OH; - CHO; -CF₃; -OCF₃; -CN; -CO₂H; -NH₂; halogen; -OH; -SH; -SCH₃.

In another embodiment of the compounds of formula III, R⁹ is H

In another embodiment of the compounds of formula III, R¹⁰ is H

The following compounds are preferred:
2-(8-(3-Aminopiperidin-1-yl)-1,3-dimethyl-2,6-dioxo-1,2,3,6-tetrahydropurin-7-ylmethyl)benzonitrile
8-(3-Aminopyrrolidin-1-yl)-7-benzyl-1,3-dimethyl-3,7-dihydropurine-2,6-dione
(S) 8-(3-Aminopyrrolidin-1-yl)-7-benzyl-1,3-dimethyl-3,7-dihydropurine-2,6-dione
2-(8-(3-Aminopyrrolidin-1-yl)-1,3-dimethyl-2,6-dioxo-1,2,3,6-tetrahydropurin-7-ylmethyl)benzonitrile
8-(3-Aminopyrrolidin-1-yl)-7-(2-iodobenzyl)-1,3-dimethyl-3,7-dihydropurine-2,6-dione
8-(3-Aminoazepan-1-yl)-7-benzyl-1,3-dimethyl-3,7-dihydropurine-2,6-dione
(S) 8-(3-Aminopyrrolidin-1-yl)-7-(2-iodobenzyl)-1,3-dimethyl-3,7-dihydropurine-2,6-dione
(S) 2-(8-(3-Aminopyrrolidin-1-yl)-1,3-dimethyl-2,6-dioxo-1,2,3,6-tetrahydropurin-7-ylmethyl)benzonitrile
8-(3-Aminopiperidin-1-yl)-7-(2-iodobenzyl)-1,3-dimethyl-3,7-dihydropurine-2,6-dione
8-(3-Aminopiperidin-1-yl)-7-(2-bromobenzyl)-1,3-dimethyl-3,7-dihydropurine-2,6-dione
(R) 8-(3-Aminopyrrolidin-1-yl)-7-(2-bromobenzyl)-1,3-dimethyl-3,7-dihydropurine-2,6-dione
(S) 8-(3-Aminopyrrolidin-1-yl)-7-(2-bromobenzyl)-1,3-dimethyl-3,7-dihydropurine-2,6-dione
(R) 8-(3-Aminopyrrofidin-1-yl)-7-benzyl-1,3-dimethyl-3,7-dihydropurine-2,6-dione
(R) 2-(8-(3-Aminopyrrolidin-1-yl)-1,3-dimethyl-2,6-dioxo-1,2,3,6-tetrahydropurin-7-ylmethyl)benzonitrile
(R) 8-(3-Aminopyrrolidin-1-yl)-7-(2-iodobenzyl)-1,3-dimethyl-3,7-dihydropurine-2,6-dione.
*Cis*-8-(2-Aminocyclohexylamino)-7-benzyl-3-methyl-1-(2-oxo-2-phenylethyl)-3,7-dihydropurine-2,6-dione
*Cis*-8-(2-Aminocyclohexylamino)-7-(2-chlorobenzyl)-1-(2-hydroxy-2-phenylethyl)-3-methyl-3,7-dihydropurine-2,6-dione
*Trans*-8-(2-(S)-Amino-cyclohexyl-(S)-amino)-7-(2-iodo-benzyl)-3-methyl-3,7-dihydro-purine-2,6-dione
*Trans*-8-(2-(R)-Amino-cyclohexyl-(R)-amino)-7-(2-iodo-benzyl)-3-methyl-3,7-dihydro-purine-2,6-dione
*Cis*-8-(2-Amino-cyclohexylamino)-7-(2-iodo-benzyl)-3-methyl-3,7-dihydro-purine-2,6-dione
*Trans*-8-(2-(S)-Amino-cyclohexyl-(S)-amino)-7-biphenyl-2-ylmethyl-3-methyl-3,7-dihydro-purine-2,6-dione
*Cis*-8-(2-Amino-cyclohexylamino)-7-biphenyl-2-ylmethyl-3-methyl-3,7-dihydro-purine-2,6-dione
*Cis*-8-(2-(S)-Amino-cyclohexyl-(S)-amino)-7-(2-bromo-benzyl)-3-methyl-3,7-dihydro-purine-2,6-dione
*Cis*-8-(2-Amino-cyclohexylamino)-7-(2-bromo-benzyl)-3-methyl-3,7-dihydro-purine-2,6-dione
*Trans*-8-(2-(S)-Amino-cyclohexyl-(S)-amino)-7-(2-chloro-benzyl)-3-methyl-3,7-dihydro-purine-2,6-dione
*Trans*-8-(2-(R)-Amino-cyclohexyl-(R)-amino)-7-(2-chloro-benzyl)-3-methyl-3,7-dihydro-purine-2,6-dione
*Cis*-8-(2-Amino-cyclohexylamino)-7-(2-chloro-benzyl)-3-methyl-3,7-dihydro-purine-2,6-dione
*Cis*-8-(2-Amino-cyclohexylamino)-1,7-bis-(2-chloro-benzyl)-3-methyl-3,7-dihydro-purine-2,6-dione
*Cis*-2-[8-(2-Amino-cyclohexylamino)-7-(2-chloro-benzyl)-3-methyl-2,6-dioxo-2,3,6,7-tetrahydro-purin-1-ylmethyl]-benzonitrile
*Cis*-8-(2-Amino-cyclohexylamino)-7-(2-chloro-benzyl)-3-methyl-1-(2-oxo-2-phenyl-ethyl)-3,7-dihydro-purine-2,6-dione
*Cis*-8-(2-Amino-cyclohexylamino)-7-(2-chloro-benzyl)-3-methyl-1-phenethyl-3,7-dihydro-purine-2,6-dione
*Cis*-8-(2-Amino-cyclohexylamino)-7-(2-bromo-benzyl)-1-(2-chloro-benzyl)-3-methyl-3,7-dihydro-purine-2,6-dione
*Cis*-2-[8-(2-Amino-cyclohexylamino)-7-(2-bromo-benzyl)-3-methyl-2,6-dioxo-2,3,6,7-tetrahydro-purin-1-ylmethyl]-benzonitrile
*Cis*-8-(2-Amino-cyclohexylamino)-7-(2-bromo-benzyl)-3-methyl-1-(2-oxo-2-phenyl-ethyl)-3,7-dihydro-purine-2,6-dione
*Cis*-8-(2-Amino-cyclohexylamino)-7-(2-bromo-benzyl)-3-methyl-1-phenethyl-3,7-dihydro-purine-2,6-dione

The Neutral Endopeptidase inhibitor used in a combination preparation according to the invention may be selected from known NEP or dual NEP/ACE inhibitors or prodrugs of such inhibitors.

In a preferred embodiment, the NEP inhibitor used in a combination preparation according to the invention is candoxatril, which is a prodrug of candoxatrilat.

In another embodiment the NEP inhibitor used in a combination preparation according to the invention is a dual NEP/ACE inhibitor.

In another embodiment the dual NEP/ACE inhibitor is omapatrilat.

The compounds of the present invention may be prepared in the form of pharmaceutically acceptable salts, especially acid-addition salts, including salts of organic acids and mineral acids. Examples of such salts include salts of organic acids such as formic acid, fumaric acid, acetic acid, propionic acid, glycolic acid, lactic acid, pyruvic acid, oxalic acid, succinic acid, malic acid, tartaric acid, citric acid, benzoic acid, salicylic acid and the like. Suitable inorganic acid-addition salts include salts of hydrochloric, hydrobromic, sulphuric and phosphoric acids and the like. Further examples of pharmaceutically acceptable inorganic or organic acid addition salts include the pharmaceutically acceptable salts listed in Journal of Pharmaceutical Science, 66, 2 (1977) that are known to the skilled artisan.

Also intended as pharmaceutically acceptable acid addition salts are the hydrates that the present compounds are able to form.

The acid addition salts may be obtained as the direct products of compound synthesis. In the alternative, the free base may be dissolved in a suitable solvent containing the appropriate acid, and the salt isolated by evaporating the solvent or otherwise separating the salt and solvent.

The compounds of this invention may form solvates with standard low molecular weight solvents using methods known to the skilled artisan.

It is to be understood that the invention extends to all of the stereo isomeric forms of the claimed compounds, as well as the racemates.

### PHARMACEUTICAL COMPOSITIONS

In another aspect, the present invention includes within its scope pharmaceutical compositions comprising, as active ingredient, both compounds of the invention, i.e. both a DPP-IV inhibitor and a NEP inhibitor, or a pharmaceutically acceptable salt or hydrate thereof together with a pharmaceutically acceptable carrier or diluent.

In another aspect, the present invention includes within its scope pharmaceutical compositions comprising, as active ingredient, a compound of the invention having dual inhibitory action, i.e. a compound which inhibits both DPP-IV and a NEP.

In another aspect, the present invention includes within its scope pharmaceutical compositions comprising, as active ingredient, one of the inhibitors, where said composition is meant to be used in a regimen where a DPP-IV inhibitor and a NEP inhibitor is to be administered separately. Pharmaceutical compositions containing a compound of the invention of the present invention may be prepared by conventional techniques, e.g. as described in Remington: The Science and Practise of Pharmacy, 19th Ed., 1995. The compositions may appear in conventional forms, for example capsules, tablets, aerosols, solutions, suspensions or topical applications.

Typical compositions include a compound of the invention which inhibits the enzymatic activity of DPP-IV or a pharmaceutically acceptable basic addition salt or hydrate thereof, associated with a pharmaceutically acceptable excipient which may be a carrier or a diluent or be diluted by a carrier, or enclosed within a carrier which can be in the form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier which may be in the form of a ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, peanut oil, olive oil, gelatine, lactose, terra alba, sucrose, dextrin, magnesium carbonate, sugar, cyclodextrin, amylose, magnesium stearate, talc, gelatine, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art. The pharmaceutical compositions can be sterilized and mixed, if desired, with auxiliary agents, emulsifiers, salt for influencing osmotic pressure, buffers and/or colouring substances and the like, which do not deleteriously react with the active compounds.

The route of administration may be any route, which effectively transports the active compound of the invention which inhibits the enzymatic activity of DPP-IV to the appropriate or desired site of action, such as oral, nasal, pulmonary, buccal, subdermal, intradermal, transdermal or parenteral e.g. rectal, depot, subcutaneous, intravenous, intraurethral, intramuscular, intranasal, ophthalmic solution or an ointment, the oral route being preferred.

If a solid carrier is used for oral administration, the preparation may be tabletted, placed in a hard gelatin capsule in powder or pellet form or it can be in the form of a troche or lozenge. If a liquid carrier is used, the preparation may be in the form of a syrup, emulsion, soft gelatin capsule or sterile injectable liquid such as an aqueous or non-aqueous liquid suspension or solution.

For nasal administration, the preparation may contain a compound of the invention which inhibits the enzymatic activity of DPP-IV, dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil. Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

A typical tablet which may be prepared by conventional tabletting techniques may contain:

| Core: | |
|---|---|
| Active compound (as free compound or salt thereof) | 250 mg |
| Colloidal silicon dioxide (Aerosil)® | 1.5 mg |
| Cellulose, microcryst. (Avicel)® | 70 mg |
| Modified cellulose gum (Ac-Di-Sol)® | 7.5 mg |
| Magnesium stearate | Ad. |

| Coating: | |
|---|---|
| HPMC approx. | 9 mg |
| *Mywacett 9-40 T approx. | 0.9 mg |

| | |
|---|---|
| *Acylated monoglyceride used as plasticizer for film coating. | |

The compounds of the invention are effective over a wide dosage range. For example, in the treatment of adult humans, dosages from about 0.05 to about 1000 mg, preferably from about 0.1 to about 500 mg, per day may be used. A most preferable dosage is about 0.5 mg to about 250 mg per day. In choosing a regimen for patients it may frequently be necessary to begin with a higher dosage and when the condition is under control to reduce the dosage. The exact dosage will depend upon the mode of administration, on the therapy desired, form in which administered, the subject to be treated and the body weight of the subject to be treated, and the preference and experience of the physician or veterinarian in charge. Generally, the compounds of the present invention are dispensed in unit dosage form comprising from about 0.05 to about 1000 mg of active ingredient together with a pharmaceutically acceptable carrier per unit dosage.

Usually, dosage forms suitable for oral, nasal, pulmonal or transdermal administration comprise from about 0.05 mg to about 1000 mg, preferably from about 0.5 mg to about 250 mg of the compounds admixed with a pharmaceutically acceptable carrier or diluent.

### EXAMPLES

### Preparative HPLC (Method A1)

Column: 1.9 x 15 cm Waters XTerra RP-18. Buffer: linear gradient 5 - 95% in 15 min, MeCN, 0.1 % TFA, flow rate of 15 ml/min. The pooled fractions are either evaporated to dryness *in vacuo*, or evaporated *in vacuo* until the MeCN is removed, and then frozen and freeze dried.

### Preparative HPLC (Method A2)

Column: Supelcosil ABZ+Plus, 25 cm x 10 mm, 5 µm. Solvent A: 0.1 % TFA/Water, solvent B: MeCN. Eluent composition: 5 min. 100% A, linear gradient 0 - 100% B in 7 min, 100% B in 2 min. Flow rate 5 ml/min. The column is allowed to equilibrate for 4 min in 100% A before the next run.

### HPLC-MS (Method B)

Column: Waters Xterra MS C-18 X 3 mm id. Buffer: Linear gradient 10 - 100% in 7.5 min, MeCN, 0.01% TFA, flow rate 1.0 ml/min. Detection 210 nm (analog output from diode array detector), MS-detection ionisation mode API-ES, scan 100-1000 amu step 0.1 amu.

### HPLC-MS (Method C)

Column: 0.3 mm x 15 cm Waters Symmetry C₁₈. Buffer: Linear gradient 5 - 90% in 15 min, MeCN, 0.05% TFA, flow rate 1 ml/min

### Analytical separation of stereoisomers (Method D)

CCE. Chiral capillary electrophoresis: Conditions: HP 3D Capillary Electrophoresis: 48.5/40cm, 50µm HP bubble capillary, Electrolyte: HS-β-CD (Regis) (2% w/v) in 50mM phosphate buffer pH2.5 (HP), Voltage: -17kV, Injection: 30mbar for 5s.

### Preparative separation of stereoisomers (Method E)

Analytical separations were performed on Hewlett Packard 1090 HPLC equipment with 5 chiral Daicel columns (AD, OD, AS, OJ and Welko-O2, 250 x 4.6 mm) with a diode array detector. The mobile phases were 2-propanol:heptane mixtures with 0.1 % DEA.

Preparative separations were performed with the above-mentioned type of columns (250 x 20 mm) on a preparative Gilson HPLC set-up. Relevant fractions were collected and evaporated (SpeedVac).

### Microwave assisted reactions (Method F)

The reactants are mixed in an appropriate solvent in a closed teflon vessel (XP 1500 Plus Vessel set) and heated in a micro wave oven (CEM MARSX microwave instrument. Magnetron frequency: 2455 MHz. Power Output: 1200 Watt.). The reaction mixture is cooled and evaporated *in vacuo*. Normally solvents like MeOH; EtOH, iPrOH; H2O; DMF and DMSO are used.

### Abbreviations

| | |
|---|---|
| DCHMA | Dicyclohexylmethylamine |
| EtOAc | Ethyl acetate |
| DCM | Dichloromethane |
| DEA | Diethylamine |
| DIEA | Diisopropylethylamine |
| DMF | Dimethylformamide |
| DMSO | Dimethyl sulfoxide |
| HOAc | Acetic acid |
| MeCN | Acetonitrile |
| TFA | Trifluoroacetic acid |
| THF | Tetrahydrofuran |
| TMG | Tetramethylguanidine |

The compounds of Formula I are prepared according to the following procedures:

### General procedure (A):

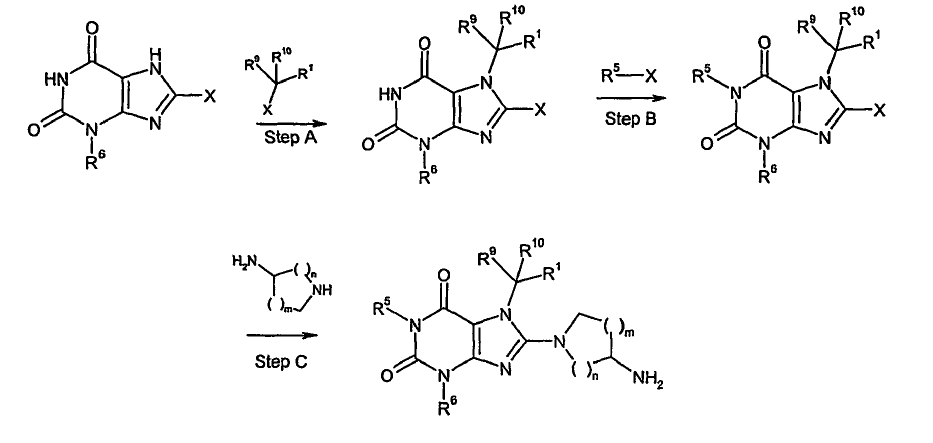

### Step A:

The starting material (16 µmol) is dissolved in a mixture of DMF and DIEA (3% DIEA, 250 µl). The alkylation reagent R¹-CR⁹R⁹-X (16.8 µmol, 1.05 equiv) is dissolved in DMF (100 µl) and added. The mixture is heated to 65 °C for 2h.

### Step B:

Alkylation reagent R⁵-Br (32 µmol) is dissolved in DMF (100 µl) and added to the reaction mixture followed by a solution of TMG in DMF (1.16 ml TMG diluted to 5.8 ml, 48 µl). The mixture is kept at 65 °C for 4h.

### Step C:

The diamine (200 µmol) is dissolved in a mixture of DMF and DIEA (3% DIEA, 200 µl) and added to the reaction mixture. The reaction is kept at 50 °C for 24h.

Samples are neutralized using HOAc (20 µl), stripped and purified by HPLC. Samples are dissolved in DMSO/H₂O (4:1, 500 µl).

### General procedure (B)

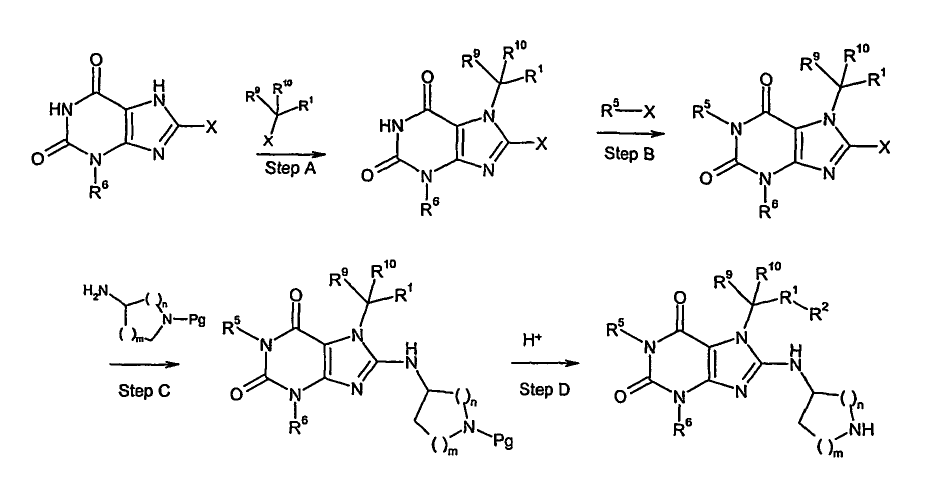

### Step A:

The starting material (16 µmol) is dissolved in a mixture of DMF and DIEA (3% DIEA, 250 µl). The alkylation reagent R¹-CR⁹R⁹-X (16.8 µmol, 1.05 equiv) is dissolved in DMF (100 µl) and added. The mixture is heated to 65°C for 2h.

### Step B:

Alkylation reagent R⁵-Br (32 µmol) is dissolved in DMF (100 µl) and added to the reaction mixture followed by a solution of TMG in DMF (1.16 ml TMG diluted to 5.8 ml, 48 µl). The mixture is kept at 65°C for 4h.

### Step C:

The monoprotected diamine (200 µmol) is dissolved in a mixture of DMF and DIEA (3% DIEA, 200 µl) and added to the reaction mixture. The reaction is kept at 50°C for 24-48h, and then all volatiles are stripped.

### Step D:

TFA (2 ml) is added, and the reaction is kept for 16 h at room temperature. The reactions are stripped from excess TFA, taken up in acetonitrile, and purified by HPLC (method A). Samples are dissolved in DMSO/H₂O (4:1, 500 µl).

### General procedure (C)

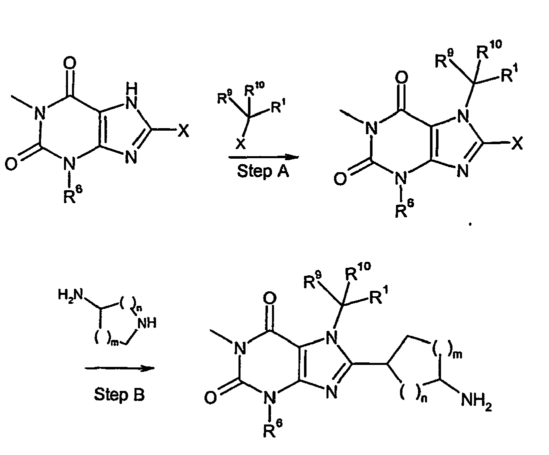

### Step A:

The starting material (16 µmol) is dissolved in a mixture of DMF and DIEA (3% DIEA, 250 µl). The alkylation reagent R¹-CR⁹R⁹-X (16.8 µmol, 1.05 equiv) is dissolved in DMF (100 µl) and added. The mixture is heated to 65°C for 2h.

### Step B:

Diamine (200 µmol) is dissolved in a mixture of DMF and DIEA (3% DIEA, 200 µl) and added to the reaction mixture. The reaction is kept at 50°C for 24-48h, and then all volatiles are stripped.

Samples are neutralized using HOAc (20 µl), stripped and purified by HPLC. Samples are dissolved in DMSO/H₂O (4:1, 500 µl).

### General procedure (D)

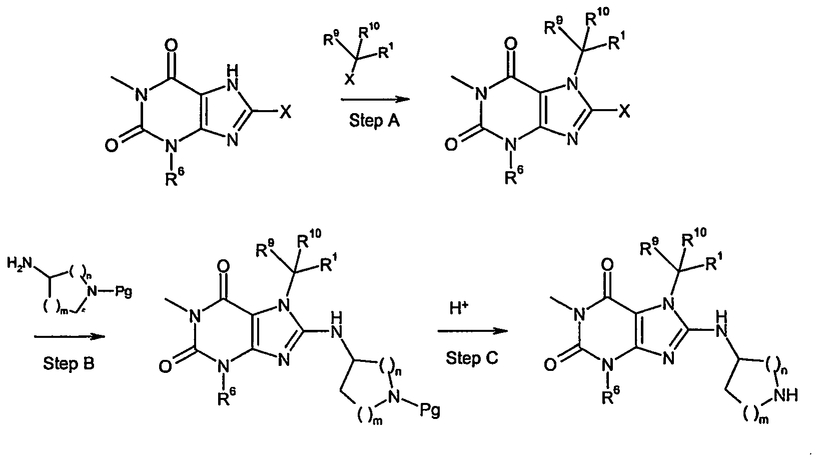

### Step A:

The starting material (16 µmol) is dissolved in a mixture of DMF and DIEA (3% DIEA, 250 µl). The alkylation reagent R¹-CR⁹R⁹-X (16.8 µmol, 1.05 equiv) is dissolved in DMF (100 µl) and added. The mixture is heated to 65°C for 2h..

### Step B:

The monoprotected diamine (200 µmol) is dissolved in a mixture of DMF and DIEA (3% DIEA, 200 µl) and added to the reaction mixture. The reaction is kept at 50°C for 24-48h, and then all volatiles are stripped.

### Step C:

TFA (2 ml) is added, and the reaction is kept for 16 h at room temperature. The reactions are stripped from excess TFA, taken up in acetonitrile, and purified by HPLC (method A). Samples are dissolved in DMSO/H₂O (4:1, 500 µl).

The R-groups in the general methods above are as defined in the description of the invention section. The Pg group is an acid labile N-protection group such as Boc or trityl.

The compounds of Formula II are prepared according to the following procedure:

### General procedure (E):

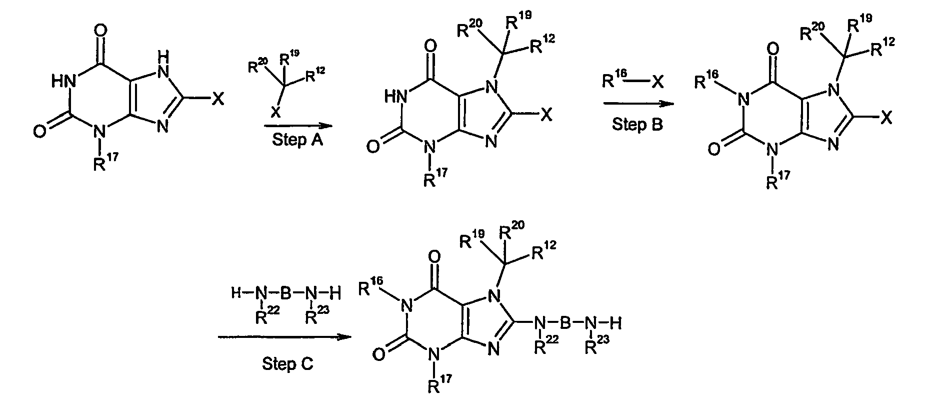

### Step A:

The starting material (16 µmol) is dissolved in a mixture of DMF and DIEA (3% DIEA, 250 µl). The alkylation reagent R¹²-CR¹⁹R²⁰-X (16.8 µmol, 1.05 equiv) is dissolved in DMF (100 µl) and added. The mixture is heated to 65 °C for 2h.

### Step B:

Alkylation reagent R¹⁶-Br (32 µmol) is dissolved in DMF (100 µl) and added to the reaction mixture followed by a solution of TMG in DMF (1.16 ml TMG diluted to 5.8 ml, 48 µl). The mixture is kept at 65 °C for 4h. Volatiles are stripped

### Step C:

The diamine (200 µmol) is dissolved in a mixture of DMSO and DCHMA (3% DCHMA, 200 µl) and added to the reaction mixture. The reaction is kept at 50 °C for 44h.

Samples are neutralized using HOAc (20 µl), stripped and purified by HPLC Method A2..

### General procedure (F):

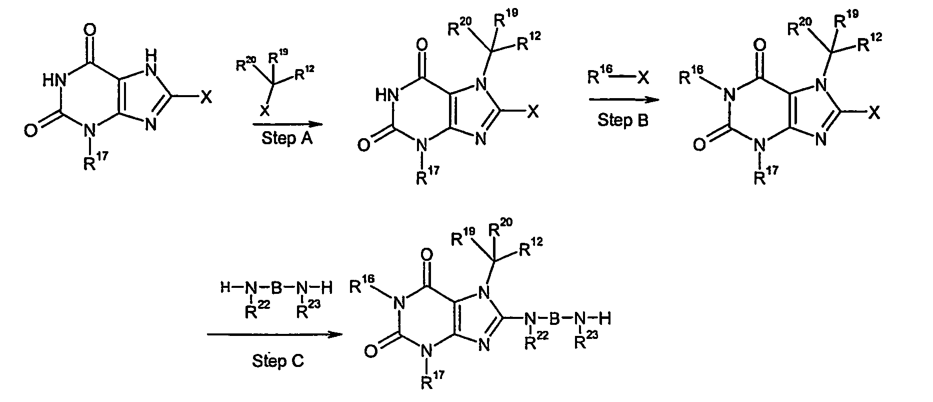

### Step A:

The starting material (32 µmol) is dissolved in a mixture of DMF and DIEA (3% DIEA, 500 µl). The alkylation reagent R¹²-CR¹⁹R²⁰-X (33.6 µmol, 1.05 equiv) is dissolved in DMF (200 µl) and added. The mixture is heated to 65 °C for 2h. Upon cooling to 25 °C, K₂CO₃ (aq) is added (5.12M, 50 µL, 256 umol). Volatiles are stripped.

### Step B:

Alkylation reagent R¹⁶-Br (64 µmol) is dissolved in DMF (250 µl) and added to the reaction mixture. The mixture is kept at 25 °C for 48h. Volatiles are stripped

### Step C:

The diamine (400 µmol) is dissolved in DMSO and added to the reaction mixture. If the dihydrochloride salt of the diamine is employed, four equivalents of DCHMA is added. The reaction is kept at 50 °C for 48h.

Samples are neutralized using HOAc (30 µl), and purified by HPLC Method (HDEM).

### General procedure (G)

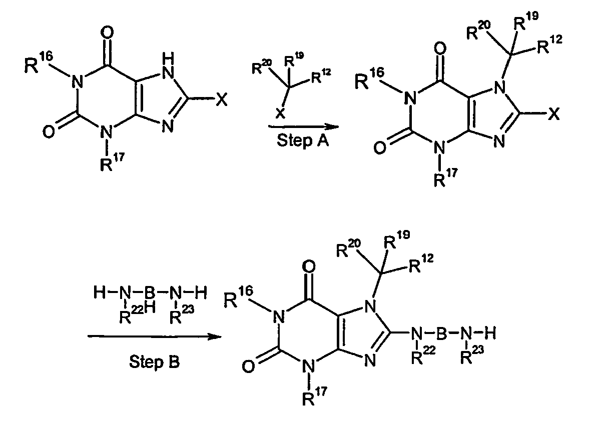

### Step A:

The starting material (4.08 mmol) is dissolved in a mixture of DMF and DIEA (3% DIEA, 65 ml). The alkylation reagent R¹²-CR¹⁹R²⁰-X (4.28 mmol, 1.05 equiv) is dissolved in DMF (25.5 ml) and added. The mixture is heated to 65°C for 2h and poured onto ice followed by filtration of the alkylated product.

### Step B:

Diamine (400 µmol) is dissolved in DMSO (400 µl) and added to the above product (32 umol). The reaction is kept at 50°C for 24-48h.

Samples are neutralized using HOAc (30 µl) and purified by HPLC Method A2.

### General procedure (H)

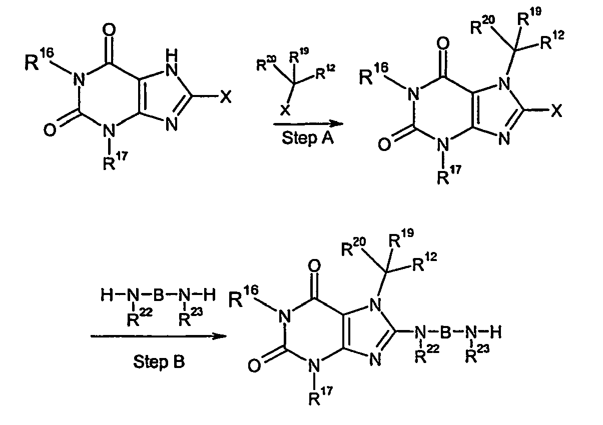

### Step A:

The starting material (32 µmol) is dissolved in a mixture of DMF and DIEA (3% DIEA, 500 µl). The alkylation reagent R¹²-CR¹⁹R²⁰-X (33.6 µmol, 1.05 equiv) is dissolved in DMF (200 µl) and added. The mixture is heated to 65°C for 2h.

### Step B:

Diamine (400 µmol) is dissolved in DMSO (400 µl) and added to the above reaction mixture. The reaction is kept at 50°C for 48h.

Samples are neutralized using HOAc (30 µl) and purified by HPLC Method A2.

### General procedure (I):

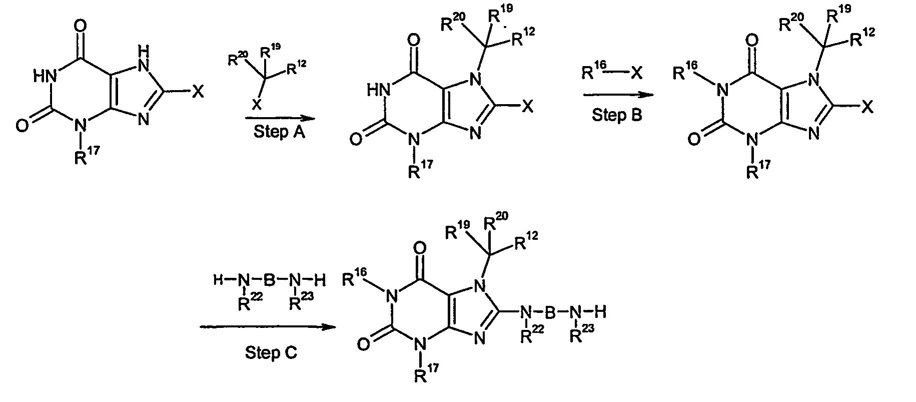

### Step A:

The starting material (20.40 mmol) is dissolved in DMF (50 ml) and DIEA (10 mL). The alkylation reagent R¹²-CR²⁰R¹⁹-X (22.03 mmol, 1.08 equiv) is dissolved in DMF (10 ml) and added. Heating the mixture to 65 °C for 2h affords the products that are isolated by filtration upon adding the reaction mixture onto ice (300 mL).

### Step B:

The product from Step A (5.56 mmol) and alkylation reagent R¹⁶-Br (11.11 µmol) are dissolved in DMF (60 mL) and potassium carbonate is added to the reaction mixture. Upon stirring at 25 °C for 16h the reaction mixture is poured onto ice (300 ml) and the product is isolated by filtration and dried *in vacuo*.

### Step C:

The product from Step B (0.472 mmol) is dissolved in DMSO (5 ml) and the diamine (2.36 mmol) is added to the reaction mixture. If the dihydrochloride salt of the diamine is employed, K₂CO₃ (2.36 mmol) is added. The reaction is kept at 50 °C for 24h and poured onto ice (20 ml). The product is isolated by filtration.

### General procedure (J) for removal of protection groups (Pg):

Sometimes mono-protected diamines are employed in the final substitution reaction. In these cases, an extra synthesis step is required to remove the protection group.

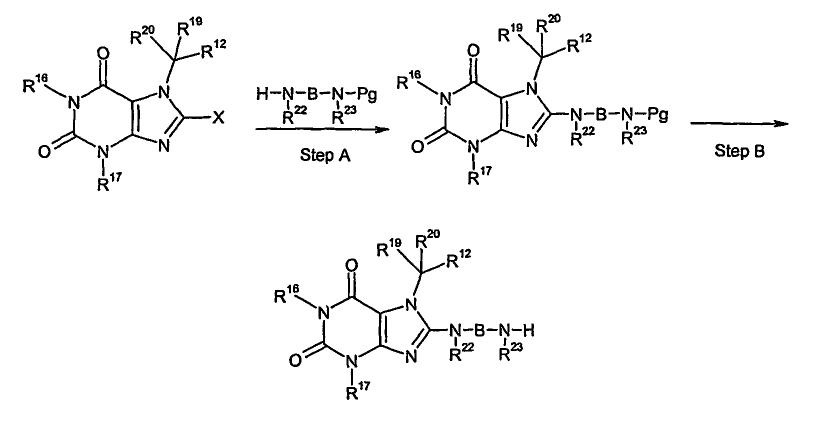

### Step A

The product generated in Step A may be obtained via any of the above-mentioned general procedures.

### Step B

The conditions required depend on the nature of the protecting group.

### Example 1

### 2-(8-(3-Aminopiperidin-1-yl)-1,3-dimethyl-2,6-dioxo-1,2,3,6-tetrahydropurin-7-ylmethyl)benzonitrile. TFA

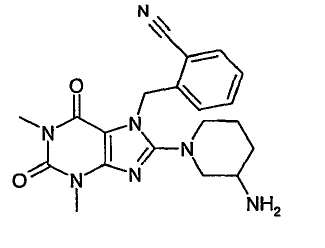

### Step A: 2-(8-Chloro-1,3-dimethyl-2,6-dioxo-1,2,3,6-tetrahydroropurin-7-ylmethyl)benzonitrile (1A)

8-Chlorotheophylline (20 g, 93.19 mmol) was dissolved in 800 ml of DMF and 2-cyanobenzyl bromide (18.28 g, 93.19 mmol), potassium carbonate (12.88 g, 93.19 mmol), and potassium iodide (10 mg, 0.06 mmol) were added. The mixture was stirred at room temperature for 20 hours. The solvent was evaporated and the residue was suspended in 900 ml of water and 900 ml of EtOAc, and compound (1A) was collected by filtration of the suspension. The layers in the mother liquor were separated and the aqueous layer was extracted with 3 x 500 ml of EtOAc. The combined organic layers were washed with 1 x 500 ml of water, and the solvent was evaporated to give compound (1A) as white crystals.

Combined yield: 28.6 g (93%). Mp. 222.5 - 223.7°C.
¹H-NMR (DMSO, 300 MHz) δ: 3.20 (s, 3H); 3.43 (s, 3H); 5.74 (s, 2H); 7.06 (d, 1H); 7.53 (t, 1 H); 7.67 (t, 1 H); 7.93 (d, 1 H). HPLC-MS (Method B): *m*/*z* = 330 (M+1); Rₜ = 2.93 min.

### Step B: 2-(8-(3-Aminopiperidin-1-yl)-1,3-dimethyl-2,6-dioxo-1,2,3,6-tetrahydropurin-7-ylmethyl)benzonitrile. TFA (1)

2-(8-Chloro-1,3-dimethyl-2,6-dioxo-1,2,3,6-tetrahydropurin-7-ylmethyl)benzonitrile (1A) (100 mg, 0.30 mmol) and 3-aminopiperidine dihydrochloride (262 mg, 1.52 mmol) were dissolved in 20 ml of 2-propanol and triethylamine (0.127 ml, 0.91 mmol) and subjected to microwaves (method F, 130°C, 300W) for ten hours. The solvents were evaporated and the crude product was purified by preparative HPLC, (method A1, Rt = 6.78 min.) to give the title compound as oily crystals.
Yield: 66 mg (43%).
¹H-NMR (MeOD, 300 MHz) δ: 1.73 (m, 3H); 2.10 (m, 1H); 3.02 (m, 1H); 3.20 (m, 2H); 3.27 (s, 3H); 3.52 (m, 4H); 3.65 (m, 1 H); 5.59 (s, 2H); 7.22 (d, 1 H); 7.47 (m, 1 H); 7.61 (m, 1 H); 7.78 (d, 1 H). HPLC-MS (Method B): *mlz =* 394 (M+1); Rₜ = 1.55 min.

### Example 2

### 8-(3-Aminopyrrolidin-1-yl)-7-benzyl-1,3-dimethyl-3,7-dihydropurine-2,6-dione. HCl

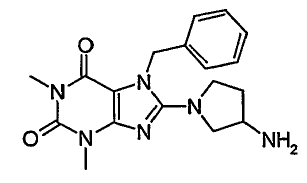

### Step A: 7-Benzyl-8-chloro-1,3-dimethyl-3,7-dihydropurine-2,6-dione (2A)

8-Chlorotheophylline (50 g, 0.23 mol) was suspended in 600 ml of DMF and benzyl bromide (31 ml, 0.26 mol) and potassium carbonate (64 g, 0.46 mol) were added. The mixture was stirred at room temperature for 20 hours. The solvent was evaporated and the residue was dissolved in 250 ml of water and 400 ml of DCM. The layers were separated and the aqueous layer was extracted with 150 ml of DCM. The combined organic layer was washed with 100 ml of brine, dried over magnesium sulphate, filtered, and the solvent was evaporated to give compound (2A) as white crystals.
Yield: 73.6 g (104%). Mp. 152 - 154°C.
¹H-NMR (CDCl₃, 200 MHz) δ: 3.42 (s, 3H); 3.55 (s, 3H); 5.55 (s, 2H); 7.35 (m, 5H). HPLC-MS (Method B): *m*/*z* = 305 (M+1); Rₜ = 3.33 min.

### Step B: 8-(3-Aminopyrrolidin-1-yl)-7-benzyl-1,3-dimethyl-3,7-dihydropurine-2,6-dione. HCl (2)

7-Benzyl-8-chloro-1,3-dimethyl-3,7-dihydropurine-2,6-dione (2A) (100 mg, 0.33 mmol) and 3-aminopyrrolidine (0.16 ml, 1.64 mmol) were dissolved in 20 ml of 2-propanol and subjected to microwaves (method F, 150°C, 300W) for one hour. The solvent was evaporated and the crude product was purified by preparative HPLC (method A1, Rt = 6.45 min.). Evaporation of the solvent afforded the title compound as a brown oil.
Yield: 111 mg (87%).
¹H-NMR (MeOD, 400 MHz) δ: 2.04 (m, 1H); 2.37 (m, 1H); 3.30 (s, 3H); 3.51 (s, 3H); 3.60 - 3.80 (m, 3H); 3.87 - 3.95 (m, 2H); 5.54 (d, 1 H); 5.64 (d, 1 H); 7.14 (d, 2H); 7.23 - 7.35 (m, 3H) HPLC-MS (Method B): *m*/*z* = 355 (M+1); Rₜ = 1.49 min.

### Example 3

### (S) 8-(3-Aminopyrrolidin-1-yl)-7-benzyl-1,3-dimethyl-3,7-dihydropurine-2,6-dione. HCl

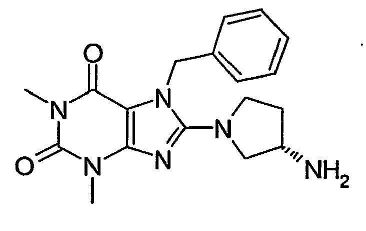

### Step A: (S) (1-(7-Benzyl-1,3-dimethyl-2,6-dioxo-1,2,3,6-tetrahydropurin-8-yl)pyrrolidin-3-yl)carbamic acid tert-butyl ester (3A)

7-Benzyl-8-chloro-1,3-dimethyl-3,7-dihydropurine-2,6-dione (2A) (100 mg, 0.33 mmol), (3S)-(-)-3-(tert-butoxycarbonylamino)pyrrolidine (305 mg, 1.64 mmol), and triethylamine (0.46 ml, 3.28 mmol) was dissolved in 20 ml of 2-propanol and 5 ml of DMF and the mixture was subjected to microwaves (method F, 130°C, 300W) for three hours. The solvent was evaporated and the crude product was purified by preparative HPLC (method A1, Rt = 11.75 min.). Evaporation of the solvent afforded compound (3A) as a brown oil.
Yield: 130 mg (87%)
¹H-NMR (CDCl₃, 200 MHz) δ: 1.42 (s, 9H); 1.89 (m, 1H); 2.12 (m, 1H); 3.34 (s, 3H); 3.37 - 3.79 (m, 7H); 4.22 (br. s, 1 H); 4.97 (d, 1 H); 5.49 (d, 1 H); 5.55 (d, 1 H); 7.04 (m, 2H); 7.28 (m, 3H). HPLC-MS (Method B): *m*/*z* = 455 (M+1); Rₜ = 3.95 min.

### Step B: (S) 8-(3-Aminopyrrolidin-1-yl)-7-benzyl-1,3-dimethyl-3,7-dihydropurine-2,6-dione. HCl (3)

(S) (1-(7-Benzyl-1,3-dimethyl-2,6-dioxo-1,2,3,6-tetrahydropurin-8-yl)pyrrolidin-3-yl)carbamic acid tert-butyl ester (3A) (130 mg, 0.29 mmol) was dissolved in 15 ml of diethyl ether, hydrochloric acid in diethyl ether (2.5 M, 5.72 ml, 14.3 mmol) was added, and the mixture was stirred at room temperature for 24 hours. The solvents were evaporated and the crude product was suspended in dry DCM and collected by filtration to afford the title compound as white crystals.
Yield: 101 mg, (91%) Mp. 166 - 169°C.
¹H-NMR (MeOD, 300 MHz) δ: 2.05 (m, 1 H); 2.37 (m, 1H); 3.29 (s, 3H); 3.52 (s, 3H); 3.58 - 3.97 (m, 5H); 5.53 (d, 1 H); 5.63 (d, 1 H); 7.13 (d, 2H); 7.21 - 7.36 (m, 3H).
HPLC-MS (Method B): *m*/*z =* 355 (M+1); Rₜ = 1.52 min.

### Example 4

### 2-(8-(3-Aminopyrrolidin-1-yl)-1,3-dimethyl-2,6-dioxo-1,2,3,6-tetrahydropurin-7-ylmethyl)benzonitrile. HCl

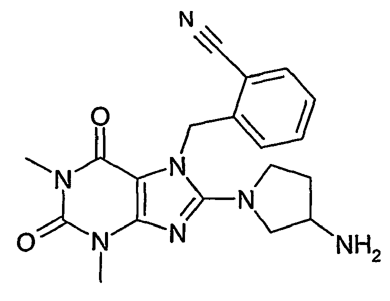

2-(8-Chloro-1,3-dimethyl-2,6-dioxo-1,2,3,6-tetrahydropurin-7-ylmethyl)benzonitrile (1A) (100 mg, 0.30 mmol) and 3-aminopyrrolidine (0.15 ml, 1.52 mmol) were reacted and purified as described in example **2**, step B, to give the title compound as a yellow foam.
Yield: 108 mg (76%). Mp.186 - 189°C.
Prep. HPLC (method A1): Rₜ = 6.19 min.
¹H-NMR (MeOD, 400 MHz) δ: 2.09 (m, 1 H); 2.40 (m, 1 H); 3.27 (s, 3H); 3.50 (s, 3H); 3.59 - 3.78 (m, 3H); 3.88 - 3.99 (m, 2H); 5.70 (d, 1H); 5.79 (d, 1 H); 7.12 (d, 1H); 7.49 (dd, 1 H); 7.62 (dd, 1 H); 7.80 (d, 1 H). HPLC-MS (Method B): *mlz* = 380 (M+1); Rₜ = 1.35 min.

### Example 5

### 8-(3-Aminopyrrolidin-1-yl)-7-(2-iodobenzyl)-1,3-dimethyl-3,7-dihydropurine-2,6-dione. HCl

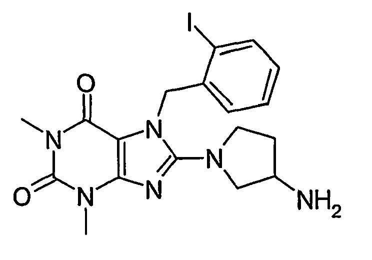

### Step A: 8-Chloro-7-(2-iodobenzyl)-1,3-dimethyl-3,7-dihydropurine-2,6-dione (5A)

8-Chlorotheophylline (8.5 g, 39.6 mmol) was dissolved in 400 ml of DMF and 2-iodobenzyl chloride (10.0 g, 39.6 mmol), potassium carbonate (5.47 g, 39.6 mmol), and potassium iodide (10 mg, 0.06 mmol) were added. The mixture was stirred at room temperature for 7 days. Water (2500 ml) and EtOAc (800 ml) were added and the layers were separated. The aqueous layer was extracted with 2 x 500 ml of EtOAc, and the combined organic layer was washed with 500 ml of water, 500 ml of brine, dried over sodium sulphate, and filtered. The solvent was evaporated and the crude product was crystallized from diethyl ether and petrol, to give compound (5A) as white crystals. The mother liquor was evaporated and resuspended in diethyl ether and petrol, to give a second crop of compound (5A).
Combined yield: 10.4 g (61 %). Mp. 177.6 - 178.2°C.
¹H-NMR (CDCl₃, 300 MHz) δ: 3.37 (s, 3H); 3.61 (s, 3H); 5.59 (s, 2H); 6.48 (d, 1H); 7.02 (t, 1H); 7.27 (t, 1 H); 7.90 (d, 1 H). HPLC-MS (Method B): *m*/*z =* 431 (M+1); Rₜ = 3.94 min.

### Step B: 8-(3-Aminopyrrolidin-1-yl)-7-(2-iodobenzyl)-1,3-dimethyl-3,7-dihydropurine-2,6-dione. HCl (5)

8-Chloro-7-(2-iodobenzyl)-1,3-dimethyl-3,7-dihydropurine-2,6-dione (5A) (100 mg, 0.23 mmol) and 3-aminopyrrolidine (0.13 ml, 1.16 mmol) were reacted and purified as described in example **2,** ste B, to give the crude product, which was further suspended in dry DCM, and filtered to afford the title compound as white crystals.
Yield: 77 mg (64%).
Prep. HPLC (method A1): Rₜ = 7.28 min.
¹H-NMR (MeOD, 200 MHz) δ: 2.02 (m, 1 H); 2.35 (m, 1 H); 3.27 (s, 3H); 3.47 - 3.74 (m, 6H); 3.82 - 3.93 (m, 2H); 5.44 (d, 1H); 5.53 (d, 1 H); 6.72 (d, 1H); 7.04 (dd, 1 H); 7.32 (dd, 1H); 7.92 (d, 1 H). HPLC-MS (Method B): *m*/*z =* 481 (M+1); Rₜ = 1.76 min.

### Example 6

### 8-(3-Aminoazepan-1-yl)-7-benzyl-1,3-dimethyl-3,7-dihydropurine-2,6-dione. TFA

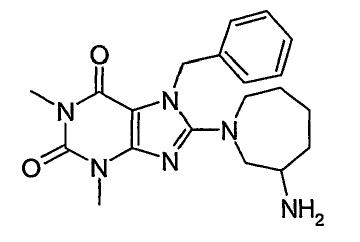

### Step A: N-(2-Oxoazepan-3-yl)-4-methylbenzenesulfonamide (6A)

DL-3-Amino-E-caprolactam (3 g, 23.4 mmol) was dissolved in 140 ml of dry DCM and dry triethylamine (4.5 ml) and 4-toluenesulfonyl chloride (4.5 g, 23.6 mmol) were added. The reaction was stirred for 3 days at room temperature and then filtered through celite. The filtrate was extracted with 50 ml of 1 M aqueous potassium hydrogen sulphate, 50 ml of saturated sodium hydrogen carbonate, 50 ml of water, and 50 ml of brine, and dried over sodium sulphate. The solvent was evaporated and the residue suspended in dry dichloromethane, and compound (6A) was collected by filtration. The mother liquor was evaporated and resuspended in DCM, to give a second crop of compound (6A) as white crystals.
Combined yield: 5.99 g (90%). Mp. 179.9 - 180.5°C.
¹H-NMR (CDCl₃, 300 MHz) δ: 1.34 (m, 1 H); 1.55 - 1.85 (m, 3H); 2.00 (m, 1 H); 2.17 (m, 1 H); 2.40 (s, 3H); 3.10 (m, 2H); 3.81 (m, 1 H); 5.86 (m, 1 H); 6.12 (d, 1 H); 7.28 (d, 2H); 7.72 (d, 2H). HPLC-MS (Method B): *mlz* = 283 (M+1); Rₜ = 2.71 min.

### Step B: N-(Azepan-3-yl)-4-methylbenzenesulfonamide (6B)

N-(2-Oxoazepan-3-yl)-4-methylbenzenesulfonamide (6A) (4.24 g, 15 mmol) was dissolved in 250 ml of dry THF under a nitrogen atmosphere, and lithium aluminium hydride (1.11 g, 30 mmol) was added slowly. The reaction was heated to reflux for 20 hours and then quenched with water until the effervescence ceased. Solid potassium carbonate was added until a white suspension appeared, and the mixture was allowed to stir for half an hour. The suspension was filtered through celite, which was washed with 3 x 50 ml of EtOAc. The solvents were evaporated and the residue was dissolved in 100 ml of EtOAc and 100 ml of water. The layers were separated and the aqueous layer was extracted with 2 x 100 ml of EtOAc. The combined organic layer was washed with brine, dried over sodium sulphate, and evaporated to give compound (6B) as an oil.
Yield: 2.89 g (71%).
¹H-NMR (CDCl₃, 300 MHz) δ: 1.37 - 1.74 (m, 6H); 2.41 (s, 3H); 2.55 - 2.93 (m, 4H); 3.45 (m, 1 H); 7.27 (d, 2H); 7.76 (d, 2H). HPLC-MS (Method B): *m*/*z =* 269 (M+1); Rₜ = 1.43 min.

### Step C: N-(1-(7-Benzyl-1,3-dimethyl-2,6-dioxo-1,2,3,6-tetrahydropurin-8-yl)azepan-3-yl)-4-methylbenzenesulfonamide (6C)

7-Benzyl-8-chloro-1,3-dimethyl-3,7-dihydropurine-2,6-dione (2A) (1.03 g, 3.40 mmol) and N-(azepan-3-yl)-4-methylbenzenesulfonamide (6B) (1.00 g, 3.73 mmol) were dissolved in 2-methoxyethanol (30 ml) and triethylamine (2.4 ml), and the mixture was heated to 120°C for 2 days. The solvents were evaporated and the crude product was dissolved in 100 ml of EtOAc and 100 ml of water. The aqueous phase was acidified with 1 M potassium hydrogen sulphate until pH = 2. The organic layer was separated and extracted with 50 ml of 1M aqueous potassium hydrogen sulphate, and 50 ml of brine, and dried over sodium sulphate. The solvent was evaporated and the crude product was purified by column chromatography on silica gel using EtOAc:heptane (1:1) as the eluent. Evaporation of the solvent gave compound (6C) as a white foam.
Yield: 548 mg (30%). Mp. 80.2 - 88.2°C.
¹H-NMR (CDCl₃, 300 MHz) δ: 1.22 - 1.84 (m, 6H); 2.41 (s, 3H); 3.00 (m, 1 H); 3.25 (dd, 1 H); 3.47 - 3.72 (m, 6H); 5.37 (d, 1H); 5.59 (d, 1 H); 7.03 (d, 2H); 7.29 (m, 5H); 7.75 (d, 2H); 7.88 (d, 1 H). HPLC-MS (Method B): *mlz =* 537 (M+1); Rₜ = 4.32 min.

### Step D: 8-(3-Aminoazepan-1-yl)-7-benzyl-1,3-dimethyl-3,7-dihydropurine-2,6-dione. TFA (6)

N-(1-(7-Benzyl-1,3-dimethyl-2,6-dioxo-1,2,3,6-tetrahydropurin-8-yl)azepan-3-yl)-4-methylbenzenesulfonamide (6C) (100 mg, 0.19 mmol) was dissolved in hydrobromic acid (48%, 5 ml) and benzene (0.07 ml), and phenol (61.4 mg, 0.65 mmol) was added. The mixture was heated to reflux for three hours, and after cooling 20 ml of EtOAc was added. The layers were separated, and the aqueous layer washed with 20 ml of EtOAc. pH was adjusted to 11 with 10M sodium hydroxide. The aqueous layer was extracted with diethyl ether (3 x 20 ml), and the combined organic layers were dried over sodium sulphate and the solvent was evaporated. The crude product was dissolved in 5 ml of DCM and 0.5 ml of trifluoroacetic acid was added. The solvents were evaporated and the crude product was purified by preparative HPLC (method A1, Rt = 7.63 min). Evaporation of the solvent gave the title compound as an oil.
Yield: 8 mg (8%).
HPLC-MS (Method B): *m*/*z* = 383 (M+1); Rₜ = 2.00 min.

### Example 7

### (S) 8-(3-Aminopyrrolidin-1-yl)-7-(2-iodobenzyl)-1,3-dimethyl-3,7-dihydropurine-2,6-dione. HCl

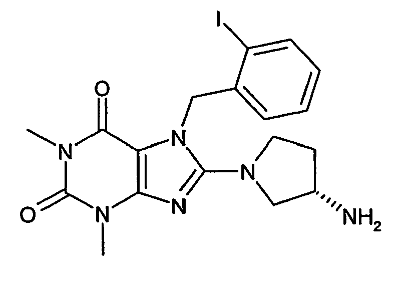

### Step A: (S) (1-(7-(2-Iodobenzyl)-1,3-dimethyl-2,6-dioxo-1,2,3,6-tetrahydropurin-8-yl)pyrrolidin-3-yl)carbamic acid tert-butyl ester (7A)

8-Chloro-7-(2-iodobenzyl)-1,3-dimethyl-3,7-dihydropurine-2,6-dione (5A) (100 mg, 0.23 mmol) and (3S)-(-)-3-(tert-butoxycarbonylamino)pyrrolidine (216 mg, 1.16 mmol), and triethylamine (0.32 ml, 2.32 mmol) were dissolved in 20 ml of 2-propanol and the mixture was subjected to microwaves (method F, 130°C, 300W) for three hours. The solvents were evaporated and the crude product was purified by preparative HPLC (method A1, Rt = 12.99 min.). Evaporation of the solvent afforded compound (7A) as white crystals.
Yield: 132 mg (98%).
HPLC-MS (Method B): *mlz* = 581 (M+1); Rₜ = 4.42 min.

### Step B: (S) 8-(3-Aminopyrrolidin-1-yl)-7-(2-iodobenzyl)-1,3-dimethyl-3,7-dihydropurine-2,6-dione. HCl (7)

(S) (1-(7-(2-Iodobenzyl)-1,3-dimethyl-2,6-dioxo-1,2,3,6-tetrahydropurin-8-yl)pyrrolidin-3-yl)carbamic acid tert-butyl ester (7A) (132 mg, 0.23 mmol) was reacted and purified as described in example **3**, step B, to give the title compound as white crystals.
Yield: 84 mg (72%). Mp. 119 - 223°C.
¹H-NMR (MeOD, 300 MHz) δ: 2.03 (m, 1 H); 2.34 (m, 1 H); 3.26 (s, 3H); 3.52 (m, 4H); 3.65 (m, 2H); 3.90 (m, 2H); 5.45 (d, 1 H); 5.52 (d, 1 H); 6.73 (d, 1 H); 7.04 (m, 1 H); 7.32 (m, 1 H); 7.92 (d, 1 H). HPLC-MS (Method B): *mlz =* 481 (M+1); Rₜ = 1.89 min.

### Example 8

### (S) 2-(8-(3-Aminopyrrolidin-1-yl)-1,3-dimethyl-2,6-dioxo-1,2,3,6-tetrahydropurin-7-ylmethyl)benzonitrile. HCl

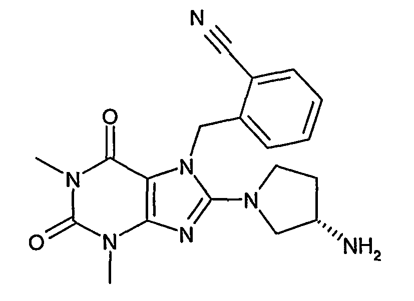

### Step A: (S) (1-(7-(2-Cyanobenzyl)-1,3-dimethyl-2,6-dioxo-1,2,3,6-tetrahydropurin-8-yl)pyrrolidin-3-yl)carbamic acid tert-buyl ester (8A)

2-(8-Chloro-1,3-dimethyl-2,6-dioxo-1,2,3,6-tetrahydropurin-7-ylmethyl)benzonitrile (1A) (100 mg, 0.30 mmol) was reacted with (3S)-(-)-3-(tert-butoxycarbonylamino)pyrrolidine (282 mg, 1.52 mmol), and purified as described in example **7,** step A, to afford compound (8A) as white crystals.
Yield: 117 mg (81%).
Prep. HPLC, (method A1): Rₜ =11.50 min.
HPLC-MS (Method B): *mlz* = 480 (M+1); Rₜ = 3.75 min.

### Step B: (S) 2-(8-(3-Aminopyrrolidin-1-yl)-1,3-dimethyl-2,6-dioxo-1,2,3,6-tetrahydropurin-7-ylmethyl)benzonitrile. HCl (8)

(S) (1-(7-(2-Cyanobenzyl)-1,3-dimethyl-2,6-dioxo-1,2,3,6-tetrahydropurin-8-yl)pyrrolidin-3-yl)carbamic acid tert-butyl ester (8A) (117 mg, 0.24 mmol) was reacted and purified as described in example **3,** step B, to give the title compound as white crystals.
Yield: 51 mg (50%). Mp.104 - 117°C.
¹H-NMR (MeOD, 300 MHz) δ: 2.08 (m, 1H); 2.40 (m, 1H); 3.26 (s, 3H); 3.52 (s, 3H); 3.53 - 3.78 (m, 3H); 3.92 (m, 2H); 5.71 (d, 1 H); 5.78 (d, 1 H); 7.13 (d, 1 H); 7.47 (m, 1H); 7.62 (m, 1 H); 7.80 (d, 1 H). HPLC-MS (Method B): *mlz =* 380 (M+1); Rₜ = 1.34 min.

### Example 9

### 8-(3-Aminopiperidin-1-yl)-7-(2-iodobenzyl)-1,3-dimethyl-3,7-dihydropurine-2,6-dione. TFA

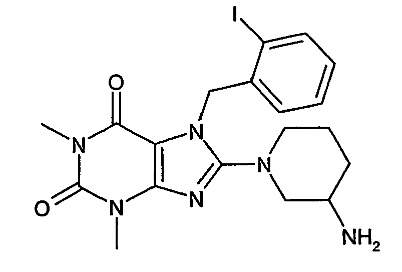

8-Chloro-7-(2-iodobenzyl)-1,3-dimethyl-3,7-dihydropurine-2,6-dione (5A) (100 mg, 0.23 mmol) and 3-aminopiperidine dihydrochloride (202 mg, 1.16 mmol) were reacted and purified as described in example **1,** step B, to give the title compound as oily brown crystals.
Yield: 19 mg (13%).
Prep. HPLC (method A1): Rₜ = 7.70 min.
¹H-NMR (MeOD, 300 MHz) δ: 1.62 (m, 2H); 1.74 (m, 1 H); 2.08 (m, 1 H); 2.94 (m, 1 H); 3.18 (m, 2H); 3.28 (s, 3H); 3.46 (m, 1 H); 3.54 (s, 3H); 3.70 (m, 1 H); 5.35 (s, 2H); 6.78 (d, 1H); 7.04 (m, 1 H); 7.32 (m, 1 H); 7.92 (d, 1 H).
HPLC-MS (Method B): *m*/*z* = 495 (M+1); Rₜ = 2.09 min.

### Example 10

### 8-(3-Aminopiperidin-1-yl)-7-(2-bromobenzyl)-1,3-dimethyl-3,7-dihydropurine-2,6-dione. TFA

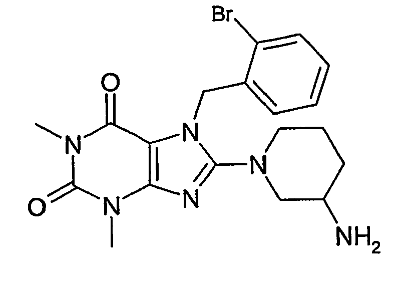

### Step A: 7-(2-Bromobenzyl)-8-chloro-1,3-dimethyl-3,7-dihydropurine-2,6-dione (10A)

8-Chlorotheophylline (10 g, 46.6 mmol) was dissolved in 250 ml of DMF and 8 ml of DIEA, and 2-bromobenzyl bromide (12.2 g, 48.9 mmol) was added. The mixture was stirred at 65°C for 2 hours. The reaction mixture was added 20 ml of EtOAc and 250 ml of cold water. The white precipitate was collected by filtration to afford compound (10A) as white crystals.
Yield: 17.2 g (96%). Mp. 165.4 - 166.7°C.
¹H-NMR (CDCl₃, 300 MHz) δ: 3.37 (s, 3H); 3.60 (s, 3H); 5.67 (s, 2H); 6.57 (d, 1H); 7.20 (m, 2H); 7.62 (d, 1 H). HPLC-MS (Method B): *mlz* = 385 (M+2); Rₜ = 3.77 min.

### Step B: 8-(3-Aminopiperidin-1-yl)-7-(2-bromobenzyl)-1,3-dimethyl-3,7-dihydropurine-2,6-dione. TFA (10)

7-(2-Bromobenzyl)-8-chloro-1,3-dimethyl-3,7-dihydropurine-2,6-dione (10A) (100 mg, 0.26 mmol) and 3-aminopiperidine dihydrochloride (226 mg, 1.31 mmol) were dissolved in 2-propanol (20 ml), triethylamine (0.109 ml, 0.78 mmol) and DMF (5 ml) and subjected to microwaves (method F, 130°C, 300W) for ten hours. The solvents were evaporated and the crude product was purified by preparative HPLC, (method A1, Rt = 7.52 min.) to give the title compound as a brown oil.
Yield: 10 mg (7%).
HPLC-MS (Method B): *mlz* = 447 (M+); Rₜ = 2.05 min.

### Example 11

### (R) 8-(3-Aminopyrrolidin-1-yl)-7-(2-bromobenzyl)-1,3-dimethyl-3,7-dihydropurine-2,6-dione. TFA

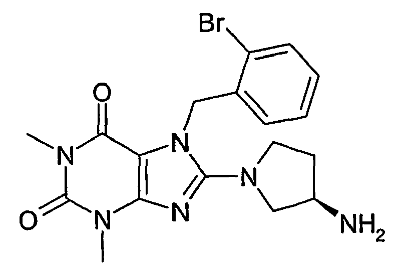

### Step A: (R) (1-(7-(2-Bromobenzyl)-1,3-dimethyl-2,6-dioxo-1,2,3,6-tetrahydropurin-8-yl)pyrrolidin-3-yl)carbamic acid tert-butyl ester (11 A)

7-(2-Bromobenzyl)-8-chloro-1,3-dimethyl-3,7-dihydropurine-2,6-dione (10A) (100 mg, 0.26 mmol) and (3R)-(+)-3-(tert-butoxycarbonylamino)pyrrolidine (243 mg, 1.30 mmol) were reacted and purified as described in example **3,** step A, to give compound (11A) as brown crystals.
Yield: 44 mg (32%). Mp. 104 - 106°C.
Prep. HPLC, (method A1): Rt = 12.66 min.
¹H-NMR (MeOD, 200 MHz) δ: 1.40 (s, 9H); 1.83 (m, 1H); 2.07 (m, 1H); 3.25 (s, 3H); 3.37 (m, 1 H); 3.48 - 3.78 (m, 6H); 4.04 (m, 1 H); 5.57 (s, 2H); 6.74 (d, 1H); 7.23 (m, 2H); 7.62 (m, 1H). HPLC-MS (Method B): *mlz* = 535 (M+2); Rₜ = 4.08 min

### Step B: (R) 8-(3-Aminopyrrolidin-1-yl)-7-(2-bromobenzyl)-1,3-dimethyl-3,7-dihydropurine-2,6-dione. TFA (11)

(R) (1-(7-(2-Bromobenzyl)-1,3-dimethyl-2,6-dioxo-1,2,3,6-tetrahydropurin-8-yl)pyrrolidin-3-yl)carbamic acid tert-butyl ester (11A) (44 mg, 0.08 mmol) was dissolved in MeCN (1 ml), water (1 ml), and TFA (0.32 ml), and the mixture was stirred at room temperature for 2 days. The solvents were evaporated and the crude product was purified by preparative HPLC (method A1, Rt = 6.92 min.) to give the title compound as a brown oil.
Yield: 13 mg (30%).
¹H-NMR (MeOD, 300 MHz) δ: 2.05 (m, 1 H); 2.35 (m, 1 H); 3.25 (s, 3H); 3.50 - 3.74 (m, 6H); 3.90 (m, 2H); 5.54 (d, 1 H); 5.61 (d, 1 H); 6.80 (dd, 1 H); 7.21 (dt, 1 H); 7.30 (dt, 1H); 7.63 (dd, 1H).
HPLC-MS (Method B): *mlz =* 433 (M+1); Rₜ = 1.83 min.

### Example 12

### (S) 8-(3-Aminopyrrolidin-1-yl)-7-(2-bromobenzyl)-1,3-dimethyl-3,7-dihydropurine-2,6-dione. HCl

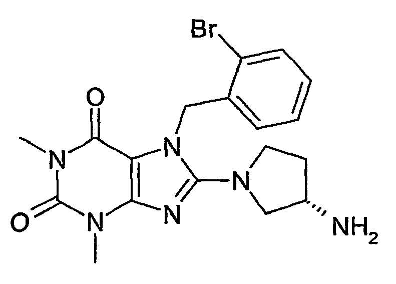

7-(2-Bromobenzyl)-8-chloro-1,3-dimethyl-3,7-dihydropurine-2,6-dione (10A) (100 mg, 0.26 mmol) and (S)-(-)-3-aminopyrrolidine (112 mg, 1.30 mmol) were dissolved in 2-propanol (20 ml) and DMF (5 ml) and subjected to microwaves (method F, 130°C, 300W) for 10 hours. The solvents were evaporated and the crude product was purified by preparative HPLC (method A1, Rt = 6.92 min.) to give the title compound as brown crystals.
Yield: 50 mg (41%). Mp. 215 - 217°C.
¹H-NMR (MeOD, 200 MHz) δ: 2.04 (m, 1H); 2.33 (m, 1H); 3.25 (s, 3H); 3.48 - 3.78 (m, 6H); 3.90 (m, 2H); 5.53 (d, 1 H); 5.60 (d, 1 H); 6.80 (dd, 1 H); 7.25 (m, 2H); 7.63 (dd, 1 H). HPLC-MS (Method B): *m*/*z* = 433 (M+); Rₜ = 1.80 min.

### Example 13

### (R) 8-(3-Aminopyrrolidin-1-yl)-7-benzyl-1,3-dimethyl-3,7-dihydropurine-2,6-dione. HCl

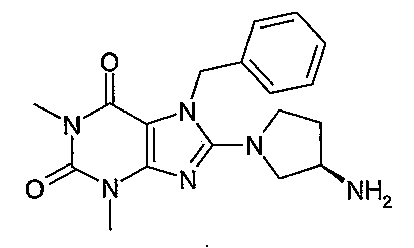

7-Benzyl-8-chloro-1,3-dimethyl-3,7-dihydropurine-2,6-dione (2A) (100 mg, 0.33 mmol) and (R)-(+)-3-aminopyrrolidine (141 mg, 1.64 mmol) were reacted and purified as described in example **12** to give the title compound as brown crystals.
Yield: 73 mg (57%). Mp. 103 - 114°C.
Prep. HPLC, (method A1): Rt = 6.38 min.
¹H-NMR (MeOD, 200 MHz) δ: 2.08 (m, 1H); 2.35 (m, 1H); 3.27 (s, 3H); 3.49 (s, 3H); 3.55 - 4.00 (m, 5H); 5.52 (d, 1H); 5.63 (d, 1H); 7.12 (m, 2H); 7.29 (m, 3H). HPLC-MS (Method B): *m*/*z* = 355 (M+1); Rₜ = 1.55 min.

### Example 14

### (R) 2-(8-(3-Aminopyrrolidin-1-yl)-1,3-dimethyl-2,6-dioxo-1,2,3,6-tetrahydropurin-7-ylmethyl)benzonitrile. HCl

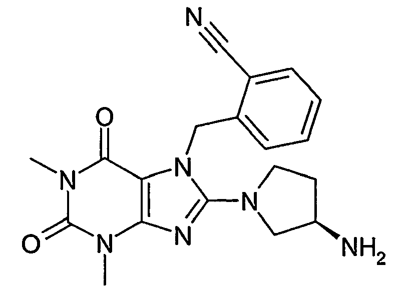

2-(8-Chloro-1,3-dimethyl-2,6-dioxo-1,2,3,6-tetrahydropurin-7-ylmethyl)benzonitrile (1A) (100 mg, 0.30 mmol) and (R)-(+)-3-aminopyrrolidine (131 mg, 1.57 mmol) were reacted and purified as described in example **2**, step B, to give the title compound as brown crystals.
Yield: 125 mg (99%). Mp. 202 - 204°C.
Prep. HPLC, (method A1): Rt = 6.17 min.
¹H-NMR (MeOD, 200 MHz) δ: 2.12 (m, 1H); 2.41 (m, 1H); 3.22 (s, 3H); 3.49 (s, 3H); 3.55 - 4.04 (m, 5H); 5.70 (d, 1H); 5.78 (d, 1H); 7.11 (d, 1H); 7.47 (t, 1H); 7.61 (t, 1H); 7.78 (d, 1H). HPLC-MS (Method B): *m*/*z* = 380 (M+1); Rₜ = 1.38 min.

### Example 15

### (R) 8-(3-Aminopyrrolidin-1-yl)-7-(2-iodobenzyl)-1,3-dimethyl-3,7-dihydropurine-2,6-dione. HCl

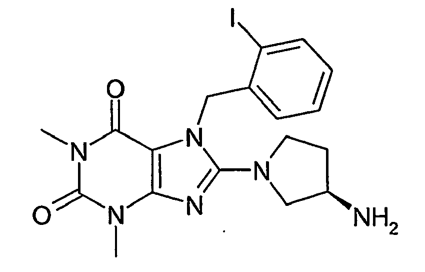

8-Chloro-7-(2-iodobenzyl)-1,3-dimethyl-3,7-dihydropurine-2,6-dione (5A) (100 mg, 0.23 mmol) and (R)-(+)-3-aminopyrrolidine (100 mg, 1.16 mmol) were reacted and purified as described in example **2**, step B, to give the title compound as white crystals.
Yield: 61 mg (51%). Mp. 233 - 235°C.
Prep. HPLC, (method A1): Rt = 7.24 min.
¹H-NMR (MeOD, 200 MHz) δ: 2.05 (m, 1H); 2.34 (m, 1H); 3.25 (s, 3H); 3.46 - 3.76 (m, 6H); 3.90 (m, 2H); 5.43 (d, 1H); 5.52 (d, 1H); 6.72 (dd, 1H); 7.03 (dt, 1H); 7.32 (dt, 1H); 7.91 (dd, 1H). HPLC-MS (Method B): *mlz* = 481 (M+1); Rₜ = 1.88 min.

### Example 16 (General procedure (E))

### Cis-8-(2-Aminocyclohexylamino)-7-benzyl-3-methyl-1-(2-oxo-2-phenylethyl)-3,7-dihydropurine-2,6-dione

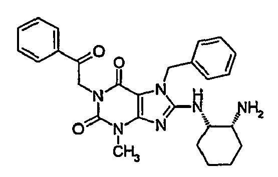

¹H NMR (DMSO-*d*₆): δ 8.10 - 8.01 (m, 2H); 7.82 (s br, 3H); 7.71 (t, 1H); 7.57 (t, 2H); 7.38 - 7.17 (m, 5H); 6.73 (d, 1H); 5.51 - 5.23 (m, 4H); 4.29 - 4.17 (m, 1 H); 3.59 (s br, 1 H); 3.42 (s, 3H); 1.89 - 1.29 (m, 8H). HPLC-MS (Method Anyone): *m*/*z* = 487 (M+1); Rₜ = 3.087 min

### Example 17 (General procedure (E))

### Cis-8-(2-Aminocyclohexylamino)-7-(2-chlorobenzyl)-1-(2-hydroxy-2-phenylethyl)-3-methyl-3,7-dihydropurine-2,6-dione

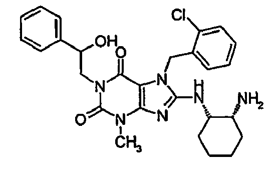

### Step B:

Styrene oxide was employed instead of R⁵-X
¹H NMR (DMSO-*d*₆): δ7.79 (s br, 3H); 7.55 - 7,48 (m, 1H); 7,38 - 7,15 (m, 7H); 6,81 - 6,71 (m, 1H); 6,63 - 6,54 (m, 1H); 5.59 - 5.35 (m, 2H); 4.93 - 4.81 (m, 1H); 4.24 (s br, 1H); 4.14 - 4.04 (m, 1H); 3.41 (s, 3H); 1.86 -1.29 (m, 8H). HPLC-MS (Method Anyone): *m*/*z* = 523 (M+1); Rₜ = 3.058 min.

### Example 18 (General procedure (G))

### Trans-8-(2-(S)-Amino-cyclohexyl-(S)-amino)-7-(2-iodo-benzyl)-3-methyl-3,7-dihydro-purine-2,6-dione

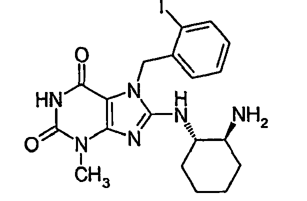

¹H NMR (DMSO-*d*₆): δ 10.68 (s, 1H); 9.92 (d, 1H); 7.85 ( s br, 3H); 7.32 (t, 1H); 7.12 - 6.97 (m, 2H); 6.42 (d, 1H); 5.36 - 4.96 (dd, 2H); 3.86 - 3.68 (m, 1H); 3.36 (s, 3H); 3.09 - 2.93 (m, 1H) 2.08- 1.12 (m, 8H). HPLC-MS (Method Anyone): *m*/*z* = 495 (M+1); Rₜ = 2.313 min.

### Example 19 (General procedure (G))

### Trans-8-(2-(R)-Amino-cyclohexyl-(R)-amino)-7-(2-iodo-benzyl)-3-methyl-3,7-dihydro-purine-2,6-dione

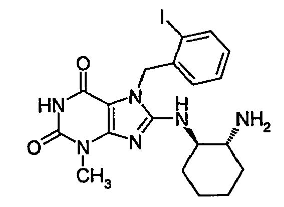

¹H NMR (DMSO-*d*₆): δ 10.68 (s, 1H); 7.92 (d, 1H); 7.85 (s br, 3H); 7.33 (t, 1H); 7.10-7.00 (m, 2H); 6.42 (m, 1H); 5.29 (d, 1H); 5.03 (d, 1 H); 3.77 (m, 1H); 3.36 (s, 3H); 3.01 (m, 1H); 1.98 (m, 2H); 1.69 (m, 2H); 1.42 (m, 1H); 1.24 (m, 3H). HPLC-MS (Method h8): *m*/*z* = 495 (M+1); Rₜ = 3.70 min.

### Example 20 (General procedure (G))

### Cis-8-(2-Amino-cyclohexylamino)-7-(2-iodo-benzyl)-3-methyl-3,7-dihydro-purine-2,6-dione

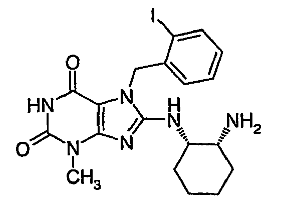

¹H NMR (DMSO-*d*₆): δ 10.67 (s, 1H); 7.91 (d, 1H); 7.76 ( s br, 3H); 7.31 (t, 1H); 7.04 (t, 1H); 6.73 (d, 1H); 6.44 (d, 1H); 5.39 - 5.14 (m, 2H); 1.06 (s br, 1H); 3.59 (s br, 1H); 3.35 (s, 3H); 1.86-1-28 (m, 8H). HPLC-MS (Method Anyone): *m*/*z* = 495 (M+1) Rₜ = 2.313

### Example 21 (General procedure (G))

### Trans-8-(2-(S)-Amino-cyclohexyl-(S)-amino)-7-biphenyl-2-ylmethyl-3-methyl-3,7-dihydro-purine-2,6-dione

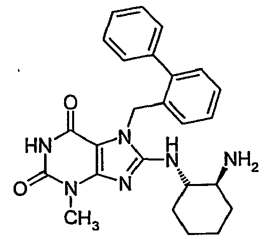

¹H NMR (DMSO-*d*₆): δ 10.58 (s, 1H); 7.87 (s br, 3H); 7.55-7.23 (m, 7H); 7.03 (d, 1H); 6.58 (d, 1H); 5.37 (d, 1H); 5.11 (d, 1H); 3.78 (m, 1H); 3.34 (s, 3H); 3.02 (m, 1H); 2.03 (m, 2H); 1.74 (m, 2H); 1.45 (m, 1H); 1.26 (m, 3H). HPLC-MS (Method h8): *m*/*z* = 445 (M+1); Rₜ = 4.03 min.

### Example 22 (General procedure (G))

### Cis-8-(2-Amino-cyclohexylamino)-7-biphenyl-2-ylmethyl-3-methyl-3,7-dihydro-purine-2,6-dione

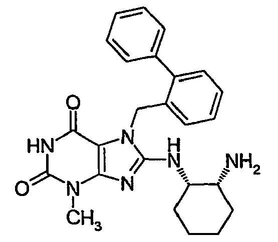

¹H NMR (DMSO-*d*₆): δ 10.57 (s, 1H); 7.79 (s br, 3H); 7.50-7.22 (m, 8H); 6.66 (d, 1H); 6.54 (d, 1H); 5.39 (d, 1H); 5.24 (d, 1H); 4.22 (m, 1H); 3.55 (m, 1H); 3.32 (s, 3H); 1.80-1.30 (m, 8H). HPLC-MS (Method h8): *m*/*z* = 445 (M+1); Rₜ = 3.92.

### Example 23 (General procedure (G))

### Cis-8-(2-(S)-Amino-cyclohexyl-(S)-amino)-7-(2-bromo-benzyl)-3-methyl-3,7-dihydro-purine-2,6-dione

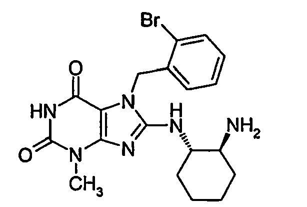

¹H NMR (DMSO-*d*₆): δ 10.68 (s, 1H); 7.87 (s br, 3H); 7.69 (d, 1H); 7.37 - 7.19 (m, 2H); 7.045 (d, 1H); 6.51 (d, 1H); 5.46 - 5.08 (dd, 2H); 3.87 - 3.71 (m, 1H); 3.36 (s, 3H); 3.10 - 2.92 (m, 1H); 2.09 - 1.09 (m, 8H). HPLC-MS (Method Anyone): *m*/*z* = 449 (M+1); Rₜ = 1.932 min.

### Example 24 (General procedure (G))

### Cis-8-(2-Amino-cyclohexylamino)-7-(2-bromo-benzyl)-3-methyl-3,7-dihydro-purine-2,6-dione

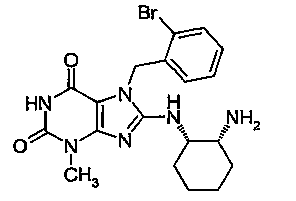

¹H NMR (DMSO-*d*₆): δ 10.67 (s, 1H); 7.77 (s br, 3H); 7.67 ( d, 1H); 7.36 - 7.17 (m, 2H); 6.74 (d, 1H); 5.51 - 5.26 (dd, 2H); 4.22 (s br, 1H); 3.58 (s br, 1H); 3.35 (s,3H); 1.87 - 1.28 (m, 8H). HPLC-MS (Method Anyone): *m*/*z* = 449 (M+1); Rₜ = 1.926

### Example 25 (General procedure (G))

### Trans-8-(2-(S)-Amino-cyclohexyl-(S)-amino)-7-(2-chloro-benzyl)-3-methyl-3,7-dihydro-purine-2,6-dione

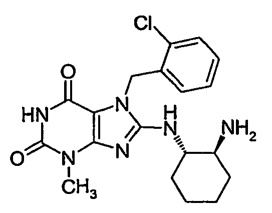

¹H NMR (DMSO-*d*₆): δ 10.68 (s br, 1H); 7.86 (s br, 3H); 7.56 - 7.48 (m, 1H); 7.37- 7.22 (m, 2H); 7.10 - 6.99 (m, 1H); 6.61 - 6.52 (m, 1H) 1.51 - 5.15 (dd, 2H); 3.86 - 3.69 (m. 1H); 3.36 (s, 3H); 3.08 - 2.93 (m, 1H); 2.09 - 1.12 (m, 8H). HPLC-MS (Method Anyone): *m*/*z* = 403 (M+1); Rₜ 2.184 min.

### Example 26 (General procedure (G))

### Trans-8-(2-(R)-Amino-cyclohexyl-(R)-amino)-7-(2-chloro-benzyl)-3-methyl-3,7-dihydro-purine-2,6-dione

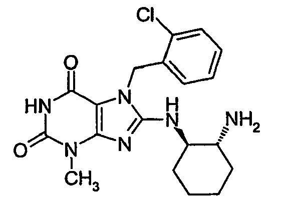

¹H NMR (DMSO-*d*₆): δ 10.68 (s, 1H); 7.92 (s br, 3H); 7.52 (d, 1H); 7.30 (t+t, 2H); 7.08 (d, 1H); 6.57 (d, 1H); 5.44 (d, 1H); 5.21 (d, 1H); 3.77 (m, 1H); 3.36 (s, 3H); 3.02 (m, 1H); 2.00 (m, 2H); 1.68 (m, 2H); 1.42 (m, 1H); 1.23 (m, 3H).

### Example 27 (General procedure (G))

### Cis-8-(2-Amino-cyclohexylamino)-7-(2-chloro-benzyl)-3-methyl-3,7-dihydro-purine-2,6-dione

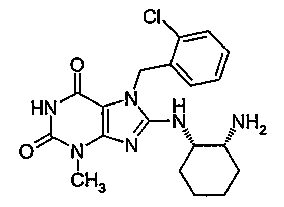

¹H NMR (DMSO-*d*₆): δ 10.68 (s br, 1H); 7.75 (s br, 3H); 7.505 (dd, 1H); 7.35 - 7.22 (m, 2H); 7.76 - 6.58 (m, 2H); 5.52 - 5.33 (dd, 2H); 4.22 (s br, 1H); 3.58 (s, 1H); 3.14 (s, 3H); 1.87 - 1.27 (m, 8H). HPLC-MS (Method Anyone): *m*/*z* = 403 (M+1); Rₜ = 2.192 min.

### Example 28 (General procedure (E))

### Cis-8-(2-Amino-cyclohexylamino)-1,7-bis-(2-chloro-benzyl)-3-methyl-3,7-dihydro-purine-2,6-dione

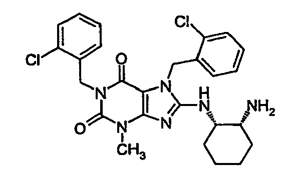

¹H NMR (DMSO-*d*₆): δ 7.79 (s br, 3H); 7.50-7.37 (m, 2H); 7.35-7.10 (m, 4H); 6.86 (d, 1H); 6.77 (d, 1H); 5.58 (d, 1H); 5.46 (dd, 2H); 4.99 (s, 2H); 4.27 (m, 1H); 3.60 (m, 1H); 3.46 (s, 3H); 1.80-1.30 (m, 8H). (Method h8): *m*/*z* = 527 (M+1); Rₜ = 5.12 min.

### Example 29 (General procedure (E))

### Cis-2-[8-(2-Amino-cyclohexylamino)-7-(2-chloro-benzyl)-3-methyl-2,6-dioxo-2,3,6,7-tetrahydro-purin-1-ylmethyl]-benzonitrile

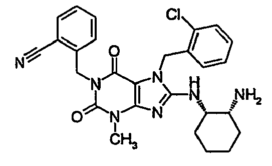

¹H NMR (DMSO-*d*₆): δ 7.80 (s + d, 4H); 7.57 (t, 1H); 7.50 (d, 1H); 7.41 (t, 1H); 7.29 (t +t, 2H); 7.09 (d, 1H); 6.86 (d, 1H); 6.68 (d, 1H); 5.48 (dd, 2H); 5.12 (s, 2H); 4.26 (m, 1H); 3.60 (m, 1H); 3.44 (s, 3H); 1.80-1.35 (m, 8H). (Method h8): *m*/*z* = 518 (M+1); Rₜ = 4.72 min.

### Example 30 (General procedure (E))

### Cis-8-(2-Amino-cyclohexylamino)-7-(2-chloro-benzyl)-3-methyl-1-(2-oxo-2-phenyl-ethyl)-3,7-dihydro-purine-2,6-dione

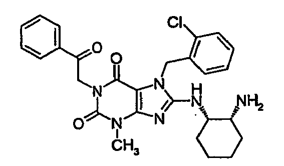

¹H NMR (DMSO-*d*₆): δ 8.01 (d, 2H); 7.77 (s br, 3H); 7.69 (t, 1H); 7.55 (t, 2H); 7.49 (d, 1H); 7.29 m, 2H); 6.86 (d, 1H); 6.69 (d, 1H); 5.46 (dd, 2H); 5.25 (dd, 2H): 4.28 (m, 1 H); 3.64 (m, 1H); 3.46 (s, 3H); 1.80-1.30 (m, 8H). (Method h8): *m*/*z* = 521 (M+1); Rₜ = 4.85 min.

### Example 31 (General procedure (E))

### Cis-8-(2-Amino-cyclohexylamino)-7-(2-chloro-benzyl)-3-methyl-1-phenethyl-3,7-dihydro-purine-2,6-dione

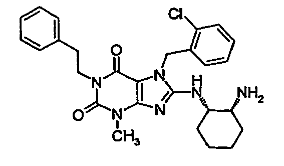

¹H NMR (DMSO-*d*₆): δ 7.78 (s br, 3H); 1.52 (d, 1H); 7.35-7.24 (m, 4H); 7.24-7.12 (m, 3H); 6.79 (d, 1 H); 6.61 (d, 1 H); 5.47 (dd, 2H); 4.24 (m, 1 H); 3.94 (t, 2H); 3.59 (m, 1 H); 3.43 (s, 3H); 2.73 (t 1 H); 1.80-1.30 (m, 8H). (Method h8): *m*/*z* = 507 (M+1); Rₜ = 5.10 min.

### Example 32 (General procedure (E))

### Cis-8-(2-Amino-cyclohexylamino)-7-(2-bromo-benzyl)-1-(2-chloro-benzyl)-3-methyl-3,7-dihydro-purine-2,6-dione

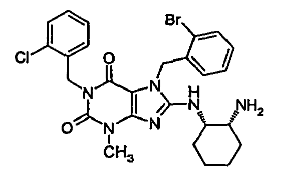

¹H NMR (DMSO-*d*₆): δ 7.76 (s br, 3H); 7.66 (d, 1H); 7.42 (d, 1H); 7.40-7.15 (m, 4H); 6.87 (d, 1H); 6.77 (d, 1H); 6.62 (d, 1H); 5.41 (dd, 2H); 4.98 (s, 2H); 4.27 (m, 1 H); 3.61 (m, 1H); 3.46 (s, 3H); 1.80-1.35 (m, 8H). (Method h8): *m*/*z* = 573 (M+1); Rₜ = 5.37 min

### Example 33 (General procedure (E))

### Cis-2-[8-(2-Amino-cyclohexylamino)-7-(2-bromo-benzyl)-3-methyl-2,6-dioxo-2,3,6,7-tetrahydro-purin-1-ylmethyl]-benzonitrile

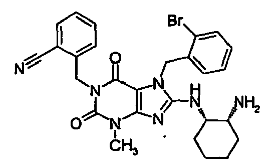

¹H NMR (DMSO-*d*₆): δ 7.78 (d, 1H); 7.74 (s br, 3H); 7.67 (d, 1H); 7.57 (t, 1H); 7.41 (t, 1H); 7.31 (t, 1H); 7.22 (t, 1H); 7.09 (d, 1H); 6.86 (d, 1H); 6.61 (d, 1H); 5.42 (dd, 2H); 5.11 (s, 2H); 4.26 (m, 1H); 3.61 (m, 1H); 3.45 (s, 3H); 1.80-1.35 (m, 8H). (Method h8): *m*/*z* = 562 (M+1); Rₜ = 4.88

### Example 34 (General procedure (E))

### Cis-8-(2-Amino-cyclohexylamino)-7-(2-bromo-benzyl)-3-methyl-1-(2-oxo-2-phenyl-ethyl)-3,7-dihydro-purine-2,6-dione

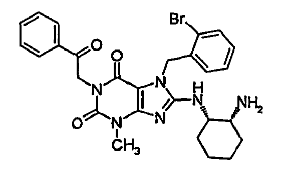

¹H NMR (DMSO-*d*₆): δ 8.01 (d, 2H); 7.74 (s br, 3H); 7.67 (m, 2H); 7.55 (m, 2H); 7.32 (t, 1H); 7.25 (t, 1H); 6.88 (d, 1H); 6.61 (d, 1H); 5.41 (dd, 2H); 5.25 (dd, 2H); 4.28 (m, 1H); 3.63 (m, 1H); 3.46 (s, 3H); 1.80-1.35 (m, 8H). (Method h8): *m*/*z* = 567 (M+1); Rₜ = 5.02 min.

### Example 35 (General procedure (E))

### Cis-8-(2-Amino-cyclohexylamino)-7-(2-bromo-benzyl)-3-methyl-1-phenethyl-3,7-dihydro-purine-2,6-dione

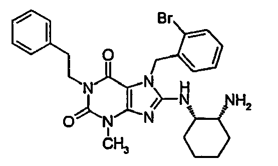

¹H NMR (DMSO-*d*₆): δ 7.75 (s br, 3H); 7.69 (d, 1H); 7.35-7.10 (m, 7H); 6.80 (d, 1H); 6.54 (d, 1H); 5.43 (dd, 2H); 4.23 (m, 1H); 3.94 (t, 2H); 3.61 (m, 1H); 3.43 (s, 3H); 2.73 (2H); 1.80-1.30 (m, 8H). (Method h8): *m*/*z* = 551 (M+1); Rₜ = 5.28 min.

### IN VIVO TESTING OF COMBINATION TREATMENT

### Methods

Non-fasted anaesthetised pigs (n = 5) were given valine-pyrrolidide (250 µmol/kg; V) to inhibit DPP IV activity throughout the experiment. An intraveneous (iv) infusion of GLP-1 (0.75 pmol/kg/min) was started, during which an iv glucose load (0.2 g/kg over 9 min) was given. 90 min after the end of the first GLP-1 infusion, candoxatril was administered iv (5 mg/kg; C), and the protocol (GLP-1 and glucose infusion) was repeated. In addition, during both GLP-1 infusions, simultaneous blood samples were taken from the carotid artery, the renal, femoral, hepatic and portal veins for determination of arterio-venous GLP-1 concentration differences. Samples were analysed for blood glucose, insulin, glucagon and GLP-1 concentrations.

### Results

Compared to V alone, co-administration of C significantly increased the plasma levels (plateau level, 73 ± 13 vs 122 ± 22 pmol/l; P < 0.018; Fig 1) and the plasma t½ (2.5 ± 0.2 vs 8.3 ± 0.9 min; P < 0.002) of GLP-1 The metabolic clearance rate of GLP-1 during DPP IV inibition alone (25.1 ± 3.9 ml/kg/min) was significantly (P < 0.022) reduced during combined DPP IV and NEP 24.11 inhibition (11.7 ± 1.2 ml/kg/min). Calculation of the individual organ extractions revealed that renal clearance was reduced (P < 0.047) by combined DPP IV and NEP 24.11 inhibition (44.1 ± 5.4% compared to 58.7 ± 4.3% for DPP IV inhibition alone). Extraction across the extremities, portal bed or liver was not affected by dual DPP IV/ NEP 24.11 inhibition compared to DPP IV inhibition alone. Combined VP+C treatment significantly (P < 0.016) reduced the glucose excursion (ΔAUC_{27-67 min}, 28 ± 10 mmol/l x min; Fig 2) compared to VP alone (ΔAUC_{27-67 min}, 59 ± 4 mmol/l x min), and the glucose elimination rate was increased (11.6 ± 1.3 vs 6.6 ± 0.5 %/min, VP+C vs VP alone, P < 0.016). VP+C significantly (P < 0.008) potentiated insulin secretion (AUC_{27-7 min}, 3606 ± 668 vs 6486 ± 1064 pmol/l x min, VP vs VP+C; Fig 3). Interestingly, glucagon concentrations were also elevated after dual inhibition of DPP IV and NEP24.11 compared to DPP IV inhibition alone, increasing from 9 ± 2 to 18 ± 2 pmol/l (P < 0.045; Fig 4) in the 20 min period following C administration (before the start of the GLP-1 infusion). The overall glucagon excursion was also greater (P < 0.030) with dual DPP IV/NEP 24.11 inhibition (AUC_{0-107 min}, 3007 ± 775 mmol/l x min) compared to DPP IV inhibition alone (AUC_{0-107 min}, 585 ± 185 mmol/l x min; Fig 4).

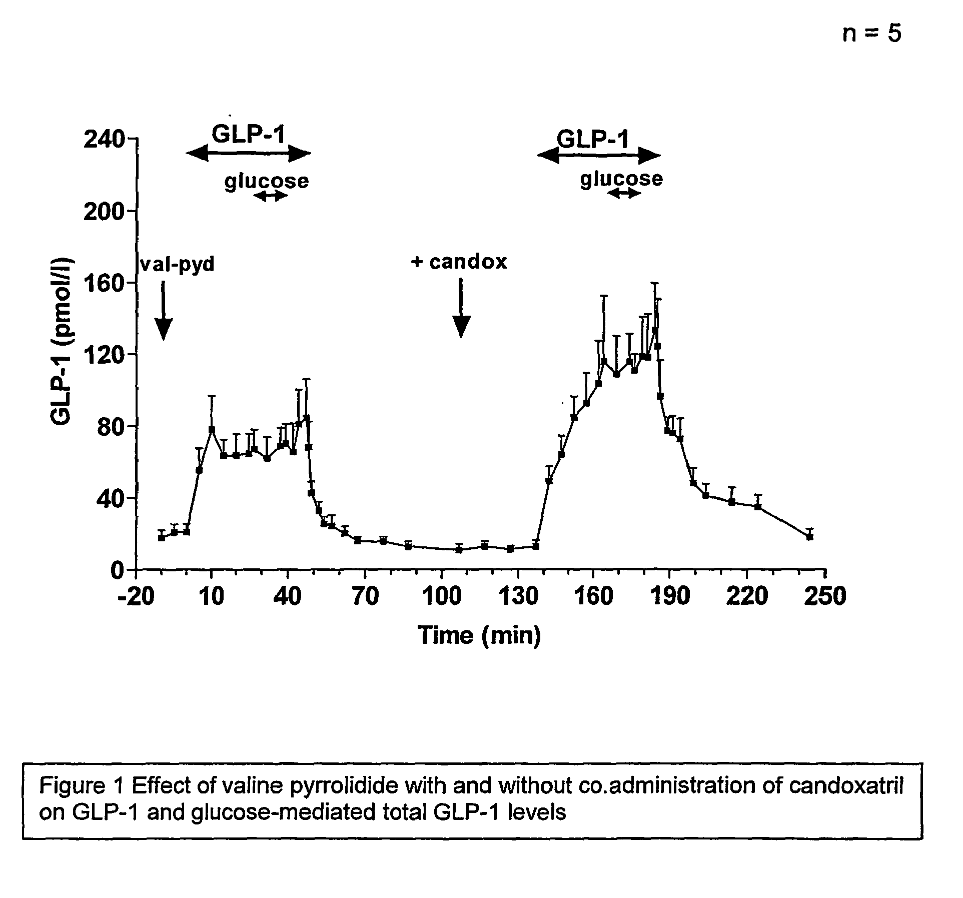

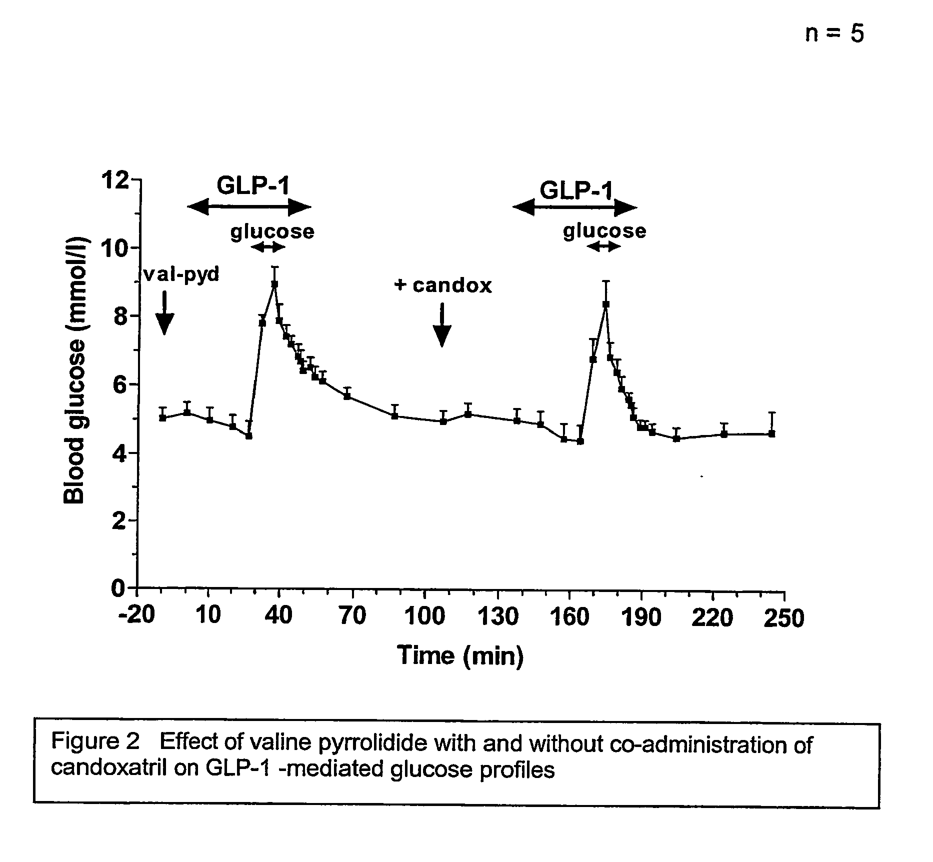

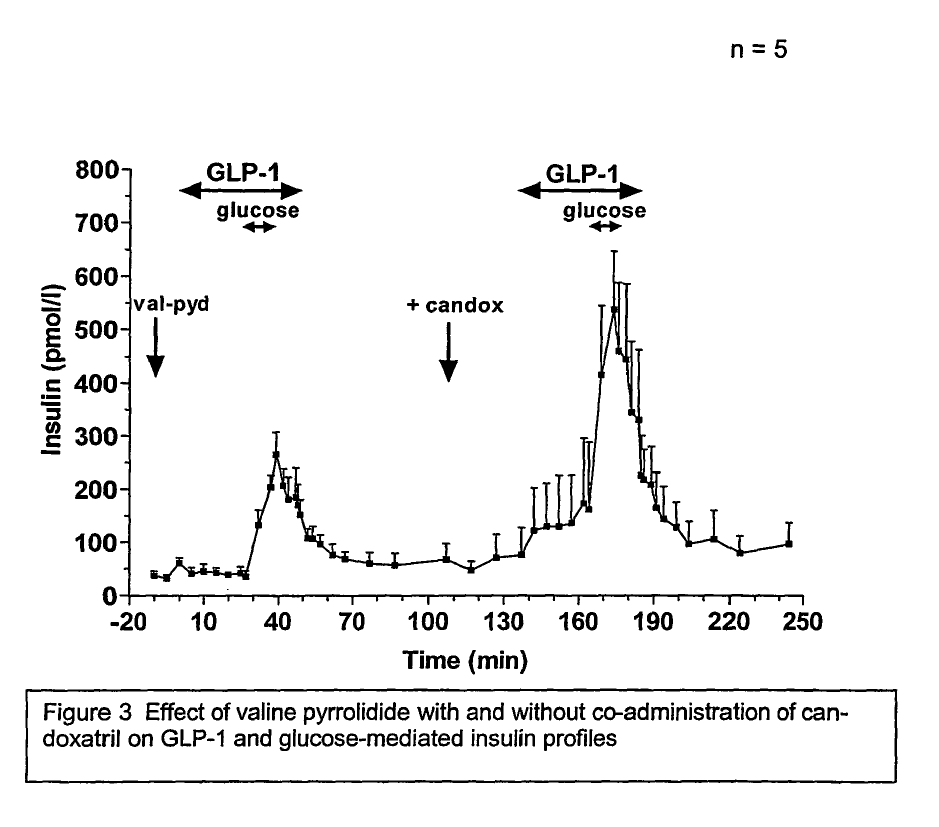

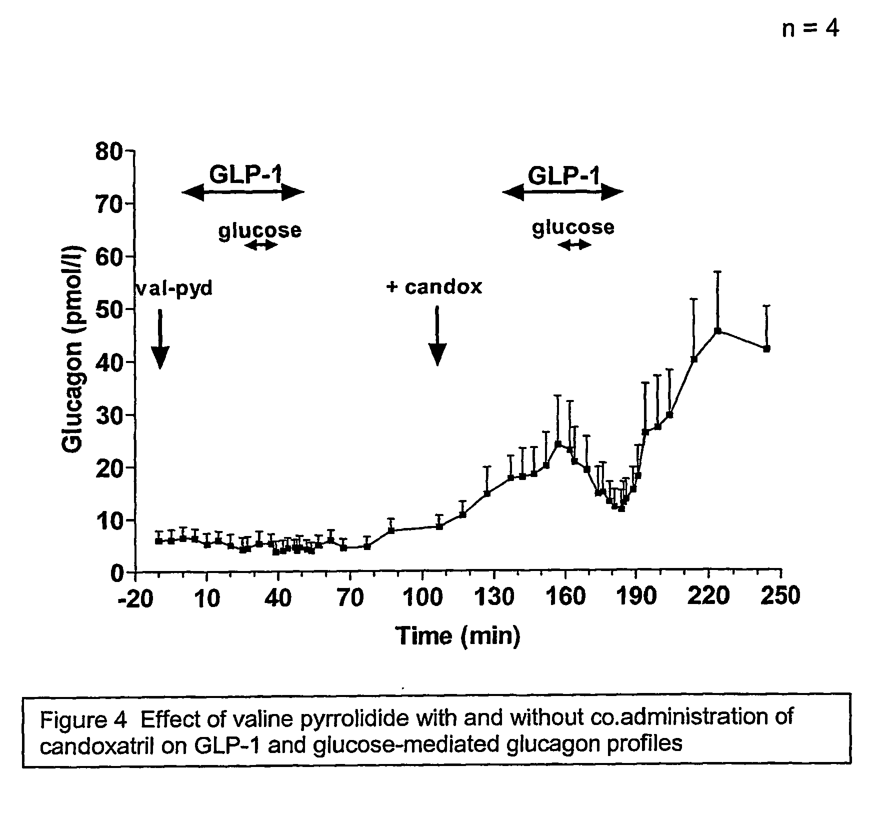

### PHARMACOLOGICAL METHODS

### Methods for measuring the activity of compounds which inhibit the enzymatic activity of CD26/DPP-IV

### Summary.

Chemical compounds are tested for their ability to inhibit the enzyme activity of purified CD26/DPP-IV. Briefly, the activity of CD26/DPP-IV is measured *in vitro* by its ability to cleave the synthetic substrate Gly-Pro-p-nitroanilide (Gly-Pro-pNA). Cleavage of Gly-Pro-pNA by

DPP-IV liberates the product p-nitroanilide (pNA), whose rate of appearance is directly proportional to the enzyme activity. Inhibition of the enzyme activity by specific enzyme inhibitors slows down the generation of pNA. Stronger interaction between an inhibitor and the enzyme results in a slower rate of generation of pNA. Thus, the degree of inhibition of the rate of accumulation of pNA is a direct measure of the strength of enzyme inhibition. The accumulation of pNA is measured spectrophotometrically. The inhibition constant, Ki, for each compound is determined by incubating fixed amounts of enzyme with several different concentrations of inhibitor and substrate.

### Materials:

The following reagents and cells are commercially available:
Porcine CD26/DPP-IV (Sigma D-7052), Gly-Pro-pNA (Sigma G0513).
Assay buffer: 50 mM Tris pH 7.4, 150 mM NaCl, 0,1% Triton X-100.

### Gly-Pro-pNA cleavage-assay for CD26:

The activity of purified CD26/DPP-IV is assayed in reactions containing:
70 µl assay buffer
10 µl inhibitor or buffer
10 µl substrate (Gly-Pro-pNA from a 0.1 M stock solution in water) or buffer
10 µl enzyme or buffer

Reactions containing identical amounts of enzyme, but varying concentrations of inhibitor and substrate, or buffer as control, are set up in parallel in individual wells of a 96-well ELISA plate. The plate is incubated at 25 °C and absorbance is read at 405 nm after 60 min incubation. The inhibitor constants are calculated by non-linear regression hyperbolic fit and the result is expressed as inhibition constant (Ki) in nM.

### Diabetes model

The Zucker Diabetic Fatty (ZDF) rat model can be used to investigate the effects of the compounds of the invention on both the treatment and prevention of diabetes as rats of this sub-strain are initially pre-diabetic although develop severe type 2 diabetes characterised by increased HbA1c levels over a period of 6 weeks. The same strain can be used to predict the clinical efficacy of other anti-diabetic drug types. For example, the model predicts the potency and limited clinical efficacy of thiazolidinedione insulin sensitizers compounds.

## Claims

1. A pharmaceutical preparation comprising a combination of a Dipeptidyl Peptidase IV inhibitor and a Neutral Endopeptidase inhibitor, or a pharmaceutically acceptable salt of either or both of these.

2. A pharmaceutical preparation according to claim 1 wherein the Dipeptidyl Peptidase IV is a N-substituted adamantyl-amino-acetyl-2-cyano pyrrolidine or a N-(substituted glycyl)-4-cyano pyrrolidine.

3. A pharmaceutical preparation according to claim 1 wherein the Dipeptidyl Peptidase IV is a compound of formula I 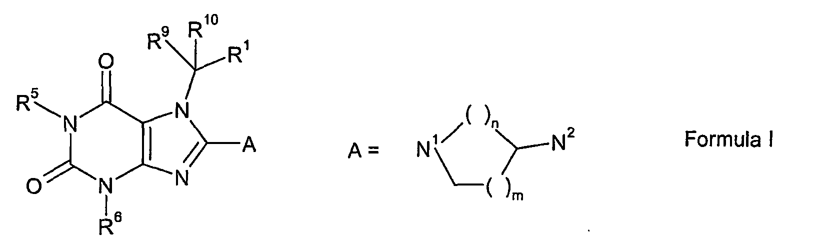 wherein A may be attached at either N1 or at N2 to the purine system and
each n and m is one or two independently
R1 is aryl optionally substituted with one or more R2 independently or heteroaryl optionally substituted with one or more R2 independently;
R2 is H; C1-C7 alkyl; C2-C7 alkenyl; C2-C7 alkynyl; C3-C7 cycloalkyl; C3-C7 cycloheteroalkyl; -NHCOR3; -NHS02R3; -SR3; -SOR3; -SO2R3; -OCOR3; -CO2R4; -CON(R4)2; -CSN(R4)2; -NHCON(R4)2; -NHCSN(R4)2; -NHCONNH2; -SO2N(R4)2; -OR4; cyano; nitro; halogen, wherein each alkyl, alkenyl, alkynyl, cycloalkyl and cycloheteroalkyl is optionally substituted with one or more R3 independently;
R3 is C1-C10 alkyl; C2-C10 alkenyl; C2-C10 alkynyl; C3-C7 cycloalkyl; aryl; heteroaryl; OR11; N(R11)2; SR11, wherein each alkyl, alkenyl, alkynyl, cycloalkyl, aryl and heteroaryl is substituted with one or more R11 independently;
R4 is H; C1-C10 alkyl; C2-C10 alkenyl; C2-C10 alkynyl; C3-C7 cycloalkyl; C3-C7 cycloheteroalkyl; aryl; aryl-C1-C5 alkyl; heteroaryl; heteroaryl-C1-C5 alkyl, wherein each alkyl, alkenyl, alkynyl, cycloalkyl, cycloheteroalkyl, aryl, aryl-C1-C5 alkyl, heteroaryl, and heteroaryl-C1-C5 alkyl is substituted with one or more R11 independently;
R5 is H; C1-C10 alkyl; C2-C10 alkenyl; C2-C10 alkynyl; C3-C7 cycloalkyl; C3-C7 cycloalkyl-C1-C5 alkyl; C3-C7 cycloheteroalkyl; C3-C7 cycloheteroalkyl-C1-C5 alkyl; aryl; heteroaryl; aryl-C1-C5 alkyl; heteroaryl-C1-C5 alkyl; -OR7; -[(CH2)o-O]p-alkyl, wherein o and p are 1-3 independently, and wherein each alkyl, alkenyl, alkynyl, cycloalkyl, cycloalkyl-C1-C5 alkyl, cycloheteroalkyl, C3-C7 cycloheteroalkyl-C1-C5 alkyl, aryl, aryl-C1-C5 alkyl, heteroaryl, aryl-C1-C5 alkyl, and heteroaryl-C1-C5 alkyl is optionally substituted with one or more R7 independently;
R6 is C1-C10 alkyl; C2-C10 alkenyl; C2-C10 alkynyl; C3-C7 cycloalkyl; C3-C7 cycloheteroalkyl; aryl; heteroaryl; aryl-C1-C5 alkyl; heteroaryl-C1-C5 alkyl; C3-C7 cycloheteroalkyl-C1-C5 alkyl, wherein each alkyl, alkenyl, alkynyl, cycloalkyl, cycloheteroalkyl, C3-C7 cycloheteroalkyl-C1-C5 alkyl, aryl, aryl-C1-C5 alkyl, heteroaryl, and heteroaryl-C1-C5 alkyl is optionally substituted with one or more R11 independently;
R7 is H; =O; C1-C10 alkyl; C2-C10 alkenyl; C2-C10 alkynyl; C3-C7 cycloalkyl; C3-C7 cycloheteroalkyl; aryl; heteroaryl, OR11; N(R11)2; SR11, wherein each alkyl, alkenyl, alkynyl, cycloalkyl, cycloheteroalkyl, aryl, and heteroaryl is optionally substituted with one or more R11 independently;
R8 is C1-C10 alkyl; C2-C10 alkenyl; C2-C10 alkynyl; C3-C7 cycloalkyl; C3-C7 cycloheteroalkyl; aryl; heteroaryl, OR11; N(R11)2; SR11, wherein each alkyl, alkenyl, alkynyl, cycloalkyl, cycloheteroalkyl, aryl, and heteroaryl is optionally substituted with one or more R11 independently;
R9 and R10 is independently H, C1-C10 alkyl optionally substituted with one or more R8 independently, halogen;
R11 is H; -CF3; -CCl3; -OCF3; -OMe; cyano; halogen; -OH, COMe; -CONH2; CONHMe; CONMe2; -NO2;
If R9 and R10 is C1-C10 alkyl they may be connected to form a cyclopropyl ring;
if two R4 or two R11 are attached to the same nitrogen they may be connected to form a 3-to 7-membered ring;
or any tautomer, enantiomer, diastereomer or mixture thereof, as well as a salt thereof with a pharmaceutically acceptable acid or base.

4. A pharmaceutical preparation according to claim 1 wherein the Dipeptidyl Peptidase IV is a compound of formula II 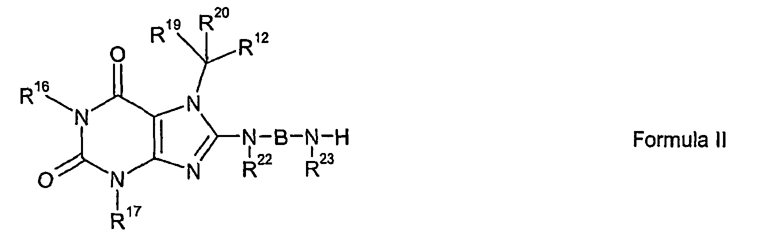 wherein
B is C2-C6 alkylene; C2-C10 alkenylene; C3-C7 cycloalkylene; C3-C7 cycloheteroalkylene; arylene; heteroarylene; C1-C2 alkylene-arylene; arylene-C1-C2 alkylene; C1-C2 alkylene-arylene-C1-C2 alkylene, wherein each alkylene, alkenylene, cycloalkylene, cycloheteroalkylene, arylene, or heteroarylene is optionally substituted with one or more R14 independently;
R12 is aryl optionally substituted with one or more R13 independently or heteroaryl optionally substituted with one or more R13 independently;
R13 is H; C1-C7 alkyl; C2-C7 alkenyl; C2-C7 alkynyl; C3-C7 cycloalkyl; C3-C7 cycloheteroalkyl; -NHCOR14; -NHSO2R14; -SR14; -SOR14; -SO2R14; -OCOR14; -CO2R15; -CON(R15)2; -CSN(R15)2; -NHCON(R15)2; -NHCSN(R15)2; -NHCONNH2; -S02N(R15)2; -OR15; cyano; nitro; halogen, wherein each alkyl, alkenyl, alkynyl, cycloalkyl and cycloheteroalkyl is optionally substituted with one or more R14 independently;
R14 is C1-C10 alkyl; C2-C10 alkenyl; C2-C10 alkynyl; C3-C7 cycloalkyl; aryl; heteroaryl; OR21; N(R21)2; SR21, wherein each alkyl, alkenyl, alkynyl, cycloalkyl, aryl and heteroaryl is optionally substituted with one or more R21 independently;
R15 is H; C1-C10 alkyl; C2-C10 alkenyl; C2-C10 alkynyl; C3-C7 cycloalkyl; C3-C7 cycloheteroalkyl; aryl; aryl-C1-C5 alkylene; heteroaryl; heteroaryl-C1-C5 alkylene, wherein each alkyl, alkenyl, alkynyl, cycloalkyl, cycloheteroalkyl, aryl, aryl-C1-C5 alkylene, heteroaryl, and heteroaryl-C1-C5 alkylene is optionally substituted with one or more R21 independently;
R16 is H; C1-C10 alkyl; C2-C10 alkenyl; C2-C10 alkynyl; C3-C7 cycloalkyl; C3-C7 cycloheteroalkyl; aryl; heteroaryl; -OR18; -[(CH2)o-O]p-C1-C5 alkyl, wherein o and p are 1-3 independently, and wherein each alkyl, alkenyl, alkynyl, cycloalkyl, cycloheteroalkyl, aryl, and heteroaryl is optionally substituted with one or more R18 independently;
R17 is H; C1-C10 alkyl; C2-C10 alkenyl; C2-C10 alkynyl; C3-C7 cycloalkyl; C3-C7 cycloheteroalkyl; aryl; heteroaryl; aryl-C1-C5 alkylene; heteroaryl-C1-C5 alkylene; C3-C7 cycloheteroalkyl-C1-C5 alkylene, wherein each alkyl, alkenyl, alkynyl, cycloalkyl, cycloheteroalkyl, C3-C7 cycloheteroalkyl-C1-C5 alkylene, aryl, aryl-C1-C5 alkylene, heteroaryl, aryl-C1-C5 alkylene, and heteroaryl-C1-C5 alkylene is optionally substituted with one or more R21 independently;
R18 is H; =O; C1-C10 alkyl; C2-C10 alkenyl; C2-C10 alkynyl; C3-C7 cycloalkyl; C3-C7 cycloheteroalkyl; aryl; heteroaryl, OR21; N(R21)2; SR21; cyano; hydroxy; halogen; -CF3; -CCl3; -OCF3; or -OCH3 wherein each alkyl, alkenyl, alkynyl, cycloalkyl, cycloheteroalkyl, aryl, and heteroaryl is optionally substituted with one or more R21 independently;
R19 is H; C1-C10 alkyl; C2-C10 alkenyl; C2-C10 alkynyl; C3-C7 cycloalkyl; C3-C7 cycloheteroalkyl; aryl; heteroaryl, OR21; N(R21)2; SR21, wherein each alkyl, alkenyl, alkynyl, cycloalkyl, cycloheteroalkyl, aryl, and heteroaryl is optionally substituted with one or more R21 independently;
R20 is H; C1-C10 alkyl optionally substituted with one or more R19 independently; or halogen;
R21 is H; -CF3; -CCl3; -OCF3; -OCH3; cyano; halogen; -OH, -COCH3; -CONH2; -CONHCH3; -CON(CH3)2; -N02; -SO2NH2; or -SO2N(CH3)2;
if two R15 or two R21 are attached to the same nitrogen they may be connected to form a 3-to 7-membered ring;
R22 is H; C1-C6 alkyl optionally substituted with one or more R14 independently;
R23 is H; C1-C6 alkyl optionally substituted with one or more R14 independently; or
If B is C3-C7 cycloalkylene or C3-C7 cycloheteroalkylene R23 may be a valence bond between the nitrogen to which R23 is attached and one of the atoms in the cycloalkylene or cycloheteroalkylene;
or any tautomer, enantiomer, diastereomer or mixture thereof, as well as a salt thereof with a pharmaceutically acceptable acid or base.

5. A pharmaceutical preparation according to claim 1 wherein the Dipeptidyl Peptidase IV is a compound of formula III 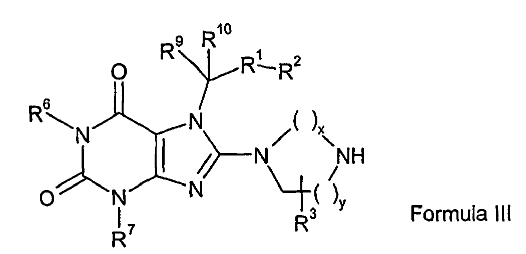 wherein
x and y are one or two independently
R1 is C=O; C=S; C1-C2 alkyl optionally substituted with one or more R4 independently; C2 alkenyl substituted with one or more R4 independently; C2 alkynyl; C3-C7 cycloalkyl optionally substituted with one or more R4 independently; C3-C7 cycloheteroalkyl optionally substituted with one or more R4 independently; aryl optionally substituted with one or more R4 independently; aryl C1-C3 alkyl optionally substituted with one or more R4 independently; heteroaryl optionally substituted with one or more R4 independently; heteroaryl C1-C3 alkyl optionally substituted with one or more R4 independently; perhalo C1-C10 alkyl; perhalo C1-C10 alkyloxy;
R2 is H; C1-C7 alkyl optionally substituted with one or more R4 independently; C2-C7 alkenyl optionally substituted with one or more R4 independently; C2-C7 alkynyl optionally substituted with one or more R4 independently; C3-C7 cycloalkyl optionally substituted with one or more R4 independently; C3-C7 cycloheteroalkyl optionally substituted with one or more R4 independently; aryl optionally substituted with one or more R4 independently; aryl C1-C3 alkyl optionally substituted with one or more R4 independently; heteroaryl C1-C3 alkyl optionally substituted with one or more R4 independently; heteroaryl optionally substituted with one or more R4 independently, -SH; -SR5; SOR5; SO2R5; -CHO; -CH(OR5)2; carboxy; -CO2R4; NHCONNH2; -NHCSNH2; -NHCONH2; -NHCOR4; -NHSO2R5; -O-CO-(C1-C5) alkyl optionally substituted with one or more R4 independently; cyano; nitro; halogen; hydroxy; perhalo C1-C7 alkyl; perhalo C1-C7 alkyloxy; -SO2NH2; -S02NH(R5); -SO2(R5)2; -CONH2; -CSNH2; -CON2H3; -CONH(R5); -CON(R5)2; C1-C10 alkyloxy optionally substituted with R4 independently; C2-C10 alkenyloxy optionally substituted with R4; C2-C10 alkynyloxy optionally substituted with R4 independently, aryloxy optionally substituted with R4 independently; heteroaryloxy optionally substituted with R4 independently;
R3 is H; C1-C10 alkyl optionally substituted with one or more R4 independently; C2-C10 alkenyl optionally substituted with one or more R4 independently; C2-C10 alkynyl optionally substituted with one or more R4 independently; C3-C7 cycloalkyl optionally substituted with one or more R4 independently; C3-C7 cycloheteroalkyl optionally substituted with one or more R4 independently; aryl optionally substituted with one or more R4 independently; aryl C1-C3 alkyl optionally substituted with one or more R4 independently; heteroaryl C1-C3 alkyl optionally substituted with one or more R4 independently; heteroaryl optionally substituted with one or more R4 independently; C1-C10 alkyl-NH(CH2)1-4NH-aryl optionally substituted with one or more R4 independently; C1-C10 alkyl-NH(CH2)1-4NH-heteroaryl optionally substituted with one or more R4 independently; C1-C10 alkyl-O(CH2)1-4NH-aryl optionally substituted with one or more R4 independently; C1-C10 alkyl-O(CH2)1-4NH-heteroaryl optionally substituted with one or more R4 independently; C1-C10 alkyl-O(CH2)1-4O-aryl optionally substituted with one or more R4 independently; C1-C10 alkyl-O(CH2)1-4O-heteroaryl optionally substituted with one or more R4 independently; C1-C10 alkyl-S(CH2)1-4NH-aryl optionally substituted with one or more R4 independently; C1-C10 alkyl-S(CH2)1-4NH-heteroaryl optionally substituted with one or more R4 independently; C1-C10 alkyl-S(CH2)1-4S-aryl optionally substituted with one or more R4 independently; C1-C10 alkyl-S(CH2)1-4S-heteroaryl optionally substituted with one or more R4 independently; C1-C10 alkyl-O-C1-C5alkyl optionally substituted with one or more R4; -NHCOR4; -NHSO2R5; -O-CO-(C1-C5) alkyl optionally substituted with one or more R4 independently; -SH; -SR5; -SOR5; -SO2R5; -CHO; -CH(OR5)2; carboxy; cyano; nitro; halogen; hydroxy; -SO2NH2; -SO2NH(R5); -SO2N(R5)2; -CONH2; -CONH(R5); -CON(R5)2; -CSNH2; -CONHNH2; -CO2R4; -NHCNHNH2; -NHCSNH2; -NHCONH2;
R4 is C1-C10 alkyl optionally substituted with one or more R8 independently; C2-C10 alkenyl optionally substituted with one or more R8 independently; C2-C10 alkynyl optionally substituted with one or more R8 independently; C3-C7 cycloalkyl optionally substituted with one or more R8 independently; C3-C7 cycloheteroalkyl optionally substituted with one or more R8 independently; aryl optionally substituted with one or more R8 independently; heteroaryl optionally substituted with one or more R8 independently; amino; amino substituted with one or more C1-C10 alkyl optionally substituted with one or more R8; amino substituted with one or two aryl optionally substituted with one or more R8 independently; heteroaryl optionally substituted with one or more R8 independently; =O; =S; -CO-R5; -COOR5; -O-CO-(C1-C5) alkyl optionally substituted with one or more R8 independently; NH(CH2)1-4NH-aryl; NH(CH2)1-4NH-heteroaryl; -NHCOR5; -SOR5; SO2R5; carboxy; cyano; N-hydroxyimino; nitro; halogen; hydroxy; perhalo C1-C10 alkyl; perhalo C1-C10 alkyloxy; -SH; -SR5; -S03H; -SO3R5; -SO2R5; -SO2NH2; -S02NH(R5); -SO2N(R5)2; -CONH2; -CONH(R5); -CON(R5)2; C1-C10 alkyloxy optionally substituted with one or more R8 independently; C2-C10 alkenyloxy optionally substituted with one or more R8 independently; C2-C10 alkynyloxy optionally substituted with one or more R8 independently; aryloxy optionally substituted with one or more R8 independently; heteroaryloxy optionally substituted with one or more R8 independently; and two R4 attached to the same carbon atom may form a spiroheterocyclic system, preferably hydantoin; thiohydantoin; oxazolidine-2,5-dione;
R5 is C1-C10 alkyl optionally substituted with one or more R8 independently; C2-C10 alkenyl optionally substituted with one or more R8 independently; C2-C10 alkynyl optionally substituted with one or more R8 independently; C3-C7 cycloalkyl optionally substituted with one or more R8 independently; C3-C7 cycloheteroalkyl optionally substituted with one or more R8 independently; aryl optionally substituted with one or more R8 independently; aryl C1-C5 alkyl optionally substituted with one or more R8 independently; heteroaryl optionally substituted with one or more R8 independently; heteroaryl C1-C5 alkyl optionally substituted with one or more R8 independently;
R6 is H; C1-C10 alkyl optionally substituted with one or more R4 independently; C2-C10 alkenyl optionally substituted with one or more R4 independently; C2-C10 alkynyl optionally substituted with one or more R4 independently; C3-C7 cycloalkyl optionally substituted with one or more R4 independently; C3-C7 cycloheteroalkyl optionally substituted with one or more R4 independently; aryl optionally substituted with one or more R4 independently; heteroaryl optionally substituted with one or more R4 independently;
R7 is H; C1-C10 alkyl optionally substituted with one or more R4 independently; C2-C10 alkenyl optionally substituted with one or more R4 independently; C2-C10 alkynyl optionally substituted with one or more R4 independently; C3-C7 cycloalkyl optionally substituted with one or more R4 independently; C3-C7 cycloheteroalkyl optionally substituted with one or more R4 independently; aryl optionally substituted with one or more R4 independently; heteroaryl optionally substituted with one or more R4 independently;
R8 is H, amidoxime; nitro, tetrazole; pentafluorophenyl; -CH2OH; -CHO; -C(OCH3)2; -COCH3; -CF3; -CCl3; -OCF3; -OCH3; -CN; -CO2H; -CO2CH3; -CONH2; -CSNH2; -CON2H3; -SO3H; -S02NH2; -S02NHCH3; -S02N(CH3)2; -SO2 (1-piperazinyl);-SO2 (4-methylpiperazin-1-yl); -S02 (pyrrolidin-1-yl); -SO2 (piperidin-1-yl); -S02 (morpholin-4-yl); N-hydroxyimino; -NH2; -NHCH3; -N(CH3)2; -NHCNHNH2; -NHCNHNHCH3; -NHCSNH2; -NHCSNHCH3; -NHCONH2; -NHCONHCH3; -NHCOCH3; -NHS02CH3; piperazinyl; morhpolin-4-yl; thiomorpholin-4-yl; pyrrolidin-1-yl; piperidin-1-yl; halogen; -OH; -SH; -SCH3; -aminoacetyl; -OP03H; -OPO20CH3; -PO3H2; -PO(OCH3)2; PO(OH)(OCH3);
R9 is H; halogen; C1-C10 alkyl optionally substituted with one or more R4 independently
R10 is H; halogen;
or, R9 and R10 may be connected to form a cyclopropyl ring;
or any tautomer, enantiomer, diastereomer or mixture thereof, as well as a salt thereof with a pharmaceutically acceptable acid or base;
with the exception of the following compounds:
1,3-dimethyl-7-(2-oxo-propyl) -8-piperazin-1-yl-3,7-dihydro-purine-2,6-dione,
1,3,1',3',7'-pentamethyl-8-piperazin-1-yl-3,7,3',7'-tetrahydro-7,8'-methanediyl-bis-purine-2,6-dione,
3,4,5-trimethoxy-benzoic acid 2-(1,3-dimethyl-2,6-dioxo-8-piperazin-1-yl-1,2,3,6-tetrahydro-purin-7-yl) -ethyl ester,
7-[2-Hydroxy-3-(4-methoxy-phenoxy) -propyl]-3-methyl-8-piperazin-1-yl-3,7-dihydro-purine-2,6-dione,
7-[2-hydroxy-2-(4-nitro-phenyl) -ethyl]-3-methyl-8-piperazin-1-yl-3,7,8,9-tetrahydro-purine-2,6-dione,
7-Benzyl-3-methyl-8-piperazin-1-yl-3,7-dihydro-purine-2,6-dione,
7-(4-Chloro-benzyl) -3-methyl-8-piperazin-1-yl-3,7-dihydro-purine-2,6-dione,
7-(2-Chloro-benzyl) -3-methyl-8-piperazin-1-yl-3,7-dihydro-purine-2,6-dione,
7-Ethyl-3-methyl-8-piperazin-1-yl-3,7-dihydro-purine-2,6-dione,
3-Methyl-8-piperazin-1-yl-1,7-dipropyl-3,7-dihydro-purine-2,6-dione,
3-Methyl-7-(3-methyl-butyl) -8-piperazin-1-yl-3,7-dihydro-purine-2,6-dione,
7-Butyl-3-methyl-8-piperazin-l-yl-3,7-dihydro-purine-2,6-dione,
3-Methyl-7-(3-phenyl-propyl) -8-piperazin-1-yl-3,7-dihydro-purine-2,6-dione,
7-But-2-enyl-3-methyl-8-piperazin-1-yl-3,7-dihydro-purine-2,6-dione,
7-(3-Chloro-but-2-enyl) -3-methyl-8-piperazin-1-yl-3,7-dihydro-purine-2,6-dione,
7-Heptyl-3-methyl-8-piperazin-1-yl-3,7-dihydro-purine-2,6-dione,
3-Methyl-7-(1-phenyl-ethyl) -8-piperazin-1-yl-3,7-dihydro-purine-2,6-dione,
3-Methyl-7-(3-methyl-benzyl) -8-piperazin-1-yl-3,7-dihydro-purine-2,6-dione,
3-Methyl-7-propyl-8-piperazin-1-yl-3,7-dihydro-purine-2,6-dione, and 3-Methyl-7-pentyl-8-piperazin-1-yl-3,7-dihydro-purine-2,6-dione

6. A pharmaceutical preparation according to any one of the claims 1 to 5 wherein the NEP inhibitor is candoxatril

7. A pharmaceutical preparation according to any one of the claims 1 to 5 wherein the NEP inhibitor is a dual NEP/ACE inhibitor

8. A pharmaceutical preparation according to any one of the claims 1 to 6 which furthermore comprises a further antidiabetic.

9. A pharmaceutical preparation according to any one of the claims 1 to 8 which furthermore comprises an Angiotensin Converting Enzyme (ACE) inhibitor.

## Patentansprüche

1. Pharmazeutisches Präparat, umfassend eine Kombination aus einem Dipeptidylpeptidase-IV-Inhibitor und einem neutralen Endopeptidaseinhibitor oder ein pharmazeutisch verträgliches Salz von einem der beiden oder beiden davon.

2. Pharmazeutisches Präparat nach Anspruch 1, wobei die Dipeptidylpeptidase IV ein N-substituiertes Adamantylaminoacetyl-2-cyanopyrrolidin oder ein N-(substituiertes Glycyl)-4-cyanopyrrolidin ist.

3. Pharmazeutisches Präparat nach Anspruch 1, wobei die Dipeptidylpeptidase IV eine Verbindung der Formel I 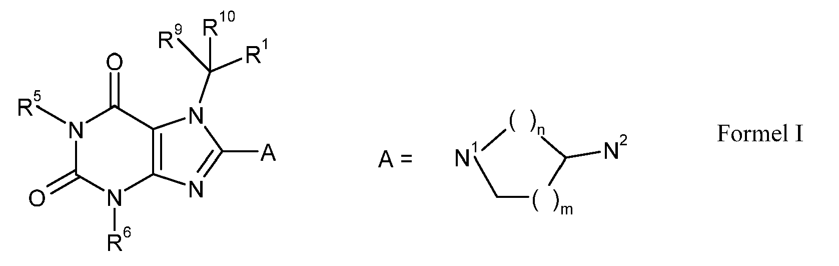 wobei A entweder an N1 oder an N2 am Purinsystem gebunden sein kann und
jedes n und m unabhängig für eins oder zwei steht;
R1 für ein Aryl, wahlweise unabhängig substituiert mit einem oder mehreren R2 oder Heteroaryl, wahlweise unabhängig substituiert mit einem oder mehreren R2, steht;
R2 für H; C1-C7-Alkyl; C2-C7-Alkenyl; C2-C7-Alkinyl; C3-C7-Cycloalkyl; C3-C7-Cycloheteroalkyl; -NHCOR3; -NHSO2R3; -SR3; -SOR3; -SO2R3; -OCOR3; -CO2R4; -CON(R4)2; -CSN(R4)2; -NHCON(R4)2; -NHCSN(R4)2; -NHCONNH2; -SO2N(R4)2; -OR4; Cyano; Nitro; Halogen steht, wobei jedes Alkyl, Alkenyl, Alkinyl, Cycloalkyl und Cycloheteroalkyl wahlweise unabhängig substituiert ist mit einem oder mehreren R3;
R3 für C1-C10-Alkyl; C2-C10-Alkenyl; C2-C10-Alkinyl; C3-C7-Cycloalkyl; Aryl; Heteroaryl; OR11; N(R11)2; SR11 steht, wobei jedes Alkyl, Alkenyl, Alkinyl, Cycloalkyl, Aryl und Heteroaryl unabhängig substituiert ist mit einem oder mehreren R11;
R4 für H; C1-C10-Alkyl; C2-C10-Alkenyl; C2-C10-Alkinyl; C3-C7-Cycloalkyl; C3-C7-Cycloheteroalkyl; Aryl; Aryl-C1-C5-alkyl; Heteroaryl; Heteroaryl-C1-C5-alkyl steht, wobei jedes Alkyl, Alkenyl, Alkinyl, Cycloalkyl, Cycloheteroalkyl, Aryl, Aryl-C1-C5-alkyl, Heteroaryl und Heteroaryl-C1-C5-alkyl unabhängig substituiert ist mit einem oder mehreren R11;
R5 für H; C1-C10-Alkyl; C2-C10-Alkenyl; C2-C10-Alkinyl; C3-C7-Cycloalkyl; C3-C7-Cycloalkyl-C1-C5-Alkyl; C3-C7-Cycloheteroalkyl; C3-C7-Cycloheteroalkyl-C1-C5-alkyl; Aryl; Heteroaryl; Aryl-C1-C5-alkyl; Heteroaryl-C1-C5-alkyl; -OR7; -[(CH2)o-O]p-Alkyl steht, wobei o und p unabhängig 1-3 sind, und wobei jedes Alkyl, Alkenyl, Alkinyl, Cycloalkyl, Cycloalkyl-C1-C5-alkyl, Cycloheteroalkyl, C3-C7-Cycloheteroalkyl-C1-C5-alkyl, Aryl, Aryl-C1-C5-alkyl, Heteroaryl, Aryl-C1-C5-alkyl und Heteroaryl-C1-C5-alkyl wahlweise unabhängig substituiert ist mit einem oder mehreren R7;
R6 für C1-C10-Alkyl; C2-C10-Alkenyl; C2-C10-Alkinyl; C3-C7-Cycloalkyl; C3-C7-Cycloheteroalkyl; Aryl; Heteroaryl; Aryl-C1-C5-alkyl; Heteroaryl-C1-C5-alkyl; C3-C7-Cycloheteroalkyl-C1-C5-alkyl steht, wobei jedes Alkyl, Alkenyl, Alkinyl, Cycloalkyl, Cycloheteroalkyl, C3-C7-Cycloheteroalkyl-C1-C5-alkyl, Aryl, Aryl-C1-C5-alkyl, Heteroaryl und Heteroaryl-C1-C5-alkyl wahlweise unabhängig substituiert ist mit einem oder mehreren R11;
R7 für H; -O; C1-C10-Alkyl; C2-C10-Alkenyl; C2-C10-Alkinyl, C3-C7-Cycloalkyl; C3-C7-Cycloheteroalkyl; Aryl; Heteroaryl, OR11; N(R11)2; SR11 steht, wobei jedes Alkyl, Alkenyl, Alkinyl, Cycloalkyl, Cycloheteroalkyl, Aryl, und Heteroaryl wahlweise unabhängig substituiert ist mit einem oder mehreren R11;
R8 für C1-C10-Alkyl; C2-C10-Alkenyl; C2-C10-Alkinyl; C3-C7-Cycloalkyl; C3-C7-Cycloheteroalkyl; Aryl; Heteroaryl, OR11; N(R11)2; SR11 steht, wobei jedes Alkyl, Alkenyl, Alkinyl, Cycloalkyl, Cycloheteroalkyl, Aryl und Heteroaryl wahlweise unabhängig substituiert ist mit einem oder mehreren R11;
R9 und R10 unabhängig für H, C1-C10-Alkyl, wahlweise unabhängig substituiert mit einem oder mehreren R8, Halogen stehen;
R11 für H; -CF3; -CC13; -OCF3; -OMe; Cyano; Halogen; -OH, COMe; -CONH2; CONHMe; CONMe2; -NO2 steht;
wenn R9 und R10 für C1-C10-Alkyl stehen, sie unter Bildung eines Cyclopropylrings verbunden sein können;
wenn R4 und R11 an denselben Stickstoff gebunden sind, sie unter Bildung eines 3- bis 7-gliedrigen Rings verbunden sein können;
oder ein beliebiges Tautomer, Enantiomer, Diastereomer oder Gemisch davon, sowie ein Salz davon mit einer pharmazeutisch verträglichen Säure oder Base ist.

4. Pharmazeutisches Präparat nach Anspruch 1, wobei die Dipeptidylpeptidase IV eine Verbindung der Formel II 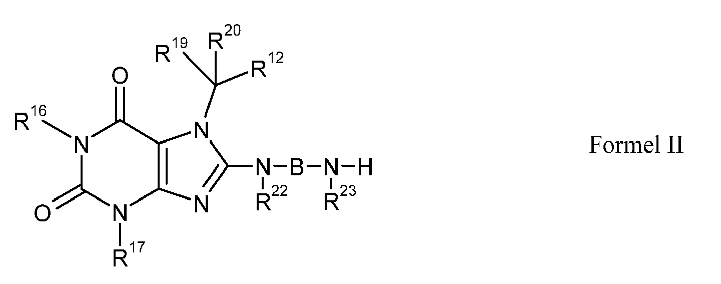 wobei
B für C2-C6-Alkylen; C2-C10-Alkenylen; C3-C7-Cycloalkylen; C3-C7-Cycloheteroalkylen; Arylen; Heteroarylen; C1-C2-Alkylenarylen; Arylen-C1-C2-alkylen; C1-C2-Alkylenarylen-C1-C2-alkylen steht, wobei jedes Alkylen, Alkenylen, Cycloalkylen, Cycloheteroalkylen, Arylen oder Heteroarylen wahlweise unabhängig substituiert ist mit einem oder mehreren R14;
R12 für Aryl, wahlweise unabhängig substituiert mit einem oder mehreren R13, oder Heteroaryl, wahlweise unabhängig substituiert mit einem oder mehreren R13, steht;
R13 für H; C1-C7-Alkyl; C2-C7-Alkenyl; C2-C7-Alkinyl; C3-C7-Cycloalkyl; C3-C7-Cycloheteroalkyl; -NHCOR14; -NHSO2R14; -SR14; -SOR14; -SO2R14; -OCOR14; -CO2R15; -CON(R15)2; -CSN(R15)2; -NHCON(R15)2; -NHCSN(R15)2; -NHCONNH2; -SO2N(R15)2; -OR15; Cyano; Nitro; Halogen steht, wobei jedes Alkyl, Alkenyl, Alkinyl, Cycloalkyl und Cycloheteroalkyl wahlweise unabhängig substituiert ist mit einem oder mehreren R14;
R14 für C1-C10-Alkyl; C2-C10-Alkenyl; C2-C10-Alkinyl; C3-C7-Cycloalkyl; Aryl; Heteroaryl; OR21; N(R21)2; SR21 steht, wobei jedes Alkyl, Alkenyl, Alkinyl, Cycloalkyl, Aryl und Heteroaryl wahlweise unabhängig substituiert ist mit einem oder mehreren R21;
R15 für H; C1-C10-Alkyl; C2-C10-Alkenyl; C2-C10-Alkinyl; C3-C7-Cycloalkyl; C3-C7-Cycloheteroalkyl; Aryl; Aryl-C1-C5-alkylen; Heteroaryl; Heteroaryl-C1-C5-alkylen steht, wobei jedes Alkyl, Alkenyl, Alkinyl, Cycloalkyl, Cycloheteroalkyl, Aryl, Aryl-C1-C5-alkylen, Heteroaryl und Heteroaryl-C1-C5-alkylen wahlweise unabhängig substituiert ist mit einem oder mehreren R21;
R16 für H; C1-C10-Alkyl; C2-C10-Alkenyl; C2-C10-Alkinyl; C3-C7-Cycloalkyl; C3-C7-Cycloheteroalkyl; Aryl; Heteroaryl; -OR18; -[(CH2)o-O]p-C1-C5-Alkyl steht, wobei o und p unabhängig für 1-3 stehen, und wobei jedes Alkyl, Alkenyl, Alkinyl, Cycloalkyl, Cycloheteroalkyl, Aryl und Heteroaryl wahlweise unabhängig substituiert ist mit einem oder mehreren R18;
R17 für H; C1-C10-Alkyl; C2-C10-Alkenyl; C2-C10-Alkinyl; C3-C7-Cycloalkyl; C3-C7-Cycloheteroalkyl; Aryl; Heteroaryl; Aryl-C1-C5-alkylen; Heteroaryl-C1-C5-Alkylen; C3-C7-Cycloheteroalkyl-C1-C5-alkylen steht, wobei jedes Alkyl, Alkenyl, Alkinyl, Cycloalkyl, Cycloheteroalkyl. C3-C7-Cycloheteroalkyl-C1-C5-alkylen, Aryl, Aryl-C1-C5-alkylen, Heteroaryl, Aryl-C1-C5-alkylen und Heteroaryl-C1-C5-alkylen wahlweise unabhängig substituiert ist mit einem oder mehreren R21;
R18 für H; =O; C1-C14-Alkyl; C2-C10-Alkenyl; C2-C10-Alkinyl; C3-C7-Cycloalkyl; C3-C7-Cycloheteroalkyl; Aryl; Heteroaryl, OR21; N(R21)2; SR21; Cyano; Hydroxy; Halogen; -CF3; - CC13; -OCF3; oder -OCH3 steht, wobei jedes Alkyl, Alkenyl, Alkinyl, Cycloalkyl, Cycloheteroalkyl, Aryl und Heteroaryl wahlweise unabhängig substituiert ist mit einem oder mehreren R21;
R19 für H; C1-C10-Alkyl; C2-C10-Alkenyl; C2-C10-Alkinyl; C3-C7-Cycloalkyl; C3-C7-Cycloheteroalkyl; Aryl; Heteroaryl, OR21; N(R21)2; SR21 steht, wobei jedes Alkyl, Alkenyl, Alkinyl, Cycloalkyl, Cycloheteroalkyl, Aryl und Heteroaryl wahlweise unabhängig substituiert ist mit einem oder mehreren R21;
R20 für H; C1-C10-Alkyl, wahlweise unabhängig substituiert mit einem oder mehreren R19; oder Halogen steht;
R21 für H; -CF3; -CCl3; -OCF3; -OCH3; Cyano; Halogen; -OH, -COCH3; -CONH2; -CONHCH3; -CON(CH3)2; -NO2; -SO2NH2; oder -SO2N(CH3)2 steht;
wenn zwei R15 oder zwei R21 an denselben Stickstoff gebunden sind, sie unter Bildung eines 3- bis 7-gliedrigen Rings verbunden sein können;
R22 für H; C1-C6-Alkyl, wahlweise unabhängig substituiert mit einem oder mehreren R14, steht;
R23 für H; C1-C6-Alkyl, wahlweise unabhängig substituiert mit einem oder mehreren R14 steht; oder
wenn B für C3-C7-Cycloalkylen oder C3-C7-Cycloheteroalkylen steht, R23 eine Valenzbindung zwischen dem Stickstoff, an welchen R23 gebunden ist und einem der Atome im Cycloalkylen oder Cycloheteroalkylen sein kann;
oder ein beliebiges Tautomer, Enantiomer, Diastereomer oder Gemisch davon, sowie ein Salz davon mit einer pharmazeutisch verträglichen Säure oder Base ist.

5. Pharmazeutisches Präparat nach Anspruch 1, wobei die Dipeptidylpeptidase IV eine Verbindung der Formel III 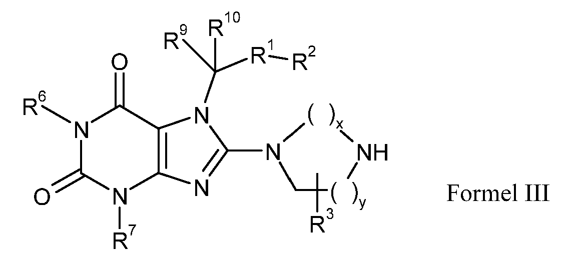 wobei
x und y unabhängig für eins oder zwei stehen;
R1 für C=O; C=S; C1-C2-Alkyl, wahlweise unabhängig substituiert mit einem oder mehreren R4; C2-Alkenyl, unabhängig substituiert mit einem oder mehreren R4; C2-Alkinyl; C3-C7-Cycloalkyl, wahlweise unabhängig substituiert mit einem oder mehreren R4; C3-C7-Cycloheteroalkyl, wahlweise unabhängig substituiert mit einem oder mehreren R4; Aryl, wahlweise unabhängig substituiert mit einem oder mehreren R4; Aryl-C1-C3alkyl, wahlweise unabhängig substituiert mit einem oder mehreren R4; Heteroaryl, wahlweise unabhängig substituiert mit einem oder mehreren R4; Heteroaryl C1-C3-alkyl, wahlweise unabhängig substituiert mit einem oder mehreren R4; Perhalogen-C1-C10-alkyl; Perhalogen-C1-C10-alkyloxy, steht;
R2 für H; C1-C7-Alkyl, wahlweise unabhängig substituiert mit einem oder mehreren R4; C2-C7-Alkenyl, wahlweise unabhängig substituiert mit einem oder mehreren R4; C2-C7-Alkinyl, wahlweise unabhängig substituiert mit einem oder mehreren C3-C7 Cycloalkyl, wahlweise unabhängig substituiert mit einem oder mehreren R4; C3-C7-Cycloheteroalkyl, wahlweise unabhängig substituiert mit einem oder mehreren R4; Aryl, wahlweise unabhängig substituiert mit einem oder mehreren R4; Aryl-C1-C3-alkyl, wahlweise unabhängig substituiert mit einem oder mehreren R4; Heteroaryl C1-C3-alkyl, wahlweise unabhängig substituiert mit einem oder mehreren R4; Heteroaryl, wahlweise unabhängig substituiert mit einem oder mehreren R4, -SH; -SR5; SOR5; SO2R5; -CHO; -CH(OR5)2; Carboxy; -CO2R4; NHCONNH2; -NHCSNH2; -NHCONH2; -NHCOR4; -NHSO2R5; -O-CO-(C1-C5)-Alkyl, wahlweise unabhängig substituiert mit einem oder mehreren R4; Cyano; Nitro; Halogen; Hydroxy; Perhalogen-C1-C7-alkyl; Perhalogen-C1-C7-alkyloxy; -SO2NH2; -SO2NH(R5); -SO2(R5)2; -CONH2; -CSNH2; -CON2H3; -CONH(R5); -CON(R5)2; C1-C10-Alkyloxy, wahlweise unabhängig substituiert mit R4; C2-C10-Alkenyloxy, wahlweise substituiert mit R4; C2-C10-Alkinyloxy, wahlweise unabhängig substituiert mit R4, Aryloxy, wahlweise unabhängig substituiert mit R4; Heteroaryloxy, wahlweise unabhängig substituiert mit R4, steht;
R3 für H; C1-C10-Alkyl, wahlweise unabhängig substituiert mit einem oder mehreren R4; C2-C 10-Alkenyl wahlweise unabhängig substituiert mit einem oder mehreren R4; C2-C10-Alkinyl, wahlweise unabhängig substituiert mit einem oder mehreren R4; C3-C7-Cycloalkyl, wahlweise unabhängig substituiert mit einem oder mehreren R4; C3-C7-Cycloheteroalkyl, wahlweise unabhängig substituiert mit einem oder mehreren R4; Aryl, wahlweise unabhängig substituiert mit einem oder mehreren R4; Aryl-C1-C3-alkyl, wahlweise unabhängig substituiert mit einem oder mehreren R4; Heteroaryl- C1-C3-alkyl, wahlweise unabhängig substituiert mit einem oder mehreren R4; Heteroaryl, wahlweise unabhängig substituiert mit einem oder mehreren R4; C1-C10-Alkyl-NH(CH2)1-4NH-aryl, wahlweise unabhängig substituiert mit einem oder mehreren R4; C1-C10-Alkyl-NH(CH2)1-4NH-heteroaryl, wahlweise unabhängig substituiert mit einem oder mehreren R4; C1-C10-Alkyl-O(CH2)1-4NH-aryl, wahlweise unabhängig substituiert mit einem oder mehreren R4; C1-C10-Alkyl-O(CH2)1-4NH-heteroaryl, wahlweise unabhängig substituiert mit einem oder mehreren R4; C1-C10-Alkyl-O(CH2)1-40-aryl, wahlweise unabhängig substituiert mit einem oder mehreren R4; C1-C10-Alkyl-O(CH2)1-40- wahlweise unabhängig substituiert mit einem oder mehreren R4; C1-C10-Alkyl-S(CH2)1-4NH-aryl, wahlweise unabhängig substituiert mit einem oder mehreren R4; C1-C10-Alkyl-S(CH2)1-4NH-heteroaryl, wahlweise unabhängig substituiert mit einem oder mehreren R4; C1-C10-Alkyl-S(CH2)1-45-aryl, wahlweise unabhängig substituiert mit einem oder mehreren R4; C1-C10-Alkyl-S(CH2)1-45-heteroaryl, wahlweise unabhängig substituiert mit einem oder mehreren R4; C1-C10-Alkyl-O-C1-C5-alkyl, wahlweise unabhängig substituiert mit einem oder mehreren R4; -NHCOR4; -NHSO2R5; -O-CO-(C1-C5)-Alkyl, wahlweise unabhängig substituiert mit einem oder mehreren R4; -SH; -SR5; -SOR5; -SO2R5;
-CHO; -CH(OR5)2; Carboxy; Cyano; Nitro; Halogen; Hydroxy; -SO2NH2; -SO2NH(R5); -SO2N(R5)2; -CONH2; -CONH(R5); -CON(R5)2; -CSNH2; -CONHNH2; -CO2R4; -NHCNHNH2; -NHCSNH2; -NHCONH2, steht;
R4 für C1-C10-Alkyl, wahlweise unabhängig substituiert mit einem oder mehreren R8; C2-C10 Alkenyl, wahlweise unabhängig substituiert mit einem oder mehreren R8; C2-C10-Alkinyl, , wahlweise unabhängig substituiert mit einem oder mehreren R8; C3-C7-Cycloalkyl, wahlweise unabhängig substituiert mit einem oder mehreren R8; C3-C7-Cycloheteroalkyl, wahlweise unabhängig substituiert mit einem oder mehreren R8; Aryl, wahlweise unabhängig substituiert mit einem oder mehreren R8; Heteroaryl, wahlweise unabhängig substituiert mit einem oder mehreren R8; Amino; Amino, substituiert mit einem oder mehreren C1-C10-Alkyl, wahlweise substituiert mit einem oder mehreren R8; Amino, substituiert mit einem oder zwei Aryl, wahlweise unabhängig substituiert mit einem oder mehreren R8; Heteroaryl, wahlweise unabhängig substituiert mit einem oder mehreren R8; =O; =S; -CO-R5; -COOR5; -O-CO-(C1-C5 )-Alkyl, wahlweise unabhängig substituiert mit einem oder mehreren R8; NH(CH2)1-4NH-aryl; NH(CH2)1-4NH-heteroaryl; -NHCOR5; -SORS; SO2R5; Carboxy; Cyano; N-Hydroxyimino; Nitro; Halogen; Hydroxy; Perhalogen-C1-C10-Alkyl; Perhalogen-C1-C10-Alkyloxy; -SH; -SR5; -SO3H; -SO3R5; -SO2R5; -SO2NH2; -SO2NH(R5); -SO2N(R5)2; -CONH2; -CONH(R5); -CON(R5)2; C1-C10-Alkyloxy, wahlweise unabhängig substituiert mit einem oder mehreren R8; C2-C10-Alkenyloxy, wahlweise unabhängig substituiert mit einem oder mehreren R8; C2-C10-Alkinyloxy, wahlweise unabhängig substituiert mit einem oder mehreren R8; Aryloxy, wahlweise unabhängig substituiert mit einem oder mehreren R8; Heteroaryloxy, wahlweise unabhängig substituiert mit einem oder mehreren R8, steht; und zwei R4, die an demselben Kohlenstoffatom gebunden sind, ein spiroheterocyclisches System, vorzugsweise Hydandtoin, Thiohydantoin, Oxazolidin-2,5-dion bilden können;
R5 für C1-C10-Alkyl, wahlweise unabhängig substituiert mit einem oder mehreren R8; C2-C10-Alkenyl, wahlweise unabhängig substituiert mit einem oder mehreren R8; C2-C10-Alkinyl, wahlweise unabhängig substituiert mit einem oder mehreren R8; C3-C7-Cycloalkyl, wahlweise unabhängig substituiert mit einem oder mehreren R8; C3-C7-Cycloheteroalkyl, wahlweise unabhängig substituiert mit einem oder mehreren R8; Aryl, wahlweise unabhängig substituiert mit einem oder mehreren R8; Aryl-C1-C5alkyl, wahlweise unabhängig substituiert mit einem oder mehreren R8; Heteroaryl, wahlweise unabhängig substituiert mit einem oder mehreren R8; Heteroaryl-C1-C5-alkyl, wahlweise unabhängig substituiert mit einem oder mehreren R8, steht;
R6 für H; C1-C10-Alkyl, wahlweise unabhängig substituiert mit einem oder mehreren R4; C2-C10 Alkenyl, wahlweise unabhängig substituiert mit einem oder mehreren R4; C2-C10-Alkinyl, wahlweise unabhängig substituiert mit einem oder mehreren R4; C3-C7-Cycloalkyl, wahlweise unabhängig substituiert mit einem oder mehreren R4; C3-C7-Cycloheteroalkyl, wahlweise unabhängig substituiert mit einem oder mehreren R4; Aryl, wahlweise unabhängig substituiert mit einem oder mehreren R4; Heteroaryl, wahlweise unabhängig substituiert mit einem oder mehreren R4, steht;
R7 für H; C1-C10-Alkyl, wahlweise unabhängig substituiert mit einem oder mehreren R4; C2-C10-Alkenyl, wahlweise unabhängig substituiert mit einem oder mehreren R4; C2-C10-Alkinyl, wahlweise unabhängig substituiert mit einem oder mehreren R4; C3-C7-Cycloalkyl, wahlweise unabhängig substituiert mit einem oder mehreren R4; C3-C7-Cycloheteroalkyl, wahlweise unabhängig substituiert mit einem oder mehreren R4; Aryl, wahlweise unabhängig substituiert mit einem oder mehreren R4; Heteroaryl, , wahlweise unabhängig substituiert mit einem oder mehreren R4, steht;
R8 für H, Amidoxim; Nitro, Tetrazol; Pentafluorphenyl; -CH2OH; -CHO; -C(OCH3)2; -COCH3; -CF3; -CM; -OCF3; -OCH3; -CH; -CO2H; -CO2CH3; -CONH2; -CSNH2; -CON2H3; -SO3H; -SO2NH2; -SO2NHCH3; -SO2N(CH3)2; -SO2 (1-Piperazinyl); -SO2 (4-Methyipiperazin-1-yl); -SO2 (Pyrrolidin-1-yl); -SO2 (Piperidin-1-yl); -SO2 (Morpholin-4-yl); N-Hydroxyimino; -NH2; -NHCH3; -N(CH3)2; -NHCNHNH2; -NHCNHNHCH3; -NHCSNH2; -NHCSNHCH3; -NHCONH2; -NHCONHCH3; -NHCOCH3; -NHSO2CH3; Piperazinyl; Morhpolin-4-yl; Thlomorpholin-4-yl; Pyrrolidin-1-yl; Piperidin-1-yl; Halogen; -OH; -SH; -SCH3; -Aminoacetyl; -OPO3H; -OPO2OCH3; -PO3H2; -PO(OCH3)2; PO(OH)(OCH3) steht;
R9 für H; Halogen; C1-C10-Alkyl, wahlweise unabhängig substituiert mit einem oder mehreren R4, steht
R10 für H; Halogen steht;
oder R9 und R10 unter Bildung eines Cyclopropylrings verbunden sein können;
oder ein beliebiges Tautomer, Enantiomer, Diastereomer oder Gemisch davon, sowie ein Salz davon mit einer pharmazeutisch verträglichen Säure oder Base ist:
mit Ausnahme der folgenden Verbindungen:
1,3-Dimethyl-7-(2-oxopropyl)-8-piperazin-1-yl-3,7-dihydropurin-2,6-dion,
1,3,1',3',7'-Pentamethyl-8-piperazin-1-yl-3,7,3',7'-tetrahydro-7,8'-methandiylbispurin-2,6-dion,
3,4,5-Trimethoxybenzoesäure-2-(1,3-dimethyl-2,β-dioxo-8-piperazin-1-yl-12,3,6-tetrahydropurin-7-yl) -ethylester,
7-[2-Hydroxy-3-(4-methoxyphenoxy)propyl]-3-methyl-8-piperazin-1-yl-3,7-dihydro-purin-2,6-dion,
7-[2-Hydroxy-2-(4-nitrophenyl)-ethyl]-3-methyl-8-piperazin-1-yl-3,7,8,9-tetrahydro-purin-2,6-dion,
7-Benzyl-3-methyl-8-piperazin-1-yl-3,7-dihydropurin-2,6-dion,
7-(4-Chlorbenzyl)-3-methyl-8-piperazin-1-yl-3,7-dihydropurin-2,6-dion,
7-(2-Chlorbenzyl)-3-methyl-8-piperazin-1-yl-3,7-dihydropurin-2,6-dion,
7-Ethyl-3-methyl-8-piperazin-1-yl-3,7-dihydropurine-2,6-dion,
3-Methyl-8-piperazin-1-yl-1,7-dipropyl-3,7-dihydropurin-2,6-dion,
3-Methyl-7-(3-methylbutyl)-8-piperazin-1-yl-3,7-dihydropurin-2,6-dion,
7-Butyl-3-methyl-8-piperazin-1-yl-3,7-dihydropurin-2,6-dion,
3-Methyl-7-(3-phenylpropyl)-8-piperazin-1-yl-3,7-dihydropurin-2,6-dion,
7-But-2-enyl-3-methyl-8-piperazin-1-yl-3,7-dihydropurin-2,6-dion,
7-(3-Chlorbut-2-enyl)-3-methyl-8-piperazin-1-yl-3,7-dihydropurin-2,6-dion,
7-Heptyl-3-methyl-8-piperazin-1-yl-3,7-dihydropurin-2,6-dion,
3-Methyl-7-(1-phenylethyl)-8-piperazin-1-yl-3,7-dihydropurin-2,6-dion,
3-Methyl-7-(3-methylbenzyl)-8-piperazin-1-yl-3,7-dihydropurin-2,6-dion,
3-Methyl-7-propyl-8-piperazin-1-yl-3,7-dihydropurin-2,6-dion und
3-Methyl-7-pentyl-8-piperazin-1-yl-3,7-dihydropurin-2,6-dion.

6. Pharmazeutisches Präparat nach einem der Ansprüche 1 bis 5, wobei der NEP-Inhibitor Cadoxatril ist.

7. Pharmazeutisches Präparat nach einem der Ansprüche 1 bis 5, wobei der NEP-Inhibitor ein zweifacher NEP/ACE-Inhibitor ist.

8. Pharmazeutisches Präparat nach einem der Ansprüche 1 bis 6, das weiterhin ein weiteres Antidiabetikum umfasst.

9. Pharmazeutisches Präparat nach einem der Ansprüche 1 bis 8, das weiterhin einen Angiotensin-Converting-Enzym(ACE; Angiotensinumwandlungsenzym)-Inhibitor umfasst.

## Revendications

1. Préparation pharmaceutique comprenant une combinaison d'un inhibiteur de la dipepdityl peptidase IV et d'un inhibiteur de l'endopeptidase neutre, ou sel acceptable d'un point de vue pharmaceutique de l'un ou de l'autre, ou des deux.

2. Préparation pharmaceutique selon la revendication 1, dans laquelle la dipeptidyl peptidase IV est une adamantyl-amino-acétyl-2-cyano-pyrrolidine N-substituée ou une N-(glycyle substituée)-4-cyano-pyrrolidine.

3. Préparation pharmaceutique selon la revendication 1, dans laquelle la dipeptidyl peptidase IV est un composé de formule I 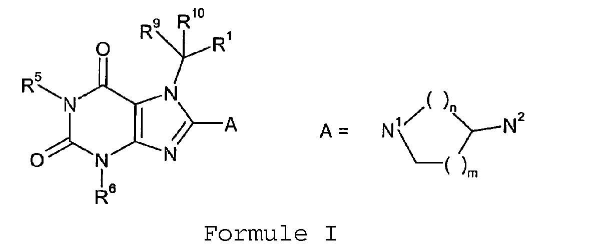 dans laquelle A peut être lié en N1 ou N2 au système purine, et chacun des indices n et m vaut, indépendamment de l'autre, un ou deux ;
R1 est un groupe aryle éventuellement substitué d'une manière indépendante par un ou plusieurs radicaux R2, ou hétéroaryle éventuellement substitué d'une manière indépendante par un ou plusieurs radicaux R2 ;
R2 est H ; un groupe alkyle en C1-C7, alcényle en C2-C7, alcynyle en C2-C7, cycloalkyle en C3-C7 ; cyclohétéroaryle en C3-C7 ; -NHCOR3 ; -NHSO2R3 ; -SR3 ; -SOR3 ; -SO2R3 ; -OCOR3 ; -CO2R4 ; -CON(R4)2 ; -CSN(R4)2 ; -NHCON(R4)2 ; -NHCSN(R4)2 ; -NHCONNH2 ; -S02N(R4)2 ; -OR4 ; cyano ; nitro ; halogéno, où chaque groupe alkyle, alcényle, alcynyle, cycloalkyle et cyclohétéroalkyle est éventuellement substitué d'une manière indépendante par un ou plusieurs radicaux R3 ;
R3 est un groupe alkyle en C1-C10 ; alcényle en C2-C10 ; alcynyle en C2-C10 ; cycloalkyle en C3-C7 ; aryle ; hétéroaryle ; OR11 ; N(R11)2 ; SR11, où chaque groupe alkyle, alcényle, alcynyle, cycloalkyle, aryle et hétéroaryle est substitué d'une manière indépendante par un ou plusieurs radicaux R11 ;
R4 est H ; un groupe alkyle en C1-C10 ; alcényle en C2-C10 ; alcynyle en C2-C10 ; cycloalkyle en C3-C7 ; cyclohétéroaryle en C3-C7 ; aryle ; aryl(alkyle en C1-C5) ; hétéroaryle ; hétéroaryl(alkyle en C1-C5), où chaque groupe alkyle, alcényle, alcynyle, cycloalkyle, cyclohétéroalkyle, aryl, aryl(alkyle en C1-C5), hétéroaryle et hétéroaryl(alkyle en C1-C5) est substitué d'une manière indépendante par un ou plusieurs radicaux R11 ;
R5 est H ; un groupe alkyle en C1-C10 ; alcényle en C2-C10 ; alcynyle en C2-C10 ; cycloalkyle en C3-C7 ; (cycloalkyle en C3-C7)(alkyle en C1-C5) ; cyclohétéro-alkyle en C3-C7 ; (cyclohétéroalkyle en C3-C7) (alkyle en C1-C5) ; aryle ; hétéroaryle ; aryl(alkyle en C1-C5) ; hétéroaryl(alkyle en C1-C5) ; -OR7 ; -[(CH2)o-O]p-alkyle où o et p valent d'une manière indépendante 1-3, et où chaque groupe alkyle, alcényle, alcynyle, cycloalkyle, cycloalkyl(alkyle en C1-C5), cyclohétéroalkyle, (cyclohétéroalkyle en C3-C7)(alkyle en C1-C5), aryle, aryl(alkyle en C1-C5), hétéroaryle, aryl(alkyle en C1-C5) et hétéroaryl(alkyle en C1-C5) est éventuellement substitué d'une manière indépendante par un ou plusieurs radicaux R7 ;
R6 est un groupe alkyle en C1-C10 ; alcényle en C2-C10 ; alcynyle en C2-C10 ; cycloalkyle en C3-C7 ; cyclohétéroalkyle en C3-C7 ; aryle ; hétéroaryle ; aryl(alkyle en C1-C5) ; hétéroaryl(alkyle en C1-C5) ; (cyclohétéroalkyle en C3-C7)(alkyle en C1-C5), où chaque groupe alkyle, alcényle, alcynyle, cycloalkyle, cyclohétéroalkyle, (cyclohétéroalkyle en C3-C7)(alkyle en C1-C5), aryle, aryl(alkyle en C1-C5), hétéroaryle et hétéroaryl(alkyle en C1-C5) est éventuellement substitué d'une manière indépendante par un ou plusieurs radicaux R11 ;
R7 est H ; =0 ; un groupe alkyle en C1-C10 ; alcényle en C2-C10 ; alcynyle en C2-C10 ; cycloalkyle en C3-C7 ; cyclohétéroalkyle en C3-C7 ; aryle ; hétéroaryle ; OR11 ; N(R11)2 ; SR11, où chaque groupe alkyle, alcényle, alcynyle, cycloalkyle, cyclohétéroalkyle, aryle et hétéroaryle est éventuellement substitué d'une manière indépendante par un ou plusieurs radicaux R11 ;
R8 est un groupe alkyle en C1-C10 ; alcényle en C2-C10 ; alcynyle en C2-C10 ; cycloalkyle en C3-C7 ; cyclohétéroalkyle en C3-C7 ; aryle ; hétéroaryle ; OR11 ; N(R11)2 ; SR11, où chaque groupe alkyle, alcényle, alcynyle, cycloalkyle, cyclohétéroalkyle, aryle et hétéroaryle est éventuellement substitué d'une manière indépendante par un ou plusieurs radicaux R11 ;
R9 et R10 représentent chacun indépendamment de l'autre H, un groupe alkyle en C1-C10 éventuellement substitué d'une manière indépendante par un ou plusieurs radicaux R8 ; halogéno ;
R11 est H ; -CF3 ; -CCl3 ; -OCF3 ; -OMe ; un groupe cyano ; halogéno ; -OH, COMe, -CONH2 ; CONHMe ; CONMe2 ; -NO2 ;
Si R9 et R10 sont des groupes alkyle en C1-C10, ils peuvent être reliés pour former un noyau cyclopropyle ;
si deux radicaux R4 ou deux radicaux R11 sont liés au même atome d'azote, ils peuvent être reliés pour former un noyau à 3 à 7 chaînons ;
ou tout tautomère, énantiomère, diastéréoisomère ou mélange de ces derniers, ainsi qu'un sel de ces derniers avec un acide ou une base acceptable d'un point de vue pharmaceutique.

4. Préparation pharmaceutique selon la revendication 1, dans laquelle la dipeptidyl peptidase VI est un composé de formule II 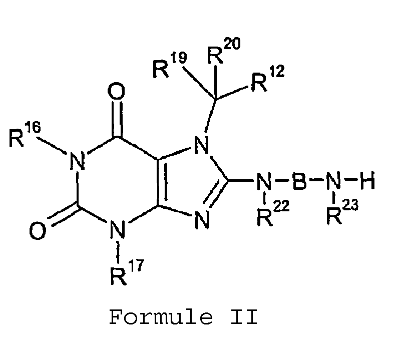 dans laquelle
B est un groupe alkylène en C2-C6 ; alcénylène en C2-C10 ; cycloalkylène en C3-C7 ; cyclohétéroalkylène en C3-C7 ; arylène ; hétéroarylène ; (alkylène en C1-C2)-arylène ; arylène(alkylène en C1-C2) ; (alkylène en C1-C2)(arylène)(alkylène en C1-C2), où chaque groupe alkylène, alcénylène, cycloalkylène, cyclohétéroalkylène, arylène ou hétéroarylène est en option substitué d'une manière indépendante par un ou plusieurs radicaux R14 ;
R12 est un groupe aryle éventuellement substitué d'une manière indépendante par un ou plusieurs radicaux R13, ou hétéroaryle éventuellement substitué d'une manière indépendante par un ou plusieurs radicaux R13 ;
R13 est H ; un groupe alkyle en C1-C7 ; alcényle en C2-C7 ; alcynyle en C2-C7 ; cycloalkyle en C3-C7 ; cyclohétéroalkyle en C3-C7 ; -NHCOR14 ; -NHSO2R14 ; -SR14 ; -SOR14 ; -SO2R14 ; -OCOR14 ; -CO2R15 ; -CON(R15)2 ; -CSN(R15)2 ; -NHCON(R15)2 ; -NHCSN(R15)2 ; -NHCONNH2 ; -SO2N(R15)2 ; -OR15 ; cyano ; nitro ; halogéno, où chaque groupe alkyle, alcényle, alcynyle, cycloalkyle et cyclohétéroalkyle est éventuellement substitué d'une manière indépendante par un ou plusieurs radicaux R14 ;
R14 est un groupe alkyle en C1-C10 ; alcényle en C2-C10 ; alcynyle en C2-C10 ; cycloalkyle en C3-C7 ; aryle ; hétéroaryle ; OR21 ; N(R21)2 ; SR21, où chaque groupe alkyle, alcényle, alcynyle, cycloalkyle, aryle et hétéroaryle est éventuellement substitué d'une manière indépendante par un ou plusieurs radicaux R21 ;
R15 est H ; un groupe alkyle en C1-C10 ; alcényle en C2-C10 ; alcynyle en C2-C10 ; cycloalkyle en C3-C7 ; cyclohétéroalkyle en C3-C7 ; aryle ; aryl(alkylène en C1-C5) ; hétéroaryle ; hétéroaryl(alkylène en C1-C5), où chaque groupe alkyle, alcényle, alcynyle, cycloalkyle, cyclohétéroalkyle, aryle, aryl(alkylène en C1-C5), hétéroaryle et hétéroaryl(alkylène en C1-C5) est éventuellement substitué d'une manière indépendante par un ou plusieurs radicaux R21 ;
R16 est H ; un groupe alkyle en C1-C10 ; alcényle en C2-C10 ; alcynyle en C2-C10 ; cycloalkyle en C3-C7 ; cyclohétéroalkyle en C3-C7 ; aryle ; hétéroaryle ; -OR18 ; -[(CH2)o-O]p-(alkyle en C1-C5), où o et p valent chacun indépendamment l'un de l'autre 1-3, et où chaque groupe alkyle, alcényle, alcynyle, cycloalkyle, cyclohétéroalkyle, aryle et hétéroaryle est éventuellement substitué d'une manière indépendante par un ou plusieurs radicaux R18 ;
R17 est H ; un groupe alkyle en C1-C10 ; alcényle en C2-C10 ; alcynyle en C2-C10 ; cycloalkyle en C3-C7 ; cyclohétéroalkyle en C3-C7 ; aryle ; hétéroaryle ; aryl-(alkylène en C1-C5) ; hétéroaryl(alkylène en C1-C5) ; (cyclohétéroalkyle en C3-C7)(alkylène en C1-C5), où chaque groupe alkyle, alcényle, alcynyle, cycloalkyle, cyclohétéroalkyle, (cyclohétéroalkyle en C3-C7)(alkylène en C1-C5), aryle, aryl(alkylène en C1-C5), hétéroaryle, aryl(alkylène en C1-C5) et hétéroaryl(alkylène en C1-C5) est éventuellement substitué d'une manière indépendante par un ou plusieurs radicaux R21 ;
R18 est H ; =0 ; un groupe alkyle en C1-C10 ; alcényle en C2-C10 ; alcynyle en C2-C10 ; cycloalkyle en C3-C7 ; cyclohétéroalkyle en C3-C7 ; aryle ; hétéroaryle ; OR21 ; N(R21)2 ; SR21 ; cyano ; hydroxy ; halogéno ; -CF3 ; -CCl3 ; -OCF3 ; ou -OCH3, où chaque groupe alkyle, alcényle, alcynyle, cycloalkyle, cyclohétéroalkyle, aryle et hétéroaryle est éventuellement substitué d'une manière indépendante par un ou plusieurs radicaux R21 ;
R19 est H ; un groupe alkyle en C1-C10 ; alcényle en C2-C10 ; alcynyle en C2-C10 ; cycloalkyle en C3-C7 ; cyclohétéroalkyle en C3-C7 ; aryle ; hétéroaryle ; OR21 ; N(R21)S ; SR21, où chaque groupe alkyle, alcényle, alcynyle, cycloalkyle, cyclohétéroalkyle, aryle et hétéroaryle est éventuellement substitué d'une manière indépendante par un ou plusieurs radicaux R21 ;
R20 est H ; un groupe alkyle en C1-C10 éventuellement substitué d'une manière indépendante par un ou plusieurs radicaux R19 ; ou halogéno ;
R21 est H ; -CF3 ; -CCl3 ; -OCF3 ; -OCH3 ; un groupe cyano ; halogéno ; -OH ; -COCH3 ; -CONH2 ; -CONHCH3 ; -CON(CH3)2 ; -NO2 ; -S02NH2 ; ou -SO2N(CH3)2 ;
si deux radicaux R15 ou deux radicaux R21 sont liés au même atome d'azote, ils peuvent être reliés pour former un noyau à 3 à 7 chaînons ;
R22 est H ; un groupe alkyle en C1-C6 éventuellement substitué d'une manière indépendante par un ou plusieurs radicaux R14 ;
R23 est H ; un groupe alkyle en C1-C6 éventuellement substitué d'une manière indépendante par un ou plusieurs radicaux R14 ; ou, si B est un groupe cycloalkylène en C3-C7 ou cyclohétéroalkylène en C3-C7, R23 peut être une liaison de valence entre l'atome d'azote auquel R23 est lié et l'un des atomes du groupe cycloalkylène ou cyclohétéroalkylène ;
ou tout tautomère, énantiomère, diastéréoisomère ou mélange de ces derniers, ainsi qu'un sel de ces derniers avec un acide ou une base acceptable d'un point de vue pharmaceutique.

5. Préparation pharmaceutique selon la revendication 1, dans laquelle la dipeptidyl peptidase IV est un composé de formule III 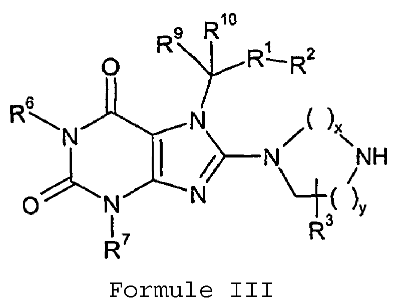 dans laquelle
x et y valent chacun d'une manière indépendante un ou deux ;
R1 est C=O ; C=S ; un groupe alkyle en C1-C2 éventuellement substitué d'une manière indépendante par un ou plusieurs radicaux R4 ; alcényle en C2 éventuellement substitué d'une manière indépendante par un ou plusieurs radicaux R4 ; alcynyle en C2 ; cycloalkyle en C3-C7 éventuellement substitué d'une manière indépendante par un ou plusieurs radicaux R4 ; cyclohétéroalkyle en C3-C7, éventuellement substitué d'une manière indépendante par un ou plusieurs radicaux R4 ; aryle, éventuellement substitué d'une manière indépendante par un ou plusieurs radicaux R4 ; aryl-(alkyle en C1-C3), éventuellement substitué d'une manière indépendante par un ou plusieurs radicaux R4 ; hétéroaryle, éventuellement substitué d'une manière indépendante par un ou plusieurs radicaux R4 ; hétéroaryl-(alkyle en C1-C3), éventuellement substitué d'une manière indépendante par un ou plusieurs radicaux R4 ; perhalogéno-(alkyle en C1-C10) ; perhalogéno(alkyloxy en C1-C10) ;
R2 est H ; un groupe alkyle en C1-C7, éventuellement substitué d'une manière indépendante par un ou plusieurs radicaux R4 ; alcényle en C2-C7, éventuellement substitué d'une manière indépendante par un ou plusieurs radicaux R4 ; alcynyle en C2-C7, éventuellement substitué d'une manière indépendante par un ou plusieurs radicaux R4 ; cycloalkyle en C3-C7, éventuellement substitué d'une manière indépendante par un ou plusieurs radicaux R4 ; cyclohétéroalkyle en C3-C7, éventuellement substitué d'une manière indépendante par un ou plusieurs radicaux R4 ; aryle, éventuellement substitué d'une manière indépendante par un ou plusieurs radicaux R4 ; aryl-(alkyle en C1-C3), éventuellement substitué d'une manière indépendante par un ou plusieurs radicaux R4 ; hétéroaryl-(alkyle en C1-C3), éventuellement substitué d'une manière indépendante par un ou plusieurs radicaux R4 ; hétéroaryle, éventuellement substitué d'une manière indépendante par un ou plusieurs radicaux R4 ; -SH ; -SR5 ; SOR5 ; SO2R5 ; -CHO ; -CH(OR5)2 ; carboxy ; -CO2R4 ; NHCONNHZ ; -NHCSNH2 ; -NHCONH2 ; -NHCOR4 ; -NHSO2R5 ; -O-CO-(alkyle en C1-C5), éventuellement substitué d'une manière indépendante par un ou plusieurs radicaux R4 ; cyano ; nitro ; halogéno ; hydroxy ; perhalogéno(alkyle en C1-C7) ; perhalogéno-(alkyloxy en C1-C7) ; -S02NH2 ; -SO2NH(R5) ; -S02(R5)2 ; -CONH2 ; -CSNH2 ; -CON2H3 ; -CONH(R5) ; -CON(R5)2 ; alkyloxy en C1-C10, éventuellement substitué d'une manière indépendante par R4 ; alcényloxy en C2-C10 éventuellement substitué par R4 ; alcynyloxy en C2-C10 éventuellement substitué d'une manière indépendante par R4, aryloxy éventuellement substitué d'une manière indépendante par R4 ; hétéroaryloxy éventuellement substitué d'une manière indépendante par R4 ;
R3 est H ; un groupe alkyle en C1-C10 éventuellement substitué d'une manière indépendante par un ou plusieurs radicaux R4 ; alcényle en C2-C10 éventuellement substitué d'une manière indépendante par un ou plusieurs radicaux R4 ; alcynyle en C2-C10, éventuellement substitué d'une manière indépendante par un ou plusieurs radicaux R4 ; cycloalkyle en C3-C7, éventuellement substitué d'une manière indépendante par un ou plusieurs radicaux R4 ; cyclohétéroalkyle en C3-C7, éventuellement substitué d'une manière indépendante par un ou plusieurs radicaux R4 ; aryle, éventuellement substitué d'une manière indépendante par un ou plusieurs radicaux R4 ; aryl-(alkyle en C1-C3), éventuellement substitué d'une manière indépendante par un ou plusieurs radicaux R4 ; hétéroaryl-(alkyle en C1-C3), éventuellement substitué d'une manière indépendante par un ou plusieurs radicaux R4 ; hétéroaryle éventuellement substitué d'une manière indépendante par un ou plusieurs radicaux R4 ; (alkyle en C1-C10)-NH(CH2)1-4NH-aryle, éventuellement substitué d'une manière indépendante par un ou plusieurs radicaux R4 ; (alkyle en C1-C10)-NH(CH2)-1-4NH-hétéroaryle, éventuellement substitué d'une manière indépendante par un ou plusieurs radicaux R4 ; (alkyle en C1-C10)-O(CH2)1-4NH-aryle, éventuellement substitué d'une manière indépendante par un ou plusieurs radicaux R4 ; (alkyle en C1-C10)-O(CH2)1-4NH-hétéroaryle, éventuellement substitué d'une manière indépendante par un ou plusieurs radicaux R4 ; (alkyle en C1-C10)-O(CH2)1-40-aryle, éventuellement substitué d'une manière indépendante par un ou plusieurs radicaux R4 ; (alkyle en C1-C10)-O(CH2)1-40-hétéroaryle, éventuellement substitué d'une manière indépendante par un ou plusieurs radicaux R4 ; (alkyle en C1-C10)-S(CH2)1-4NH-aryle, éventuellement substitué d'une manière indépendante par un ou plusieurs radicaux R4 ; (alkyle en C1-C10)-S(CH2)1-4NH-hétéroaryle, éventuellement substitué d'une manière indépendante par un ou plusieurs radicaux R4 ; (alkyle en C1-C10)-S(CH2)1-4S-aryle, éventuellement substitué d'une manière indépendante par un ou plusieurs radicaux R4 ; (alkyle en C1-C10)-S(CH2)1-4S-hétéroaryle, éventuellement substitué d'une manière indépendante par un ou plusieurs radicaux R4 ; (alkyle en C1-C10)-O-(alkyle en C1-C5) éventuellement substitué par un ou plusieurs radicaux R4 ; -NHCOR4 ; -NHSO2R5 ; -O-CO-(alkyle en C1-C5), éventuellement substitué d'une manière indépendante par un ou plusieurs radicaux R4 ; -SH ; -SR5 ; -SOR5 ; -SO2R5 ; -CHO ; -CH(OR5)2 ; carboxy ; cyano ; nitro ; halogéno ; hydroxy ; -SO2NH2 ; -S02NH(R5) ; -SO2N(R5)2 ; -CONH2 ; -CONH(R5) ; -CON(R5)2 ; -CSNH2 ; -CONHNH2 ; -CO2R4 ; -NHCNHNH2 ; -NHCSNH2 ; -NHCONH2 ;
R4 est un groupe alkyle en C1-C10, éventuellement substitué d'une manière indépendante par un ou plusieurs radicaux R8 ; alcényle en C2-C10, éventuellement substitué d'une manière indépendante par un ou plusieurs radicaux R8 ; alcynyle en C2-C10, éventuellement substitué d'une manière indépendante par un ou plusieurs radicaux R8 ; cycloalkyle en C3-C7, éventuellement substitué d'une manière indépendante par un ou plusieurs radicaux R8 ; cylohétéroalkyle en C3-C7, éventuellement substitué d'une manière indépendante par un ou plusieurs radicaux R8 ; aryle, éventuellement substitué d'une manière indépendante par un ou plusieurs radicaux R8 ; hétéroaryle, éventuellement substitué d'une manière indépendante par un ou plusieurs radicaux R8 ; amino ; amino substitué par un ou plusieurs groupes alkyle en C1-C10, éventuellement substitués par un ou plusieurs radicaux R8 ; amino substitué par ou deux groupes aryle, éventuellement substitués d'une manière indépendante par un ou plusieurs radicaux R8 ; hétéroaryle, éventuellement substitué d'une manière indépendante par un ou plusieurs radicaux R8 ; =0 ; =S ; -CO-R5 ; -COOR5 ; -O-CO(alkyle en C1-C5) éventuellement substitué d'une manière indépendante par un ou plusieurs radicaux R8 ; NH(CH2)1-4NH-aryle ; NH(CH2)1-4NH-hétéroaryle ; -NHCOR5 ; -SOR5 ; SO2R5 ; carboxy ; cyano ; N-hydroxyimino ; nitro ; halogéno ; hydroxy ; perhalogéno(alkyle en C1-C10) ; perhalogéno-(alkyloxy en C1-C10) ; -SH ; -SR5 ; -SO3H ; -S03R5 ; -SO2R5 ; -SO2NH2 ; -SO2NH(R5) ; -SO2N(R5)2 ; -CONH2 ; -CONH(R5) ; -CON(R5)2 ; alkyloxy en C1-C10, éventuellement substitué d'une manière indépendante par un ou plusieurs radicaux R8 ; alcényloxy en C2-C10, éventuellement substitué d'une manière indépendante par un ou plusieurs radicaux R8 ; alcynyloxy en C2-C10, éventuellement substitué d'une manière indépendante par un ou plusieurs radicaux R8 ; aryloxy, éventuellement substitué d'une manière indépendante par un ou plusieurs radicaux R8 ; aryloxy, éventuellement substitué d'une manière indépendante par un ou plusieurs radicaux R8 ; hétéroaryloxy, éventuellement substitué d'une manière indépendante par un ou plusieurs radicaux R8 ; et deux radicaux R4 liés au même atome de carbone peuvent former un système spirohétérocyclique, de préférence d'hydantoïne ; la thiohydantoïne ; l'oxazolidine-2,5-dione ;
R5 est un groupe alkyle en C1-C10 éventuellement substitué d'une manière indépendante par un ou plusieurs radicaux R8 ; alcényle en C2-C10, éventuellement substitué d'une manière indépendante par un ou plusieurs radicaux R8 ; alcynyle en C2-C10, éventuellement substitué d'une manière indépendante par un ou plusieurs radicaux R8 ; cycloalkyle en C3-C7, éventuellement substitué d'une manière indépendante par un ou plusieurs radicaux R8 ; cyclohétéroalkyle en C3-C7, éventuellement substitué d'une manière indépendante par un ou plusieurs radicaux R8 ; aryle, éventuellement substitué d'une manière indépendante par un ou plusieurs radicaux R8 ; aryl-(alkyle en C1-C5), éventuellement substitué d'une manière indépendante par un ou plusieurs radicaux R8 ; hétéroaryle, éventuellement substitué d'une manière indépendante par un ou plusieurs radicaux R8 ; hétéroaryl(alkyle en C1-C5), éventuellement substitué d'une manière indépendante par un ou plusieurs radicaux R8 ;
R6 est H ; un groupe alkyle en C1-C10, éventuellement substitué d'une manière indépendante par un ou plusieurs radicaux R4 ; alcényle en C2-C10, éventuellement substitué d'une manière indépendante par un ou plusieurs radicaux R4 ; alcynyle en C2-C10, éventuellement substitué d'une manière indépendante par un ou plusieurs radicaux R4 ; cycloalkyle en C3-C7, éventuellement substitué d'une manière indépendante par un ou plusieurs radicaux R4 ; cyclohétéroalkyle en C3-C7, éventuellement substitué d'une manière indépendante par un ou plusieurs radicaux R4 ; aryle, éventuellement substitué d'une manière indépendante par un ou plusieurs radicaux R4 ; hétéroaryle, éventuellement substitué d'une manière indépendante par un ou plusieurs radicaux R4 ;
R7 est H ; un groupe alkyle en C1-C10, éventuellement substitué d'une manière indépendante par un ou plusieurs radicaux R4 ; alcényle en C2-C10, éventuellement substitué d'une manière indépendante par un ou plusieurs radicaux R4 ; alcynyle en C2-C10, éventuellement substitué d'une manière indépendante par un ou plusieurs radicaux R4 ; cycloalkyle en C3-C7, éventuellement substitué d'une manière indépendante par un ou plusieurs radicaux R4 ; cyclohétéroalkyle en C3-C7, éventuellement substitué d'une manière indépendante par un ou plusieurs radicaux R4 ; aryle, éventuellement substitué d'une manière indépendante par un ou plusieurs radicaux R4 ; hétéroaryle, éventuellement substitué d'une manière indépendante par un ou plusieurs radicaux R4 ;
R8 est H, un groupe aminoxime ; nitro, tétrazole ; pentafluorophényle ; -CH2OH ; -CHO ; -C(OCH3)2 ; -COCH3 ; -CF3 ; -CCl3 ; -OCF3 ; -OCH3 ; -CN ; -CO2H ; -CO2CH3 ; -CONH2 ; -CSNH2 ; -CON2H3 ; -S03H ; -SO2NH2 ; -SO2NHCH3 ; -SO2N(CH3)2 ; -SO2(1-pipérazinyle) ; -SO2(4-méthyl-pipérazine-1-yle) ; -S02(pyrrolidine-1-yle) ; -SO2(pipéridine-1-yle) ; -SO2(morpholine-4-yle) ; N-hydroxyimino ; -NH2 ; -NHCH3 ; -N(CH3)2 ; -NHCNHN2 ; -NHCNHNHCH3 ; -NHCSNH2 ; -NHCSNHCH3 ; -NHCONH2 ; -NHCONHCH3 ; -NHCOCH3 ; -NHSO2CH3 ; pipérazinyle ; morpholine-4-yle ; thiomorpholine-4-yle ; pyrrolidine-1-yle ; pipéridine-1-yle ; halogéno ; -OH ; -SH ; -SCH3 ; aminoacétyle ; -OPO3H ; -OPO2OCH3 ; -PO3H2 ; -PO(OCH3)2 ; PO(OH)(OCH3) ;
R9 est H ; un groupe halogéno ; alkyle en C1-C10, éventuellement substitué d'une manière indépendante par un ou plusieurs radicaux R4 ;
R10 est H ; un groupe halogéno ;
ou R9 et R10 peuvent être reliés pour former un noyau cyclopropyle ;
ou tout tautomère, énantiomère, diastéréoisomère ou mélange de ces derniers, ainsi qu'un sel de ces derniers avec un acide ou une base acceptable d'un point de vue pharmaceutique ;
à l'exception des composés suivants :
1,3-diméthyl-7-(2-oxo-propyl)-8-pipérazine-1-yl-3,7-dihydro-purine-2,6-dione,
1,3,1',3',7'-pentaméthyl-8-pipérazine-1-yl-3,7,3',7'-tétrahydro-7,8'-méthanediyl-bis-purine-2,6-dione,
ester 2-(1,3-diméthyl-2,6-dioxo-8-pipérazine-1-yl)-1,2,3,6-tétrahydro-purine-7-yl)éthylique de l'acide 3,4,5-triméthoxy-benzoïque,
7-[2-hydroxy-3-(4-méthoxy-phénoxy)-propyl]-3-méthyl-8-pipérazine-1-yl-3,7-dihydro-purine-2,6-dione,
7-[2-hydroxy-2-(4-nitro-phényl)-éthyl]3-méthyl-8-pipérazine-7-yl-3,7,8,9-tétrahydro-purine-2,6-dione,
7-benzyl-3-méthyl-8-pipérazine-1-yl-3,7-dihydro-purine-2,6-dione,
7-(4-chloro-benzyl)-3-méthyl-8-pipérazine-1-yl-3,7-dihydro-purine-2,6-dione,
7-(2-chloro-benzyl)-3-méthyl-8-pipérazine-1-yl-3,7-dihydro-purine-2,6-dione,
7-éthyl-3-méthyl-8-pipérazine-1-yl-3,7-dihydro-purine-2,6-dione,
3-méthyl-8-pipérazine-1-yl-1,7-dipropyl-3,7-dihydro-purine-2,6-dione,
3-méthyl-7-(3-méthyl-butyl)-8-pipérazine-1-yl-3,7-dihydro-purine-2,6-dione,
7-butyl-3-méthyl-8-pipérazine-1-yl-3,7-dihydro-purine-2,6-dione,
3-méthyl-7-(3-phényl-propyl)-8-pipérazine-1-yl-3,7-dihydro-purine-2,6-dione,
7-but.-2-ényl-3-méthyl-8-pipérazine-1-yl-3,7-dihydro-purine-2,6-dione,
7-(3-chloro-but.-2-ényl)-3-méthyl-8-pipérazine-1-yl-3,7-dihydro-purine-2,6-dione,
7-heptyl-3-méthyl-8-pipérazine-1-yl-3,7-dihydro-purine-2,6-dione,
3-méthyl-7-(1-phényl-éthyl)-8-pipérazine-1-yl-3,7-dihydro-purine-2,6-dione,
3-méthyl-7-(3-méthyl-benzyl)-8-pipérazine-1-yl-3,7-dihydro-purine-2,6-dione,
3-méthyl-7-propyl-8-pipérazine-1-yl-3,7-dihydro-purine-2,6-dione, et
3-méthyl-7-pentyl-8-pipérazine-1-yl-3,7-dihydro-purine-2,6-dione.

6. Préparation pharmaceutique selon l'une quelconque des revendications 1 à 5, dans laquelle l'inhibiteur de la NEP est le candoxatril.

7. Préparation pharmaceutique selon l'une quelconque des revendications 1 à 5, dans laquelle l'inhibiteur de la NEP est un inhibiteur double de NEP/ACE.

8. Préparation pharmaceutique selon l'une quelconque des revendications 1 à 6, qui comprend en outre un autre antidiabétique.

9. Préparation pharmaceutique selon l'une quelconque des revendications 1 à 8, qui comprend en outre un inhibiteur de l'enzyme de conversion de l'angiotensine (ACE).
